# EUROPEAN PATENT APPLICATION

(11) **EP 1 338 896 A2**
(43) Date of publication of application: **27.08.2003**
(21) Application number: 03008188.9
(22) Date of filing: 02.10.1998
(51) Int. Cl.: G01N 33/577, G01N 33/68

(54) **ICAM-4 and diagnostic uses thereof**

(30) Priority: 02.10.1997 US 942867
(62) Divisional of application: 98953249.4
(71) Applicant: ICOS CORPORATION, Bothell, WA 98021 (US)
(72) Inventor: Kilgannon, Patrick D., Bothell, WA 98011 (US); Gallatin, Michael W., Mercer Island, WA 98040 (US)
(74) Representative: Richardson, Kate

(57) **Abstract**

"Method For Quantitating Concentration Of Circulating ICAM-4 In Association With Various Neurodegenerative Disorders Are Provided"

## Description

This application is a continuation-in-part of U.S. Patent Application Serial No. 08/656,984, filed June 6, 1996 and currently pending, is a continuation-in-part of U.S. Patent Application Serial No. 08/481,130, filed June 7, 1995 and currently pending, which is a continuation-in-part of U.S. Patent Application Serial No. 08/245,295, filed May 18, 1994 and currently pending, which in turn is a continuation-in-part of U.S. Patent Application Serial No. 08/102,852, filed August 5, 1993 and now abandoned, which is a continuation-in-part of U.S. Patent Application Serial No. 08/009,266, filed January 22, 1993 and now abandoned, which is a continuation-in-part of U.S. Patent Application Serial No. 07/894,061, filed June 5, 1992 and now abandoned, which is a continuation-in-part of U.S. Patent Application Serial No. 07/889.724, filed May 26, 1992 and now abandoned which is a continuation-in-part of co-pending U.S. Patent Application Serial No. 07/827,689, filed January 27. 1992 and now abandoned.

### FIELD OF THE INVENTION

The present invention relates generally to cellular adhesion molecules and more particularly to the cloning and expression of DNA encoding a heretofore unknown polypeptide designated "ICAM-4" which possesses structural relatedness to the intercellular adhesion molecules ICAM-1, ICAM-2, and ICAM-R.

### BACKGROUND OF THE INVENTION

Research spanning the last decade has significantly elucidated the molecular events attending cell-cell interactions in the body, especially those events involved in the movement and activation of cells in the immune system, and more recently, those involved in development and normal physiological function of cells in the nervous system. See generally, Springer, *Nature,* 346: 425-434 (1990) regarding cells of the immune system, and Yoshihara, *et al. Neurosci.Res.* 10:83-105 (1991) and Sonderegger and Rathjen, *J.Cell Biol.* 119:1387-1394 (1992) regarding cells of the nervous system. Cell surface proteins, and especially the so-called Cellular Adhesion Molecules ("CAMS") have correspondingly been the subject of pharmaceutical research and development having as its goal intervention in the processes of leukocyte extravasation to sites of inflammation and leukocyte movement to distinct target tissues, as well as neuronal differentiation and formation of complex neuronal circuitry. The isolation and characterization of cellular adhesion molecules, the cloning and expression of DNA sequences encoding such molecules, and the development of therapeutic and diagnostic agents relevant to inflammatory processes and development and function of the nervous system have also been the subject of numerous U.S. and foreign applications for Letters Patent. See Edwards, *Current Opinion in Therapeutic Patents, 1(11):* 1617-1630 (1991) and particularly the published "patent literature references" cited therein.

Of fundamental interest to the background of the present invention are the prior identification and characterization of certain mediators of cell adhesion events, the "leukointegrins," LFA-1, MAC-1 and gp 150.95 (referred to in WHO nomenclature as CD18/CD11a, CD18/CD11b, and CD18/CD11c, respectively) which form a subfamily of heterodimeric "integrin" cell surface proteins present on B lymphocytes, T lymphocytes, monocytes and granulocytes. See, e.g., Table 1 of Springer, *supra*, at page 429. Also of interest are other single chain adhesion molecules (CAMs) that have been implicated in leukocyte activation, adhesion, motility and the like, which are events attendant to the inflammatory process. For example, it is presently believed that prior to the leukocyte extravasation which characterizes inflammatory processes, activation of integrins constitutively expressed on leukocytes occurs and is followed by a tight ligand/receptor interaction between the integrins (e.g., LFA-1) and one or both of two distinct intercellular adhesion molecules (ICAMs) designated ICAM-1 and ICAM-2 which are expressed on blood vessel endothelial cell surfaces and on other leukocytes.

Like the other CAMs characterized to date, [e.g., vascular adhesion molecule (VCAM-1) as described in PCT WO 90/13300 published November 15, 1990; and platelet endothelial cell adhesion molecule (PECAM-1) as described in Newman *et al., Science,* 247: 1219-1222 (1990) and PCT WO 91/10683 published July 25, 1991], ICAM-1 and ICAM-2 are structurally homologous to other members of the immunoglobulin gene superfamily in that the extracellular portion of each is comprised of a series of domains sharing a similar carboxy terminal motif. A "typical" immunoglobulin-like domain contains a loop structure usually anchored by a disulfide bond between two cysteines at the extremity of each loop. ICAM-1 includes five immunoglobulin-like domains; ICAM-2, which differs from ICAM-1 in terms of cell distribution, includes two such domains; PECAM-1 includes six; VCAM includes six or seven, depending on splice variations, and so on. Moreover, CAMs typically include a hydrophobic "transmembrane" region believed to participate in orientation of the molecule at the cell surface and a carboxy terminal "cytoplasmic" region. Graphic models of the operative disposition of CAMs generally show the molecule anchored in the cell membrane at the transmembrane region with the cytoplasmic "tail" extending into the cell cytoplasm and one or more .immunoglobulin-like loops extending outward from the cell surface.

A number of neuronal cells express surface receptors with extracellular Ig-like domains, structurally similarity to the ICAMs. See for example, Yoshihara, *et al., supra*. In addition to Ig-like domains, many adhesion molecules of the nervous system also contain tandemly repeated fibronectin-like sequences in the extracellular domain.

A variety of therapeutic uses has been projected for intercellular adhesion molecules, including uses premised on the ability of ICAM-1 to bind human rhinovirus. European Patent Application 468 257 A published January 29, 1992, for example, addresses the development of multimeric configurations and forms of ICAM-1 (including full length and truncated molecular forms) proposed to have enhanced ligand/receptor binding activity, especially in binding to viruses, lymphocyte associated antigens and pathogens such as *Plasmodium falciparum.*

In a like manner, a variety of uses has been projected for proteins immunologically related to intercellular adhesion molecules. WO91/16 published November 14, 1991, for example, addresses humanized chimeric anti-ICAM-1 antibodies and their use in treatment of specific and non-specific inflammation, viral infection and asthma. Anti-ICAM-1 antibodies and fragments thereof are described as useful in treatment of endotoxic shock in WO92/04034, published March 19, 1992. Inhibition of ICAM-1 dependent inflammatory responses with anti-ICAM-1 anti-idiotypic antibodies and antibody fragments is addressed in WO92/06119 published April 16, 1992.

Despite the fundamental insights into cell adhesion phenomena which have been gained by the identification and characterization of intercellular adhesion proteins such as ICAM-1 and lymphocyte interactive integrins such as LFA-1, the picture is far from complete. It is generally believed that numerous other proteins are involved in inflammatory processes and in targeted lymphocyte movement throughout the body. For example, U.S. Patent Application Serial Nos. 07/827,689, 07/889,724, 07/894,061 and 08/009,266 and corresponding published PCT Application WO 93/14776 (published August 5, 1993) disclose the cloning and expression of an ICAM-Related protein, ICAM-R. The disclosures of these applications are specifically incorporated by reference herein and the DNA and amino acid sequences of ICAM-R are set out in SEQ ID NO. 4 herein. This new ligand has been found to be expressed on human lymphocytes, monocytes and granulocytes.

Of particular interest to the present application, still another ICAM-like surface molecule was identified which. has a tissue specific expression unlike that of any known ICAM molecule. Mori, *et al*., [Proc.Natl.Acad.Sci. *(USA)* 84:3921-3925 (1987)] reported identification of a telencephalon-specific antigen in rabbit brain, specifically immunoreactive with monoclonal antibody 271A6. This surface antigen was named telencephalin. Imamura, *et al.,* [*Neurosci.Letts.* 119:118-121 (1990)], using a polyclonal antibody to assess localized expression, asserted that expression of telencephalin in visual cortex of cats showed variation in layers of the tissue, and also reported telencephalin expression was variable as a function of development. Oka, *et al.*, *[Neuroscience* 35:93-103 (1990)] subsequently reported isolation of telencephalin using monoclonal antibody 271A6. The publication reports a molecular weight for the surface molecule of about 500 kD and that the molecule was composed of four subunits, each with a native molecular weight of 130 kD and approximately 100 kD following *N*-glycanase treatment. Yoshihiro, *et al.,* [*Neuroscience*, Research Supplement 18, p. S83 (1994)], reported the cDNA and amino acid sequences for rabbit telencephalin at the 17th Annual Meeting of the Japan Neuroscience Society in Nagoya, Japan, December 7-9, 1993, and the 23rd Annual Meeting of the Society for Neuroscience in Washington, D.C., November 9, 1993 [Society for Neuroscience Abstracts 19 (1-3) p. 646 (1993)]. The deduced amino acid sequence reported suggested that the 130 kD telencephalon is an integral membrane protein with nine extracellular immunoglobulin (Ig)-like domains. The distal eight of these domains showed homology to other ICAM Ig-like domains. This same information was reported by Yoshihara, *et al., in Neuron 12*:543-553 (1994).

There thus continues to be a need in the art for the discovery of additional proteins participating in human cell-cell interactions and especially a need for information serving to specifically identify and characterize such proteins in terms of their amino acid sequence. Moreover, to the extent that such molecules might form the basis for the development of therapeutic and diagnostic agents, it is essential that the DNA encoding them be elucidated. Such seminal information would *inter alia,* provide for the large scale production of the proteins, allow for the identification of cells naturally producing them, and permit the preparation of antibody substances or other novel binding proteins specifically reactive therewith and/or inhibitory of ligand/receptor binding reactions in which they are involved.

### BRIEF SUMMARY OF THE INVENTION

In one of its aspects, the present invention provides purified and isolated polynucleotides (e.g., DNA sequences, RNA transcripts and anti-sense oligonucleotides thereof) encoding a novel polypeptide, "ICAM-4," as well as polypeptide variants (including fragments and deletion, substitution, and addition analogs) thereof which display one or more ligand/receptor binding biological activities and/or immunological properties specific to ICAM-4. ICAM-4-specific ligand/receptor binding. biological activities encompass interactions of both the ICAM-4 extracellular and cytoplasmic domains with other molecules (e.g., in processes of cell-cell adhesion and/or signal transduction). Preferred DNA sequences of the invention include genomic and cDNA sequences as well as wholly or partially chemically synthesized DNA sequences. A presently preferred polynucleotide is set out in SEQ ID NO: 1 and encodes rat species ICAM-4. Biological replicas (i.e., copies of isolated DNA sequences made *in vivo* or *in vitro*) of DNA sequences of the invention are contemplated. Also provided autonomously replicating recombinant constructions such as plasmid and viral DNA vectors incorporating ICAM-4 sequences and especially vectors wherein DNA encoding ICAM-4 or an ICAM-4 variant is operatively linked to an endogenous or exogenous expression control DNA sequence.

According to another aspect of the invention, host cells, especially unicellular host cells such as procaryotic and eucaryotic cells, are stably transformed with DNA sequences of the invention in a manner allowing the desired polypeptides to be expressed therein. Host cells expressing such ICAM-4 and ICAM-4 variant products can serve a variety of useful purposes. To the extent that the expressed products are "displayed" on host cell surfaces, the cells may constitute a valuable immunogen for the development of antibody substances specifically immunoreactive with ICAM-4 and ICAM-4 variants. Host cells of the invention are conspicuously useful in methods for the large scale production of ICAM-4 and ICAM-4 variants wherein the cells are grown in a suitable culture medium and the desired polypeptide products are isolated from the cells or from the medium in which the cells are grown.

Novel ICAM-4 of the invention may be obtained as isolates from natural cell sources, but, along with ICAM-4 variant products, are preferably produced by recombinant procedures involving host cells of the invention. A presently preferred amino acid sequence for an ICAM-4 polypeptide is set out in SEQ ID NO: 2. The products may be obtained in fully or partially glycosylated, partially or wholly de-glycosylated, or non-glycosylated forms, depending on the host cell selected for recombinant production and/or post-isolation processing. ICAM-4 variants of the invention may comprise water soluble or insoluble monomeric, multimeric or cyclic ICAM-4 fragments which include all or part of one or more of the domain regions specified above and having a biological or immunological property of ICAM-4 including, *e.g.*, the ability to bind to a binding partner of ICAM-4 and/or inhibit binding of ICAM-4 to a natural binding partner. ICAM-4: variants of the invention may also comprise polypeptide analogs wherein one or more of the specified amino acids is deleted or replaced: (1) without loss, and preferably with enhancement, of one or more biological activities or immunological characteristics specific for ICAM-4; or (2) with specific disablement of a particular ligand/receptor binding function. Analog polypeptides including additional amino acid (*e.g*., lysine or cysteine) residues that facilitate multimer formation are contemplated.

Also comprehended by the present invention are antibody substances (*e.g*., monoclonal and polyclonal antibodies, antibody fragments, single chain antibodies, chimeric antibodies, CDR-grafted antibodies and the like) and other binding proteins (*e.g*., polypeptides and peptides) which are specific *(i.e.,* non-reactive with the ICAM-1, ICAM-2, and ICAM-R intercellular adhesion molecules to which ICAM-4 is structurally related) for ICAM-4 or ICAM-4 variants. The invention also comprehends hybridoma cell lines which specifically secrete monoclonal antibodies of the invention. Presently preferred hybridomas of the invention include those designated 127A, 127H, 173E, 179I, and 179H. Antibody substances can be developed using isolated natural or recombinant ICAM-4 or ICAM-4 variants or cells expressing such products on their surfaces. Binding proteins of the invention are additionally useful for characterization of binding site structure(s) *(e.g.,* epitopes and/or sensitivity of binding properties to modifications in ICAM-4 amino acid sequence).

Binding proteins are useful, in turn, in compositions for immunization as well as for purifying polypeptides of the invention and identifying cells displaying the polypeptides on their surfaces. They are also manifestly useful in modulating (i.e., blocking, inhibiting or stimulating) ligand/receptor binding biological activities involving ICAM-4, especially those ICAM-4 effector functions involved in specific and non-specific immune system responses. Anti-idiotypic antibodies specific for anti-ICAM-4 antibody substances and uses of such anti-idiotypic antibody substances in modulating immune responses are also contemplated. The invention further provides methods of screening for neuropathology in an individual comprising. the steps of: a) obtaining a fluid sample from the individual; b) contacting the sample with an antibody specifically immunoreactive with ICAM-4; c) quantitating the level of ICAM-4/antibody binding in the sample; and d) comparing the level of ICAM-4/antibody binding in the sample to the level of ICAM-4/antibody binding in individuals (controls) known to be free of the neuropathology. Assays for the detection and quantification of ICAM-4 on cell surfaces and in body fluids, such as serum or cerebrospinal fluid, may involve, for example, a single antibody substance or multiple antibody substances in a "sandwich" assay format. In detecting ICAM-4 in a body fluid, antibodies of the invention are also useful for assessing the occurrence of neuropathologies which can be correlated to increased levels of circulating ICAM-4. Such neuropathologies include, but are not limited to, cerebral ischemia (*i.e*., stroke) resulting from various disorders including, for example, thrombosis, embolism, cerebral aneurysmal hemorrhage, vasospasm, and the like. Quantitation of circulating ICAM-4 can also distinguish between various forms of epilepsy and may also permit determination of the stage of AIDS progression. Still other neurodegenerative disorders for which measurement of circulating ICAM-4 can be useful for diagnosis include various forms of Alzheimer's disease and other cortical dementias (such as Pick's disease, diffuse cortical Lewy body disease, and frontal lobe degeneracy), subcortical dementias (including Parkinson's disease, Huntington's disease, and progressive supranuclear), a number of the primary psychiatric disorders (such as depression, schizophrenia and psychosis), as well as nongenetic dementias arising from, for example, infections, vasculitis, metabolic and nutritional disorders (*e.g*., thyroid, vitamin B12 deficiency), vascular disorders (multiple infarct, lacunar state, Binswanger's disease), toxic encephalopathies (*e.g*., exposure to carbon monoxide, heavy metals or other industrial pollutants) and tumors.

The scientific value of the information contributed through the disclosures of DNA and amino acid sequences of the present invention. is manifest. As one series of examples, knowledge of the sequence of a cDNA for ICAM-4 makes possible the isolation by DNA/DNA hybridization of genomic DNA sequences encoding ICAM-4 and specifying ICAM-4 expression control regulatory sequences such as promoters, operators and the like. DNA/DNA hybridization procedures carried out with DNA sequences of the invention and under stringent conditions are likewise expected to allow the isolation of DNAs encoding.allelic variants of ICAM-4, other structurally related proteins sharing one or more of the biological and/or immunological properties specific to ICAM-4, and proteins homologous to ICAM-4 from other species. DNAs of the invention are useful in DNA/RNA hybridization assays to detect the capacity of cells to synthesize ICAM-4. Also made available by the invention are anti-sense polynucleotides relevant to regulating expression of ICAM-4 by those cells which ordinarily express the same. As another series of examples, knowledge of the DNA and amino acid sequences of ICAM-4 makes possible the generation by recombinant means of ICAM-4 variants such as hybrid fusion proteins (sometimes referred to as "immuno-adhesions") characterized by the presence of ICAM-4 protein sequences and .immunoglobulin heavy chain constant regions and/or hinge regions. See, Capon *et al., Nature*, 337: 525-531 (1989); Ashkenazi *et al., P.N.A.S*. *(USA),* 88: 10535-10539 (1991); and PCT WO 89/02922, published April 6, 1989. ICAM-4 variant fusion proteins may also include, for example, selected extracellular domains of ICAM-4 and portions of other cell adhesion molecules.

DNA of the invention also permits identification of untranslated DNA sequences which specifically promote expression of polynucleotides operatively linked to the promoter regions. Identification and use of such promoter sequences are particularly desirable in instances, for example gene transfer, which can specifically require heterologous gene expression in a limited neuronal environment. The invention also comprehends vectors comprising promoters of the invention, as well as chimeric gene constructs wherein the promoter of the invention is operatively linked to a heterologous polynucleotide sequence and a transcription termination. signal.

The DNA and amino acid sequence information provided by the present invention also makes possible the systematic analysis of the structure and function of ICAM-4 and definition of those molecules with which it will interact on extracellular and intracellular levels. The idiotypes of anti-ICAM-4 monoclonal antibodies of the invention are representative of such molecules and may mimic natural binding proteins (peptides and polypeptides) through which ICAM-4 intercellular and intracellular activities are modulated or by which ICAM-4 modulates intercellular and intracellular events. Alternately, they may represent new classes of modulators of ICAM-4 activities. Anti-idiotypic antibodies, in turn, may represent new classes of biologically active ICAM-4 equivalents. *In vitro* assays for identifying antibodies or other compounds that modulate the activity of ICAM-4 may involve, for example, immobilizing ICAM-4 or a natural ligand to which ICAM-4 binds, detectably labelling the nonimmobilized binding partner, incubating the binding partners together and determining the effect of a test compound on the amount of label bound wherein a reduction in the label bound in the presence of the test compound compared to the amount of label bound in the absence of the test compound indicates that the test agent is an inhibitor of ICAM-4 binding.

The DNA sequence information provided by the present invention also makes possible the development, by homologous recombination or "knockout" strategies [see, *e.g.,* Kapecchi, *Science*, 244: 1288-1292 (1989)], of rodents that fail to express a functional ICAM-4 protein or that express a variant ICAM-4 protein. Such rodents are useful as models for studying the activities of ICAM-4 and ICAM-4 modulators *in vivo*.

### DETAILED DESCRIPTION OF THE INVENTION

The disclosures of parent U.S. Patent Application Serial No. 08/102,852, filed August 5, 1993, are specifically incorporated by reference. The examples of that application address, *inter alia*: design and construction of oligonucleotide probes for PCR amplification of ICAM related DNAs; use of the probes to amplify a human genomic fragment homologous to, but distinct from DNAs encoding ICAM-1 and ICAM-2; screening of cDNA libraries with the genomic fragment to isolate additional ICAM-R coding sequences; screening of cDNA libraries to isolate a full length human cDNA sequence encoding ICAM-R; characterization of DNA and amino acid sequence information for ICAM-R, especially as related to ICAM-1 and ICAM-2; development of mammalian host cells expressing ICAM-R; assessment of indications of ICAM-R participation in adhesion events involving CD18-dependent and CD18-independent pathways; inhibition of cell adhesion to ICAM-R by ICAM-R-derived peptides; expression of variants of ICAM-R; preparation and characterization of anti-ICAM-R antibodies and fragments thereof; mapping of ICAM-R epitopes recognized by anti-ICAM-R monoclonal antibodies; assessment of the distribution and biochemical characterization of ICAM-R and RNA encoding the same; assessment of ICAM-R in homotypic cell-cell adhesion and immune cell activation/proliferation; characterization of ICAM-R monoclonal antibodies; and assessment of differential phosphorylation and cytoskeletal associations of the cytoplasmic domain of ICAM-R. Also disclosed was the identification of a rodent ICAM-encoding DNA that, at the time, appeared to be the rat homolog of human ICAM-R, and the use of this DNA to construct and express DNAs encoding glutathione-S-transferase fusion proteins. The detailed description of how this rodent DNA was identified can be found in the parent application (U.S.S.N. 08/102,852) in Example 6, and is reproduced herein as Example 1. As more of the rodent ICAM-coding sequence was identified, it became apparent that the rodent ICAM DNA did not encode a rat species homolog of human ICAM-R, but, in. fact, encoded a novel ICAM polypeptide, herein named ICAM-4. In order to appreciate the events which led to the identification of ICAM-4, a chronology is provided which is followed by a detailed description of the invention.

A first rodent genomic ICAM-4 sequence was identified which encoded a region homologous to domain 2 (herein SEQ ID NO: 3, and SEQ ID NO: 23 of U.S.S.N. 08/102,852) of human ICAM-R (herein as SEQ ID NO: 4). A second, overlapping genomic DNA (herein SEQ ID NO: 5, and SEQ ID NO: 26 of U.S.S.N. 08/102,852) was also identified which encoded both the domain 2 region of SEQ ID NO: 3, and sequences for ICAM-1. Using SEQ ID NO: 3 as a probe, a rodent spleen cDNA (herein SEQ ID NO: 6, and SEQ ID NO: 25 in U.S.S.N. 08/102,852) was identified which encoded domains 2 through 5 as well as a fifth domain not previously observed as an ICAM domain. At this time, these newly identified rodent DNAs appeared to encode a rodent homolog of human ICAM-R, however alignment of 3' regions of these DNAs with other ICAMs proved difficult.

The subsequent isolation of a 1 kb cDNA clone from a rat spleen library, and amplification of an RT-PCR fragment indicated that a portion of both the cDNA and genomic clones had not been sequenced. Another RT-PCR amplification product (SEQ ID NO: 7) confirmed this omission. It was determined that a fragment of 177 bp was excised from the genomic and cDNA clones by EcoRI digestion of the clones to isolate these sequences from λ phage for DNA sequencing studies. Reanalysis of SEQ ID NOs: 5 and 6 in light of these other sequences permitted identification of more accurate and complete sequences for the originally isolated genomic and cDNA clones, presented in corrected form herein as SEQ ID NOs: 8 and 9.

In order to identify a complete coding sequence for ICAM-4, a rat brain cDNA (SEQ ID NO: 10) was isolated, and 5' end sequence determined by 5' rapid amplification of cDNA ends (5' RACE), the amplification product set forth in SEQ ID NO: 11. Combining information from the RT-PCR clone (SEQ ID NO: 7), the brain cDNA (SEQ ID NO: 10) and the RACE amplification product (SEQ ID NO: 11) permitted identification of the complete coding sequence for ICAM-4 (SEQ ID NO: 1).

The present invention is thus illustrated by the following examples. More particularly, Example 1 addresses cloning of a partial rodent ICAM-4 DNA. Example 2 describes Northern blot analysis of rodent ICAM-4 transcription. Example 3 describes isolation of a full length rodent ICAM-4 cDNA. Example 4 relates the *in situ* hybridization of rodent ICAM-4 in brain tissue. Example 5 addresses generation of ICAM-4 fusion proteins in prokaryotes. Example 6 describes production of monoclonal antibodies specific for rat ICAM-4/GST fusion proteins. Example 7 describes expression of soluble rat ICAM-4 proteins in a baculovirus expression system. Example 8 addresses production of monoclonal antibodies specific for rat ICAM-4 expressed in a baculovirus system. Example 9 describes immunocytochemical analysis of rat ICAM-4 expression. Example 10 relates cloning of a human genomic ICAM-4-encoding DNA. Example 11 addresses cloning of a human ICAM-4-encoding cDNA. Example 12 describes Northern analysis of human ICAM-4 expression. Example 13 describes generation of human ICAM-4/GST fusion proteins. Example 14 addresses production of monoclonal antibodies immunospecific for human ICAM-4. Example 15 describes development of a capture assay for determining the concentration of soluble ICAM-4 in a particular fluid. Example 16 applies the capture assay method in assessing ICAM-4 concentration in the serum of stroke patients. Example 17 relates to assessment of ICAM-4 transcription in a epilepsy model. Example 18 describes measurement of circulating ICAM-4 concentration as an assessment of various neurodegenerative disorders. Example 19 addresses cloning of a promoter region for human ICAM-4.

### EXAMPLE 1

### Cloning of Rat ICAM-Related DNA

### A. Isolation of a Rat Genomic ICAM-Related Domain 2 DNA

A rat genomic library constructed in λ EMBL3 was screened a with [³²P]-labeled probe generated by PCR from DNA encoding human ICAM-3 domain 2 The sequence of the probe is set forth in SEQ ID NO: 12. Library plaques were transferred to Hybond N+ nylon membranes (Amersham, Arlington Heights, IL). Screening of all cDNA and genomic libraries was performed according to standard protocols. Prehybridization and hybridizations were carried out in a solution of 40-50% formamide, 5X Denhardt's, 5X SSPE and 1.0% SDS at 42°C. Probes ([³²P]-labeled) were added at a concentration of 10⁵-10⁶ cpm/ml of hybridization solution. Following 16-18 hours of hybridization, nylon membranes were washed extensively at room temperature in 2X SSPE with 0.1 % SDS and subsequently exposed to X-ray film at -80°C overnight. Positive plaques were subjected to one or more rounds of hybridization to obtain clonal phage. DNA prepared from lysate of the positive clones was subcloned into pBS+ and sequenced.

A first genomic clone encoding a rat ICAM-related domain 2 was identified that was determined to be homologous to domain 2 regions in other ICAM family members (see for example, Table 1 of U.S. Patent Application Serial No. 08/102,852), yet was distinct from the previously reported nucleotide sequences for rat ICAM-1 [Kita, et al., *Biochem.Biophys.-Acta 1131*:108-110 (1992)] or mouse. ICAM-2 [Xu, *et al., J.Immunol. 149*:2560-2565 (1992)]. The nucleic acid and deduced amino acid sequences for this clone were disclosed in the co-pending parents to the present application as purportedly variant forms of rat ICAM-R and were set forth as SEQ ID NOs: 23 and 24, respectively, in U.S.S.N. 08/102,852. Herein, these same sequences are set out in SEQ ID NOs: 3 and 13, respectively.

A second, overlapping clone was also identified with the same probes and was determined to contain the ICAM domain 2 sequence of SEQ ID NO: 3 and 5' DNA encoding at least part of rat ICAM-1. The nucleic acid sequence for this clone was set forth in the co-pending parent to the present application as SEQ ID NO: 26 and is set forth herein as SEQ ID NO: 5. This second clone indicated that the ICAM-related gene fragment of the first clone and the gene encoding rat ICAM-1 are located on the same rat chromosome within 5 kb of each other.

### B. Isolation of Rat ICAM-Related cDNA

In order to identify a more complete protein coding sequence for the ICAM-related polypeptide, [³²P]-labeled DNA encoding the domain 2 sequence from the rat genomic clone identified in Section A (SEQ ID NO: 3), *supra*, was used to screen a number of cDNA libraries from various rat and mouse cell types, including rat macrophage (Clontech, Palo Alto, CA), peripheral blood lymphocyte (PBL) (Clontech), T cell (constructed in-house), and spleen (Clontech), and mouse PBL (Clontech), T cell (constructed in-house), and B cell (constructed in-house).

A single clone was identified in a rat spleen cDNA library (Clontech) which contained five Ig-like domains, four of which were homologous to domains 2 through 5 in both ICAM-1 and ICAM-R. Moreover, this clone included 3' DNA encoding an apparent fifth Ig-like domain which had not been previously identified in any other ICAM polypeptide. In addition, the clone contained an unusual 3' sequence subsequently determined to be a partial intron (discussed *infra*) located between domains 4 and 5, suggesting that the clone was the product of an immature or aberrantly spliced transcript. The presence of the unique domain and the determination that the 3' region did not properly align with other known ICAMs suggested that the ICAM-related DNA potentially encoded a novel rat ICAM polypeptide. The nucleic acid sequence for this clone was set forth in the parent to the present application as SEQ ID NO: 25; herein the nucleic acid sequence for this spleen cDNA clone is set forth in SEQ ID NO: 6.

### C. Re-analysis of Rat cDNA and Genomic DNAs

Subsequent to the August 5, 1993 filing of U.S. Patent Application Serial No. 08/102,852, it was determined that the partial rat spleen cDNA clone (SEQ ID NO: 25 in the parent and SEQ ID NO: 6 herein) and the rat liver genomic clone (SEQ ID NO: 26 of the parent and SEQ ID NO: 5 herein) were missing an internal 177 bp EcoRI fragment that was part of each of these clones but lost in a subcloning step when the library inserts were removed from the λ vector with *Eco*RI digestion and ligated into a sequencing vector. The observation that the cDNA and genomic clones might be missing a coding fragment became apparent upon alignment of the rat genomic and cDNA sequences with various RT-PCR amplification products, including SEQ ID NO: 7, which revealed a gap in the rat sequence.

Subsequent isolation and sequence alignment of a cDNA from a spleen library using the spleen cDNA clone (SEQ ID NO: 6) as a probe provided a first indication that a portion of the spleen cDNA and genomic clones were not sequenced. Further confirmation of this idea became apparent upon amplification of an RT-PCR fragment, spanning domains 3 through 5, using a 5' primer (RRD3 5 'Xho, containing a 5' *Xho*I restriction site to facilitate cloning) set out in SEQ ID NO: 14, and a 3' primer (RRD5 3 'Hind, containing a *Hind*III site to facilitate cloning) set out in SEQ ID NO: 15.
GAACTCGAGGCCATGCCTCCACTTTCC (SEQ ID NO: 14)
CCATAAGCTTTATTCCACCGTGACAGCCAC (SEQ ID NO: 15)
Alignment of these two DNAs clearly revealed that the cDNA and genomic clones had lost a fragment prior to sequencing; this idea was further supported following sequencing of the RT-PCR DNA discussed *infra.* It was concluded that restriction digestion with *Eco*RI to remove the cDNA and genomic fragments prior to sequencing resulted in the excision of a 177 bp fragment that was not detected visually in the agarose gel separation of the clones from the λ phage sequences. Subsequent sequence analysis confirmed the location of two *Eco*RI sites flanking a 177 bp fragment in both of the original clones.

The 177 bp *Eco*RI fragment is situated between nucleotides 719 and 896 in the rat partial cDNA clone as set out in SEQ ID NO: 9 and between nucleotides 2812 and 2989 in the partial genomic clone as set out in SEQ ID NO: 8.

### D. DNA Isolated by RT-PCR Clone

RT-PCR was utilized to generate more complete sequence information for the rat ICAM-related gene. Sequence information from the genomic clone (SEQ ID NO: 3) was used to design sense primers complementary to a region 5' of the protein coding region, as determined from the cDNA clone, and antisense primers designed complementary to coding sequences and regions 3' to the coding sequence in the cDNA clone (SEQ ID NO: 6).

Template cDNA for PCR reactions was prepared as follows. Approximately 2 µg of poly A⁺RNA isolated from rat spleen cells was denatured by heating at 65°C in a 10 µl volume. Following denaturation, 0.1 µl RNasin (Invitrogen, San Diego, CA), 5 µl 5X RTase Buffer (BRL, Bethesda, MD), 2 µl random hexamer (pd(N)6 at 100 µg/ml) (Pharmacia, Piscataway, NJ), 6 µl dNTPs (2 mM each) and 2 µl AMV RTase (BRL) were added and the reaction was incubated at 42°C for 60-90 min. Reactions were stored at -20°C until needed.

An initial series of experiments was conducted to identify oligonucleotides primer pairs that produced an amplification product in PCR reactions using rat spleen cDNA as the template. Various 5' sense primers were paired in PCR with a 3' primer which was designed to be complementary to an internal, coding sequence; the 3' primer was designated RRD2 3-1 and is set forth in SEQ ID NO: 16.
AACGTGCGGAGCTGTCTG (SEQ ID NO: 16)
(In the ultimately isolated RT-PCR product, SEQ ID NO: 7, *infra*, primer RRD2 3-1 corresponded to nucleotides 719 through 736.) Similarly,various 3' antisense primers were paired with a 5' primer designed complementary to another internal, coding sequence; the 5' primer in these reactions was designated RGen3900S and is set forth in SEQ ID NO: 17.
ACGGAATTCGAAGCCATCAACGCCAGG (SEQ ID NO: 17)
(In SEQ ID NO: 7, *infra,* primer RGen3900S corresponded to nucleotides 1719 through 1736.) Based on the size of the amplification products and the ability of these products to hybridize with the partial cDNA clone, one pair of primers was determined to be most efficient and was used in subsequent PCR amplifications. The 5' primer was designated RGen780S (SEQ ID NO: 18) and the 3' primer was designated RGen4550AS (SEQ ID NO: 19).
CATGAATTCCGAATCTTGAGTGGGATG (SEQ ID NO: 18)
ATAGAATTCCTCGGGACACCTGTAGCC (SEQ ID NO: 19)
(In SEQ ID NO: 7, *infra*, primer RGen780S corresponded to nucleotides 1 through 18, and primer RGen4550AS corresponded to nucleotides 2197 through 2214.)

This primer pair was used in PCR under a variety of conditions to optimize amplification. A total of 15 different PCR buffers that varied in pH and Mg⁺⁺ concentration were used at two different annealing temperatures, and a sample of the product from each reaction was separated on a 1 % agarose gel. Because no amplification product could be detected by visual inspection of the ethidium bromide stained gel from any of the reaction conditions, more sensitive Southern hybridization was employed to detect the PCR products.

Aliquots of the amplified. DNA were separated by electrophoresis, transferred to a Hybond N+ nylon membrane using conventional Southern blotting wicking techniques, and hybridized with the entire rat cDNA which was [³²P]-labeled. Hybridization conditions were essentially as described for the library screening procedure in Section A, *supra.* Autoradiography indicated that a small amount of DNA of approximately 2.2 kb had been generated in two of the reactions, and the remainder of the amplification product from the two reactions was separated on an agarose gel. The 2.2 kb region was eluted from the gel, even though no band was evident upon visual inspection, and used as a template in another PCR reaction using the same primers (SEQ ID NOs: 18 and 19), Tris-HCl buffer, pH 8.0, containing 1 mM Mg⁺⁺, and 55°C annealing temperature. The amplification product from the secondary PCR was visible in the gel and was eluted and cloned into a pBS⁺ plasmid (Stratagene, La Jolla, CA) for sequence analysis.

The resulting RT-PCR clone was determined to contain 2214 bp as set forth in SEQ ID NO: 7. The clone encoded domains 2 through 6 found in the rat spleen cDNA clone, an additional amino terminal domain 1, an additional carboxy terminal domain 7, and 164 bp of what appeared to be a further carboxy terminal domain 8. Immediately 5' to domain 1 was an additional 144 bp sequence presumed to have been derived from an intron between the leader and the first domain. This clone did not contain a 5' leader sequence or 3' transmembrane and cytoplasmic regions. In addition to the previously identified domain 6 in the spleen cDNA clone, the 7th and 8th domains in the RT-PCR clone supported the hypothesis that this clone was a novel rodent ICAM.

### EXAMPLE 2

### Northern Blot Analysis

In order to further investigate the possibility that the ICAM-related clones identified in Example 1 encoded a novel ICAM polypeptide as suggested by the unique Ig-like domains, tissue specific expression was examined by Northern blot analysis to permit comparison with the previously reported expression patterns of human ICAMs [ICAM-1, Dustin, *et al*., *J.Immunol.* 137:245-254 (1986); ICAM-2, Staunton, *et al*., *Nature* 339:61-64 (1989); ICAM-R, de Fourgerolles and Springer, *J.Exp.Med*. 175:185-190 (1992)].

Total cellular RNA from rat lung, brain, spinal cord, liver, digestive tract, thymus, lymph nodes, and spleen was prepared using STAT60 RNA isolation reagents (Tel-test "B", Inc, Friendswood, Texas) according to the manufacturer's suggested protocol. Poly A⁺ RNA was purified from total RNA using oligo dT cellulose columns. Approximately 5 µg of RNA derived from each tissue was separated on a 1% formaldehyde agarose gel, and transferred to hybond-C nitrocellulose membranes (Amersham).

A fragment of the rat spleen cDNA from Example 1 corresponding to domains 2 through 4 (nucleotides 1 through 724 in SEQ ID NO: 6) was subcloned into pBluescript SK⁺ (Stratagene) and an antisense riboprobe was generated by *in vitro* transcription using ³²P-labeled UTP and approximately 500 ng of linearized template according to a manufacturer's (Boehringer Mannheim, Indianapolis, IN) suggested protocol. The membrane-bound RNA was prehybridized in a solution containing 50% formamide; 5X SSC, 1X PE (50 mM Tris-HCl, pH 7.5, 0.1 % sodium pyrophosphate, 0.2% polyvinylpyrrolidone, 0.2% ficoll, 5 mM EDTA, 1% SDS) and 150 µg/ml denatured salmon sperm DNA. The radiolabeled probe was denatured by boiling and added to the prehybridization solution to a final concentration of 1 x 10⁶ cpm/ml. Hybridization was allowed to proceed for 16-18 hours at 65°C. The membranes were then washed at 65°C in 2X SSC containing 0.1 % SDS and subsequently exposed to X-ray film for 3-16 hours.

The Northern blot analysis indicated that the ICAM-related cDNA identified in Example 1 was expressed only in rat brain, a tissue specificity not previously reported for any other ICAM polypeptides. This expression pattern, in combination with the unique Ig-like domains not known to exist in other ICAM polypeptides, indicated that the ICAM-related clone was a novel member of the ICAM family of proteins, and was named ICAM-4.

The fact that the initially identified cDNA clones were detected in a rat spleen library suggested that a subset of cells in the spleen may express ICAM-4 at low levels. However, a properly spliced clone could not be detected in numerous hemopoietic cDNA libraries which led to doubt if ICAM-4 protein is actually expressed in tissue other than brain. One explanation for the detection of ICAM-4 cDNA in spleen is that the sensitivity of PCR may have amplified a trace amount of transcript even though these tissues do not express the encoded protein.

### EXAMPLE 3

### Isolation of Full Length Rat ICAM-4 cDNA

### A. Identification of a Rat Brain cDNA Clone

In view of the tissue specific expression of ICAM-4, brain tissue mRNA was utilized in an attempt to isolate a full length cDNA encoding ICAM-4. Two probes, one complementary to domains 1 through 2 and a second complementary to domains 3 through 5 of the spleen cDNA clone identified in Example 1 (SEQ ID NO: 7), were radiolabeled and used to screen a rat brain cDNA library in λgt10 which was previously constructed in-house. Hybridization conditions were as described in Example 1, and positive plaques were subjected to one or more rounds of screening to obtain clonal phage.

Nine positive clones were identified, two of which hybridized to both probes. The longest of the two clones, designated clone 7, contained 2550 bp encoding four of the five Ig-like domains found in the probe cDNA. In addition, clone 7 encoded four other Ig-like domains not found in the probe. Putative transmembrane and cytoplasmic domains were identified which were followed by a stop codon, a poly-adenylation signal, and a poly A tail. Clone 7 was lacking at least one 5' Ig-like domain as determined by comparison to the RT-PCR clone (SEQ ID NO: 7), and also lacked a leader sequence; re-screening of the library did not yield any longer clones which contained these sequences. The nucleic acid sequence for clone 7 is set forth in SEQ ID NO: 10.

### B. Determination of the 5' End

In order to isolate domain 1 and other 5' sequences, a PCR technique termed 5' Rapid Amplification of cDNA Ends (RACE) [PCR Protocols: A Guide to Methods and Applications, Innis, et al., (eds) Academic Press: New York (1990) pp:28-38] was employed using a 5' RACE kit (Clontech). This technique utilizes an internal primer paired with a second primer complementary to an adapter sequence ligated to the 5*'* end of cDNA library molecules. PCR with this primer pair will therefore amplify and facilitate identification of the intervening sequences. Overlapping sequence information can then be used to generate a complete sequence of the gene.

RACE-ready cDNA from rat brain (supplied with kit) was used in a PCR with the kit oligonucleotide and an antisense primer based on an internal ICAM-4 sequence. The 3' antisense primer, designated Spot714AS, was designed according to an ICAM-4 domain 4 sequence and is set forth in SEQ ID NO: 20.
CARGGTGACAAGGGCTCG (SEQ ID NO: 20)

The amplification product resulting from this primer pair was subsequently subjected to a secondary PCR using the same 5' kit primer paired with a 3' primer complementary to a region in ICAM-4 domain 1. The second 3' primer was designated RRACE2 and is set forth in SEQ ID NO: 21.
TATGAATTCAGTTGAGCCACAGCGAGC (SEQ ID NO: 21)

Each primer used in the secondary PCR contained an EcoRl site to facilitate cloning of the resulting amplification products into pBS⁺ (Stratagene). The resulting plasmid DNA which contained the 5 ' end of the gene was identified by hybridization to a rat ICAM-4 domains 1 and 2 probe, corresponding to nucleotides 1 through 736 in SEQ ID NO: 7. Partial sequence information for domain 1 and the hydrophobic leader was determined from the resulting amplification product.

The product from the 5 *'* RACE method was a DNA fragment 222 bp long containing 60 bp upstream of the initiating methionine residue, an 82 bp leader sequence, and an 80 bp sequence from domain 1. The amplification product is set forth in SEQ ID NO: 11.

### C. Full Length Sequence of Rat ICAM-4

A composite clone of the full length ICAM-4 was constructed from the sequence information derived from the 5' RACE method (SEQ ID NO: 11), the RT-PCR clone (SEQ ID NO: 7) and the brain cDNA clone 7 (SEQ ID NO: 10). The full length gene for rat ICAM-4 was determined to contain 2985 bp with a single open reading frame encoding a deduced 917 amino acid protein. A putative Kozak sequence is located upstream of the methionine residue in the leader sequence. A 27 amino acid hydrophobic leader sequence is followed by nine Ig-like domains, a transmembrane region and a 58 amino acid cytoplasmic tail. The composite ICAM-4 cDNA is set for in SEQ ID NO: 1, and the deduced amino acid sequence is set forth in SEQ ID NO: 2.

Like other ICAM polypeptides, ICAM-4 contains extracellular, transmembrane, and cytoplasmic domains. In the extracellular domain, the amino terminus of ICAM-4 is a leader sequence comprising amino acids 1 through 27 which is followed by nine immunoglobulin (Ig)-like domains, a characteristic unique to ICAM-4 in that ICAM-1, ICAM-2, and ICAM-R contain five, two, and five extracellular Ig-like domain, respectively. In ICAM-4, domain 1 comprises amino acids 28 through 118; domain 2 comprises amino acids 119 through 224; domain 3 comprises amino acids 225 through 321; domain 4 comprises amino acids 322 through 405; domain 5 comprises amino acids 406 through 488; domain 6 comprises amino acids 489 through 569; domain 7 comprises amino acids 570 through 662; domain 8 comprises amino acids 663 through 742; and domain 9 comprises amino acids 743 through 830. Within each domain, a characteristic "loop" structure is formed by a disulfide bond between cysteine residues located generally at opposite ends of the domain amino acid sequence. Other structural features of ICAM-4 include the transmembrane region comprising amino acids 831 through 859 and the cytoplasmic region comprising amino acids 860 through 917.

Comparison of amino acid sequence homology of each domain in rat ICAM-4 with the other members of the ICAM family was limited to the corresponding sequences of human ICAM-1, ICAM-2, and ICAM-R since sequence information for all three rodent homologs has not been previously reported. In the first domain, the rodent ICAM-4 shows 21, 30, and 28 percent identity with human ICAM-1, ICAM-2, and ICAM-R, respectively. The second domain is more conserved, with the amino acid percent identities being 60, 42 and 62 with ICAM-1, -2, and -3, respectively. Domains 3-5 show percent identities of 48, 49, and 40 with ICAM-1 and 60, 59 and 29 respectively for ICAM-R. Interestingly, rat ICAM-4 domains 6 through 8 are most homologous with domain 5 (ranging from 29-42% identical), possibly arising from a gene segment duplication event. The ninth and final extracellular domain aligns poorly with other ICAM domains but has 22% identity with the 3rd and 6th domains of human VCAM-1, another member of the Ig family of protein which participate in cell adhesion. The cytoplasmic tail is 58 amino acids long. This is longer than the other members of the ICAM family wherein human ICAM-1, -2, and -3 contain 28, 26, and 37 amino acids,respectively. As with the ninth domain, rat ICAM-4 cytoplasmic tail is . most homologous with the cytoplasmic tail of human VCAM-1, which contains only 19 amino acids. The membrane proximal 19 amino acids of rat ICAM-4 share 7 amino acid residues with VCAM-1 (37%).

Finally, functional binding to LPA-1 (CD11a/CD18) maps to the first domain in the ICAMs. Vonderheide *et al.,[J. Cell. Biol*., 125:215-222 (1994)] identified a sequence motif purportedly involved in integrin binding. Despite the relatively low homology between rat ICAM-4 and other ICAMs in domain 1, this binding sequence motif is conserved, suggesting that rat ICAM-4 may be a ligand for LFA-1 and perhaps other integrins.

### EXAMPLE 4

### In situ Hybridization in Brain Tissue

In order to localize the specific brain tissue which expressed ICAM-4, *in situ* hybridization with ICAM-4 domain 1 and ICAM-4 domains 3 through 4 anti-sense riboprobes was employed. The probes were labeled by *in vitro* transcription using ³⁵S-labeled UTP.

Frozen tissue sections of normal rat brain were fixed in 4% paraformaldehyde for 20 minutes, rinsed and dehydrated, and the fixed RNA denatured for 2 minutes in 2X SSC, 70% formamide at 70°C prior to hybridization. Tissue sections were hybridized overnight at 50°C in a solution containing 50% formamide, 0.3 M Nacl, 20 mM Tris-HCI, pH 7.4, 5 mM EDTA, 10% dextran sulfate, 1X Denhardt, 0.5 mg/ml yeast RNA,. 100 mM DTT and a probe concentration of 50,000 cpm/µl. Slides were washed once in 4X SSC, 10 mM DTT at room temperature for 60 minutes, once in 50% formamide, 2X SSC, 10 mM DTT at 60°C for 40 minutes, and once in each 2X SSC and 1X SSC for 30 minutes each at room temperature. Specificity of hybridization was determined in parallel experiments performed with the same protocol but also including a more stringent wash in 50% formamide, 1X SSC, 10 mM DTT at 60°C for 40 minutes. After washing, the slides were dipped in NTB2 emulsion (Kodak, Rochester, NY) and exposed from 2 to 21 days before being developed and counter-stained. Negative controls included sense probes generated from ICAM-4 domain 1 and ICAM-4 domain 3 through 4 sense riboprobes, in addition to a human immunodeficiency virus (HIV-1) riboprobe.

The signal detected in brain tissue was primarily. localized in the gray matter with the strongest signal in the cerebral cortex and hippocampus. The hybridization profile was consistent with ICAM-4 expression primarily in cerebral neurons.

### EXAMPLE 5

### Generation of ICAM-4 fusion proteins

Rat ICAM-4/glutathione S-transferase (GST) fusion proteins were generated using the prokaryote expression vector pGEX (Pharmacia, Alameda, CA) in order to generate monoclonal antibodies against specific ICAM-4 polypeptide fragments.

PCR primers corresponding to the 5' and3' ends of domain 1 and the 5' and 3' ends of domain 2 were used to amplify DNA fragments encoding the individual domains. The resulting fragments were separately cloned into an *Eco*RI site of pGEX-2T; DNA sequence analysis confirmed the correct orientation and reading frame. Transformants were subsequently screened for their ability to produce fusion protein of the appropriate molecular weight.

Both ICAM-4 domain 1/GST and ICAM-4 domain 2/GST fusion proteins remained in the insoluble fraction after the bacteria were lysed by sonication in PBS containing 1 % SDS. The insoluble protein fraction from 100 ml cultures were boiled in SDS loading dye and separated on a 10% preparative polyacrylamide-SDS gel. The gel was stained in ice cold 0.4 M KCl and the fusion protein bands were excised. Fusion proteins were electroeluted from the gel slices in dialysis tubing in buffer containing 25 mM Tris-HCl and 192 mM glycine. Approximate protein concentration was determined by OD₂₈₀ and purity of the preparation was determined on SDS-PAGE stained with Coomassie blue.

### EXAMPLE 6

### Production of Monoclonal Antibodies Against Rat ICAM-4/GST Fusion Proteins

Balb/c mice were immunized by subcutaneous injection with 40-50 µg ICAM-4 domain-2/GST fusion protein (described in Example 5) emulsified in Freund's complete adjuvant (FCA). Two weeks later, the mice were again immunized by subcutaneous injection with the same protein, emulsified however in Freund's incomplete adjuvant. Two final intraperitoneal immunizations given two weeks after the second immunization included soluble antigen with no adjuvant given at two week intervals. Serum from each immunized mouse was assayed by ELISA for its ability to specifically react with rat ICAM-4 produced by the baculovirus expression system described *infra*.

The spleen from mouse #1654 was sterilely removed and placed in 10 ml serum-free RPMI 1640. A single-cell suspension was formed by grinding the spleen tissue between frosted ends of two glass microscope slides submerged in serum free RPMI 1640 (Gibco, Burlington, Ottawa, Canada) supplemented with 2 mM L-glutamine, 1 mM sodium pyruvate, 100 units/ml penicillin, and 100 µg/ml streptomycin. The cell suspension was filtered through a sterile 70-mesh Nitex cell strainer (Becton Dickinson, Parsippany, NJ), and washed twice with RPMI followed by centrifuging at 200 x g for 5 minutes. The resulting pellet from the final wash was resuspended in 20 ml serum-free RPMI. Thymocytes taken from three naive Balb/c mice were prepared in an identical manner.

Prior to fusion, NS-1 myeloma cells were maintained in log phase growth in RPMI with 11 % Fetalclone serum (FBS) (Hyclone Laboratories, Logan, Utah) for three days. Once harvested, the cells were centrifuged at 200 x g for 5 minutes, and the pellet was washed twice as described in the foregoing paragraph. After washing, the cell suspension was brought to a final volume of 10 ml in serum free RPMI. A 20 µl aliquot was removed and diluted 1:50 with serum free RPMI, and a 20 µl aliquot of this dilution was removed, mixed with 20 µl 0.4% trypan blue stain in 0.85% saline (Gibco), loaded onto a hemacytometer (Baxter Healthcare, Deerfield, IL) and the cells counted. Approximately 2.425 x 10⁸ spleen cells were combined with 4.85 x 10⁷ NS-1 cells, the mixture centrifuged and the supernatant removed. The resulting pellet was dislodged by tapping the tube and 2 ml of 50% PEG-1500 in 75 mM Hepes, pH 8.0, (Boehringer Mannheim, Indianapolis, IN) was added with stirring over the course of 1 minute. Subsequently, an additional 14 ml serum free RPMI was added over 7 minutes. The cell suspension was centrifuged at 200 x g for 10 minutes and the supernatant discarded. The pellet was resuspended in 200 ml RPMI containing 15% FBS, 100 µM sodium hypoxanthine, 0.4 µM aminopterin, 16 µM thymidine (HAT) (Gibco), 25 units/ml IL-6 (Boehringer Mannheim) and 1.5 x 10⁶ thymocytes/ml. The suspension was first placed in a 225 cm² flask (Coming, Essex, United Kingdom) at 37°C for four hours before being dispensed into ten 96-well flat bottom tissue culture plates (Coming) at 200 µl/well. Cells in the plates were fed on days 3, 4, 5, and 6 post fusion by aspirating approximately 100 µl from each well with a 20 G needle (Becton Dickinson), and adding 100 µl/well plating medium described above except containing 10 units/ml IL-6 and lacking thymocytes.

The fusion plates were screened initially by antigen capture ELISA as follows. Immulon 4 plates (Dynatech, Cambridge, MA) were coated overnight at 4°C with 100 ng/well of either domain 1-GST or domain 2-GST fusion protein in 50 mM carbonate buffer. The plates were blocked with 100 µl/well 0.5% fish skin gelatin (Sigma, St. Louis, MO) in PBS for 30 minutes at 37°C. After blocking, the plates were washed 3X with PBS containing 0.05 % Tween 20 (PBST) and 50 µl/well of hybridoma supernatant from each fusion was added. After incubation at 37°C for 30 minutes, the plates were washed as described above, and 50 µl of a 1:3500 dilution of horseradish peroxidase-conjugated goat anti-mouse IgG (Fc) (Jackson ImmunoResearch, West Grove, Pennsylvania) was added. Plates were again incubated for 30 minutes and washed 4X with PBST. Substrate, 100 µl/well, consisting of 1 mg/ml o-phenylene diamine (Sigma) and 0.1 µl/ml 30% H₂O₂ in 100 mM citrate, pH 4.5, was added. The color reaction was allowed to proceed 10 minutes and quenched with the addition of 50 µl/well of 15% H₂SO₄. Absorbance at 490 nm was then determined on an automated plate reader (Dynatech).

Wells which were positive for domain 2-GST protein, but not for domain 1-GST protein, were then screened by ELISA against a Baculovirus supernatant (described *infra).* ELISA was performed as described above except that the Immulon 4 plates were initially coated overnight with Baculovirus supernatant diluted 1:4 in 50 mM carbonate buffer. Three wells (103A, 103B and 103F) were cloned two to three times, successively, by doubling dilution in RPMI, 15% FBS, 100 µM sodium hypoxanthine, 16 µM thymidine, and 10 units/ml IL-6. Wells of clone plates were scored visually after 4 days and the number of colonies in the least dense wells was recorded. Selected wells of each cloning were again assayed by ELISA after 7 to 10 days against either domain 1-GST protein and domain 2-GST protein, or Baculovirus supernatant.

The monoclonal antibodies produced by the hybridomas were isotyped by ELISA. Immulon 4 plates (Dynatech) were coated at 4°C with 50 µl/well goat anti-mouse IgA, IgG, or IgM (Organon Teknika, Durham, NC) diluted 1:5000 in 50 mM carbonate buffer, pH 9.6. Wells were blocked for 30 minutes at 37°C with 1 % BSA in PBS, washed 3X with PBST. A 1:10 dilution of hybridoma culture supernatant (50 µl) was added to each plate, incubated, and washed as above. After removal of the last wash, 50 µl horseradish peroxidase-conjugated rabbit anti-mouse IgG₁, G₂ₐ, G_{2b}, or G₃ (Zymed, San Francisco, CA) (diluted 1:1000 in PBST with 1% normal goat serum) was added. Plates were incubated as above, washed 4X with PBST and 100 µl substrate, was added. The color reaction was quenched after 5 minutes with addition of 50 µl 15% H₂SO₄, and absorbance at 490 nm determined on a plate reader (Dynatech).

Results indicated that antibodies 103A, 103B, and 103F were all IgG₁ isotype. These antibodies were subsequently used in immunocytochemical analyses, Western blotting, and for purification of protein expressed in baculovirus.

### EXAMPLE 7

### Baculovirus Expression of Rat ICAM-4

A baculovirus expression system (Invitrogen) was used to generate soluble protein corresponding to domains 1 through 6 of ICAM-4. Because the leader sequence for ICAM-4 was not known at the time, the expression construct was made containing the coding sequence for ICAM-4 fused 3' to the ICAM-1 leader sequence in proper reading frame. Specific details regarding construction of the ICAM-1/ICAM-4 expression plasmid is as follows.

Rat ICAM-1 DNA encoding the five Ig-like domains was amplified by PCR using primers which incorporated several features to facilitate construction of the fusion plasmid. The 5' oligonucleotide primer included *Hind*III and *Bgl*II sites, in addition to a consensus Kozak sequence upstream of the first methionine in the leader sequence. The 3' oligonucleotide primer included a coding sequence for six histidines followed by a stop codon and a *Hind*III cloning site. The PCR amplification product was cloned into a *Hind*III-digested pBS⁺ vector and sequence analysis confirmed the appropriate construction. An internal *Sma*I site in the ICAM-1 leader sequence and another *Sma*I site in the vector's multiple cloning region (3' to ICAM-1 Ig-like domain 5) were digested which removed most of the ICAM-1 coding sequence. After these manipulations, the linearized, blunt-ended vector contained a portion of the upstream multiple cloning region (those restriction sites 5' of the original *Hind*III site in the multiple cloning region), the Kozak sequence and most of the ICAM-1 leader sequence.

The coding sequence for rat ICAM-4 domains 1 through 6 was amplified by PCR utilizing primers designed to permit cloning of this sequence into the linearized vector described above. The 5' oligonucleotide primer included an *EcoRV* site and the codons needed to complete the ICAM-1 leader sequence. The 3' oligonucleotide primer included codons for six histidine residues, a stop codon, and *Hind*III and *Eco*RV restriction sites. The amplification product from this PCR was digested with *Eco*RV to produce a blunt-ended sequence which was then ligated into the blunt-ended *Sma*I-digested pBS⁺ linearized vector. The entire sequence containing the ICAM-1 leader sequence 5' to the ICAM-4 domains 1 through 6 was removed from the construct with *Bgl*II and *Hind*III digestion and the purified ICAM-1/ICAM-4 fusion sequence cloned directly into a *Bgl*II/*Hind*III- digested pBluesac m vector (Invitrogen).

Protein production by the recombinant virus was assayed for by ELISA, initially using immune sera from mice immunized with rat ICAM-4 domain-2/GST fusion protein described in Example 5. In later work, monoclonal antibodies generated from those mice were used to purify ICAM-4 protein produced by the recombinant baculovirus in SF9 cells.

### EXAMPLE 8

### Production of Monoclonal Antibodies Against Baculovirus-expressed Rat ICAM-4

Rat ICAM-4 domains 1-6 were expressed in the baculovirus expression system as described in Example 7. The recombinant protein was purified using monoclonal antibody 103A (as described in Example 6).

Briefly, 30 mg of purified monoclonal 103A (in 100 mM . sodium borate, 500 mM sodium chloride) were coupled to three grams of Activated Cyanogen Bromide Sepharose 4B (Pharmacia, Piscataway, NJ). Baculovirus supernatant containing recombinant rat ICAM-4 (domains 1-6) was loaded on the Sepharose column overnight at 4°C. The column was washed in calcium- magnesium-free phosphate buffered saline (CMF-PBS) and bound material was eluted in 50 mM citric acid, 500 mM NaCI pH 4.0. The sample was neutralized with 1/10 volume Tris pH 10 and stored at -20°C. The purified protein separated on SDS-PAGE appeared greater than 90% pure and migrated at approximately 80 kD.

Mice were immunized with the purified recombinant rat ICAM-4 domains 1-6 protein in a similar manner as described in Example 6. The spleen from mouse #1945 was used for fusion #127. The fusion protocol was as described in Example 6. The fusion wells were screened by ELISA on the recombinant ICAM-4 protein. The secondary screen included immunocyto-chemistry on rat brain sections (as below described in Example 9). Four additional antibodies specific for rat ICAM-4 were cloned out of this fusion: 127A, 127E, 127F and 127H. The immunocytochemical staining pattern of each antibody on rat brain sections was the same as observed with monoclonal antibody 103A (see Example 9). The monoclonal antibodies were tested for their ability to bind the D1/GST and D2/GST fusion proteins (described in Example 5). Monoclonal antibody 127A recognized the D1/GST fusion protein and 127H recognized the D2/GST fusion protein. These two distinct binding specificities along with the others that did not bind either GST protein suggest that at least 3 different epitopes were being recognized by the panel of antibodies. Hybridomas 127A and 127H were deposited May 31, 1995 and June 1, 1995, respectively, with the American Type Culture Collection, 12301 Parklawn Drive, Rockville, Maryland 20852, and assigned Accession Numbers HB11905 and HB11911, respectively.

### EXAMPLE 9

### Immunocytochemistry of Rat ICAM-4 Expression

Immunocytochemistry with monoclonal antibody 103A was performed to localize the protein production within the rat brain.

A brain was harvested from a normal adult female Lewis rat, sagittally sectioned, and washed in RNase-free 1X PBS on ice for 30 min. The brain sections were then placed in Tissue Tek II cryomolds (Miles Laboratories, Inc., Naperville, IL) with a small amount of O.C.T. compound (Miles, Inc., Elkhart, IN). The brains were centered in the cryomold, the cryomold filled with OCT compound, then placed in a container with 2-methylbutane (Aldrich Chemical Company, Inc., Milwaukee, WI) and the container placed in liquid nitrogen. Once the tissue and OCT compound in the cryomold were frozen, the blocks were stored at -80°C until sectioning.

The tissue was sectioned at 6 µm thickness, adhered to Vectabond (Vector Laboratories, Inc., Burlingame, CA) coated slides and allowed to air-dry at room temperature overnight until use. The sections were fixed in ethyl ether (Malinckrodt, Paris, KY) for 5 minutes at room temperature. Once the slides were removed from the ether, the reagent was allowed to evaporate. Each tissue section was blocked with 150 µl 50% Normal rat serum (Sigma) and 2% bovine serum albumin (BSA) (Sigma) in 1X PBS (made with sodium phosphates only) for 30 minutes at room temperature. After blocking, the solution was gently blotted from the sections and the purified supernatant antibody 103A (1.65 mg/ml) was diluted 1:10 in the blocking solution and 150 µl applied to each tissue section. The slides were placed in a humidity chamber and incubated at 4°C overnight.

The next day the antibody solution was blotted gently from the section and the slides washed three times in 1X PBS for four minutes in each wash. The excess PBS was aspirated from the slide and 100 µl of the secondary, rat anti mouse-biotin conjugated antibody (Jackson ImmunoResearch Laboratories), diluted 1:100 in a solution of 10% normal rat serum and 2% BSA in 1X PBS, applied to the tissues. Incubation was allowed to proceed for one hour at room temperature. The sections were washed two times in 1X PBS for four minutes in each wash, then 100 µl of ABC reagent from an Elite Rat IgG Vectastain ABC kit (Vector Laboratories, Inc., Burlingame, CA), prepared according to the product insert, was applied to each section. Incubation was allowed to proceed for 30 minutes at room temperature. After incubation, the slides were washed two times in 1X PBS (four minutes each wash) and 150 µl of Vector VIP Peroxidase Substrate Solution (Vector Laboratories, Inc., Burlingame, CA) applied to each section for approximately ten minutes. After color development, the sections were rinsed under running tap water for five minutes, counterstained with Mayer's hematoxylin (Sigma) for 20 seconds, and rinsed again in gently running tap water for five minutes. The slides were dehydrated across a graded series of ethanols, passed through xylene and mounted with Accumount 60 (Stephens Scientific, Riverdale, NJ).

Immunohistochemistry of rat brain sections strained with mAb 103A indicated that rat ICAM-4 is expressed in the neuronal cells of the hippocampus. Staining pattern suggested that the protein might be limited to the neuronal processes (dendrites). Brain sections stained in a similar manner with an irrelevant antibody or second step reagent alone do not show the distinct expression pattern seen with MAb 103A.

### EXAMPLE 10

### Cloning of a Human ICAM-4 Genomic DNA

During the cloning of rat ICAM-4 from genomic DNA, it was discovered that ICAM-4 and ICAM-1 were located within 5 kb of each other and this information was utilized in an attempt to clone the human homologue of ICAM-4.

Genome Systems Inc. (St. Louis, MO) amplified fragments in a human P1 library by PCR using human ICAM-1 domain 3 primers, a sense primer designed complementary to human ICAM-1 domain 3 (H-1/D3 S) and an antisense primer designed complementary to human ICAM-1 domain 3 (H-1/D3 AS). These primers are set forth in SEQ ID NOs: 22, and 23, respectively.

CCGGGTCCTAGAGGTGGACACGCA (SEQ ID NO: 22)
TGCAGTGTCTCCTGGCTCTGGTTC (SEQ ID NO: 23)

Two clones, designated 1566 and 1567, were identified and subjected to further analysis. Both P1 clones contained approximately 75-95 kb genomic DNA inserts. The clones were digested with B*am*H1, separated with agarose gel electrophoresis, and blotted onto nylon membranes. Southern blots hybridization were performed under either low stringency (30% formamide) or high stringency (60% formamide) at 42°C with human ICAM-1, ICAM-3 or rat ICAM-4 radiolabeled probes; other constituents of the hybridization solution were as described in Example 1. The low stringency hybridization series was washed at room temperature in 2X SSPE containing 0.1% SDS. The high stringency hybridization was washed at 65° C in 0.2X SSPE containing 0.1 % SDS. The washed membranes were exposed to X-ray film for 3.5 hours.

The differential hybridization indicated that human ICAM-1 was contained on a 5.5 kb *Bam*HI fragment while human ICAM-3 was located on a 4.0 kb and a 1.5 kb *Bam*H1 fragment. The human ICAM-1 and ICAM-R fragments were subcloned into pBS⁺ and their identity confirmed by limited sequence analysis.

A 7.0 kb *Bam*H1 fragment that hybridized with rat ICAM-4 under high stringency conditions was subcloned and further fragmented with *Rsa*I restriction digestion. Three *Rsa*I fragments that hybridized with rat ICAM-4 were identified and their sequences determined. Based on homology to rat ICAM-4, these fragments appeared to contain domains 2, 3, 4, 5 and part of domain 6.

### EXAMPLE 11

### Cloning of a Human ICAM-4 cDNA

The fragments of genomic DNA corresponding to domains 2-5 of human ICAM-4 (described in Example 10) were used as probes to screen a λgt10 Human hippocampus cDNA library (Clontech, Palo Alto, CA). The library screening protocol was essentially as described in Example 1.

The longest human ICAM-4 clone (#18) that was found in that library was only 992 bp (SEQ ID: 24) and corresponded to roughly the middle of the predicted 3 kb gene. The 992 bp DNA insert from clone 18 (SEQ ID: 24) was used as a probe to screen a λZAPII human hippocampus cDNA library (Stratagene, La Jolla, CA). This library yielded a number of positive clones. The longest clone, #34, was 2775 bp (SEQ ID: 25). Based on alignments to the full length rat ICAM-4, it was predicted that this clone was missing the leader sequence and approximately 30 bp at the 5 end of domain 1. The poly A⁺ tail at the 3' end was missing, but the translation stop codon was present.

A fragment of DNA corresponding to the first 3 domains (nucleotides 1 to 840 in clone #34) was used as a probe to screen a λgt10 cDNA library derived from human cerebral cortex(Clontech, Palo Alto, CA). One clone, 16-1 (SEQ ID: 26), was identified as having 1557 bp, and included 39 bp of 5' untranslated DNA, a leader sequence and sequence information through the fifth domain. Overlapping clones #34 (SEQ ID: 25) and 16-1 (SEQ ID: 26) were used to generate a composite of the full length human ICAM-4 sequence (SEQ ID: 27).

The full length gene is 2927 bp long and encodes a 924 amino acid protein. The ICAM-4 nucleotide sequence is set out in SEQ ID NO: 27 and the amino acid sequence is set out in SEQ ID NO: 28. Sequence alignment with the full length rat ICAM-4 gene (SEQ ID: 11) revealed an overall DNA sequence identity of 82% and 85% identity at the amino acid level. The apparent 9 Ig like extracellular domain structure of the protein is conserved between rat and human. The leader sequence extends from amino acid 1 to 28; domain 1 from amino acid 29 to 117; domain 2 from amino acid 118 to 224; domain 3 from amino acid 225 to 320; domain 4 from amino acid 321 to 405; domain 5 from amino acid 406 to 488; domain 6 from amino acid 489 to 570; domain 7 from amino acid 571 to 663; domain 8 from amino acid 664 to 743; domain 9 from amino acid 744 to 837; the transmembrane region from amino acid 838 to 857 and the cytoplasmic tail from amino acid 858 to 924.

Human ICAM-4 (HuICAM-4), in addition to being genetically linked to ICAM-1 and ICAM-R, also showed certain common structural features that group them together as a family of molecules. A domain by domain alignment of HuICAM-4 with the other members of the ICAM family shows varying degrees of homology. Domain 1 amino acid sequence of HuICAM-4 is 21, 30 and 26% identical to domain 1 of ICAMs 1, 2 and 3 respectively. Domain 2 of HuICAM-4 is 61, 39 and 62% identical to ICAMs 1, 2 and 3 respectively. Domain 3 of HuICAM-4 is 50 and 65% identical to ICAMs 1 and 3 respectively. Domain 4 of HuICAM-4 is 54 and 64% identical to ICAMs 1 and 3 respectively. Domains 5-8 of HuICAM-4 are most homologous to the fifth domains of ICAM=1 and 3, with percent identities ranging from 33-47 for ICAM-1 domain 5 and 21-31 for ICAM-R domain 5. The ninth domain of HuICAM-4 aligns poorly with the other members of the ICAM family but is homologous to domains 3 (24% identical) and 6 (23% identical) of HuICAM-1.

### EXAMPLE 12

### Northern Analysis of Human ICAM-4 Expression

Two human multiple tissue Northern (MTN) blots were purchased from Clontech (Palo Alto, CA). These contained at least 2 µg of poly A⁺ RNA from 16 different human tissues (as shown in Table 1) run on a denaturing formaldehyde 1.2% agarose gel and transferred to nylon membrane. The blots were prehybridized for three hours at 42°C in 10 ml of a solution containing 5X SSPE, 10X Denhardts solution, 50% formamide, 2% SDS and 100 µg/ml denatured salmon sperm DNA. The blots were hybridized in the above solution with a radiolabeled human ICAM-4 probe (clone #18, SEQ ID: 24) for 16 hours at 42°C. The following day, the blots were washed in a solution of 0.1X SSC/0.1% SDS at room temperature followed by a wash at 50°C. The blots were exposed to x-ray film at -80°C for 24 hours. Results of the analysis are shown below in Table 1.

Only the lane containing RNA from the brain hybridized to the ICAM-4 probe, giving a single band at approximately 3 kb. Longer exposure (five days) confirmed that only the brain had a detectable level of message. In order to determine if all lanes contained comparable amounts of RNA of comparable quality, the same blot was hybridized with a control β-actin probe. Blots were stripped of the ICAM-4 probe by treatment with a boiling solution of 0.1% SDS for 15 minutes, and subsequently probed in a similar manner with a β actin probe provided by the manufacturer. Except for minor variation in amounts, all lanes were shown to have good quality RNA.

**TABLE 1**

| **Northern Tissue Analysis of Human ICAM-4 Expression** | | |
|---|---|---|
| | **PROBE** | |
| Tissue | ICAM-4 | β-Actin |
| Heart | - | + + + |
| Brain | + | + + |
| Placenta | - | + + + |
| Lung | - | +++ |
| Liver | - | + + + |
| Skeletal muscle | - | ++++ |
| Kidney | | +++ |
| Pancreas | - | ++ |
| Spleen | - | +++ |
| Thymus | - | +++ |
| Prostate | - | +++ |
| Testis | - | +++ |
| Ovary | - | + + + |
| Small intestine | | +++ |
| Colon | - | +++ |
| Peripheral blood leukocyte | - | + + + |

Two additional Northern blots were purchased from Clontech that contained poly A⁺ RNA from 16 different sub-regions of human brain (as shown in Table 2). Blots were probed in a manner similar to that used for tissue analysis and results are shown in Table 2. RNA quality and quantity loaded was checked by probing the blots with a β actin probe.

All of the regions that showed ICAM-4 expression are part of the telencephalon, with the exception of the thalamus which is considered part of the diencephalon. The hippocampus and cerebral cortex appeared to have the highest level of expression. The transcript size in all cases was the same, 3 kb. The exquisite tissue distribution of the ICAM expression suggests that the promoter region may contain elements that confer the observed developmental and spatial expression of the gene product. The utility of such information may provide insight into the understanding of control of neural gene expression in general.

**TABLE 2**

| **Northern Brain Cell Type Analysis of Human ICAM-4 Expression** | | |
|---|---|---|
| | **PROBE** | |
| Brain Region | ICAM-4 | β-Actin |
| Amygdala | ++ | +++ |
| Caudate nucleus | ++ | + + + |
| Corpus callosum | + | +++ |
| Hippocampus | ++ | + + + |
| Hypothalamus | - | +++ |
| Substantia nigra | - | +++ |
| Subthalamic nucleus | + | +++ |
| Thalamus | + | +++ |
| Cerebellum | - | +++ |
| Cerebral cortex | + + + | + + + |
| Medulla | - | + + + |
| Spinal cord | - | +++ |
| Occipital pole | ++ + | + + + |
| Frontal lobe | ++ | +++ |
| Temporal lobe | + + | + + + |
| Putamen | ++ | +++ |

### EXAMPLE 13

### Generation of Human ICAM-4/IgG fusion Proteins

Human ICAM-4/IgG1 fusion proteins expression plasmids were constructed to produce proteins for generating monoclonal antibodies and for use in adhesion assays to identify potential ICAM-4 ligands. Two constructs were made; the first included DNA encoding domains 1-3 of HuICAM-4 and the second, domains 4-8. Both were linked to the Fc region of human IgG1 in vector pDCS1 that uses the cytomegalovirus (CMV) promoter to drive expression and the signal sequence from IgG4 to facilitate secretion of the molecules.

PCR primers (shown below as SEQ ID NOs: 29-32) were designed to generate the necessary DNA fragments for sub-cloning. The "sense" primer for the 5' end of domain 1 (HI4-D1(s), SEQ ID NO: 29) was designed to fill in 30 base pairs of domain 1 missing in clone #34. Primers HI4-D1(S) (SEQ ID NO: 29) and HI4-D3(AS) (SEQ ID NO: 30) were used to generate a DNA fragment encoding domains 1-3 of human ICAM-4, corresponding to a region in SEQ ID NO: 1 from nucleotide 130 to nucleotide 996. Primers HI4-D3(S) (SEQ ID NO: 31) and HI4-D8(AS) (SEQ ID NO: 32) were used to generate a DNA fragment encoding domains 4-8 of human ICAM-4, corresponding to a region in SEQ ID NO: 30 from nucleotide 997 to nucleotide 2268. Each 5' primer encoded a *Bam*HI restriction site (GGATCC, indicated in bold below) and each 3' (antisense) primer contained a XhoI site (CTCGAG, indicated in bold below) to facilitate subcloning 5' to the IgG1 gene. All oligonucleotides contain spacer nucleotides (underlined, below) at the 5' end to permit restriction digestion.

HI4-D1(S) (SEQ ID NO: 29)
GTACTTACAGGATCCGCGGTCTCGCAG-
GAGCCCTTCTGGGCGGACCTACAGCCTGCGTGGCGTTC

HI4-D3(AS) (SEQ ID NO: 30)
ATTTCTCTCGAGGATGGTCACGTTCTCCCGG

HI4-D4(S) (SEQ ID NO: 31)
ATTTCTGGATCCTACAGCTTCCCGGCACCACTC

HI4-D8(AS) (SEQ ID NO: 32)
ATTTCTCTCGAGTTCCACGCCCACAGTGACGG

PCR reactions were carried out in a 50 µl volume using buffers supplied by Perkin Elmer with the AmpliTaq enzyme. Primers were added at a final concentration of 10 µg/ml and all four dNTPs were included at 2 mM. The reactions were continued through 30 cycles of denaturation (94°C for four minutes), annealing (50°C for two minutes) and extension (72°C for one minute). PCR products were visualized on agarose gels and an aliquot of each reaction was used to subclone the PCR products into vector pCRII (Invitrogen, SanDiego, CA). Sequence analysis was performed to detect possible errors resulting from the amplification process and to confirm proper orientation. Appropriate clones were digested with *Bam*HI and *Xho*I and fragments separated with agarose gel electrophoresis. Purified fragments were ligated into a pDCS 1 vector previously digested with *Bam*HI and *Xho*I and the resulting plasmids were sequenced to confirm proper orientation and reading frame.

Human ICAM-4 domains 1-3 and 4-8/IgG1 fusion proteins were obtained following transient transfection of the expression plasmids into COS7 cells and isolation of the secreted protein from the culture media. Transfection was carried out as follows. Adherent COS7 cells at approximately 50-60% confluence were washed with CMF-PBS and subsequently contacted with 10-15 µg of plasmid DNA in 7.5 ml serum-free DMEM media (Gibco, Gaithersburg, MD) containing 6 µl of 0.25 M chloroquine (Sigma, St. Louis, MO). An additional 7.5 ml of serum-free media containing 150 µl of DEAE dextran (50 mg/ml) (Sigma, St. Louis, MO) were added and the plates incubated 2-3 hours before the media was removed and replaced with 10% DMSO (Mallinckrodt, McGaw Park, Illinois) in PBS. After a one minute incubation, the DMSO solution was removed and replaced with fresh media containing 5 % FBS. Each transfection included multiple plates, and media from cells expressing the same protein were pooled for protein isolation.

Media were collected every three days over the course of 3-4 harvests. Proteins were purified using a 0.4 - 0.,8 ml Procep A column (Bioprocessing Ltd, England) pre-equilibrated with 35 mM Tris, 150 mM NaCI, pH 7.5. Culture media was loaded onto the column two times at a flow rate of less than 60 column volumes per hour. The column was washed one time with each of 20 column volumes of Tris/Nacl buffer; 20 column volumes of 0.55 M diethanolamine, pH 8.5, and 20 column volumes of 50 mM citric acid, pH 5.0. The fusion proteins were eluted into one ml fractions using 50 mM citric acid pH 3.0 and each fraction was neutralized with 1/10 volume 1 M Tris, pH 9.5. Protein concentration was determined by OD_{280,} and purity was determined using SDS-PAGE.

A significant contamination from bovine IgG (present in the FBS) was noted. Even though the domains 1-3 fusion protein was predicted to be smaller than the domains 4-8 fusion protein, both migrated at approximately 90 kD. One possible explanation for the observation is that the smaller domains 1-3 fusion protein may be more heavily glycosylated than the larger domains 4-8 fusion protein.

In addition to use of the purified proteins for monoclonal antibody production, described below, the proteins will also be used in adhesion assays to identify ICAM-4 ligands.

### EXAMPLE 14

### Monoclonal Antibody Production

The purified protein described in Example 13 was utilized to generate monoclonal antibodies using an immunization protocol as described in Example 6.

The spleen from mouse #2250 (immunized with HuICAM-4 D1-3/IgG1) was used for fusion 172 and the spleen from mouse #2272 (immunized with HuICAM-4 D4-8/IgG1) was used for fusion 173. The fusion protocol utilized was as described in Example 6. Fusion plates were screened by ELISA (essentially as described in Example 6) using each HuICAM-4/IgG1 fusion protein. Fusion well supematants that recognized the immunogen protein, and no other, were considered for cloning. Immunocytochemistry on human hippocampus sections was used as a secondary screen.

One primary clone from each fusion was positive by immunocy-tochemistry and was cloned. One of the two clones failed to grow upon cloning, leaving only one candidate to pursue, clone 173E which was derived from the HuICAM-4 D4-8/IgG1 immunized mouse. Hybridoma 173E was deposited June 1, 1995 with the American Type Culture Collection, 12301 Parklawn Drive, Rockville, Maryland 20852, and assigned Accession Number HB11912.

From another fusion derived from a mouse immunized with a soluble ICAM-4 fragment corresponding to domains 1-3, six clones (179A, 179B, 179D, 179H, 179I, and 179K) were found to be specific for HuICAM4 domains 1 through 3 (D1-3). All six antibodies in the 179 series bound to the dendritic processes in the dentate gyrus, as well as the polymorphic and pyramidal cell layers. The monoclonal antibody 179A stained neuronal cell bodies from these areas in addition to the dendritic processes. The hybridoma cell lines producing antibodies 179I and 179H were deposited on June 10, 1996 with the American Type Culture Collection, 12301 Parklawn Drive, Rockville Maryland, 20852 and assigned Accession Numbers HB 12123 and HB 12124, respectively.

Additional fusions are similarly performed to generate other antibodies specifically immunoreactive with particular ICAM-4 regions.

### EXAMPLE 15

### Capture Assay Development

The six monoclonal antibodies from fusion 179 were tested in various combinations for their ability to capture and detect soluble ICAM-4 in solution. The assay, as described below, was established in order to evaluate soluble ICAM-4 levels in human fluids in relation to normal and disease conditions.

Antibody 179I was coated on Immulon 4 (Dynatech) 96 well plates at 3 µg/ml, 125 µl/well for two hours at 37°C. The antibody solution was removed by aspiration and the wells were blocked for 30 minutes at room temperature with 300 µl of blocking solution containing 5% Teleostean gelatin in calcium-free, magnesium-free PBS (CMF-PBS). The blocking solution was removed by aspiration, a 100 µl of sample fluid diluted in Omni Diluent (CMF-PBS, 1 % gelatin, and 0.05% Tween 20) was added to each well, and the mixture incubated at 37°C for 30 minutes. The plates were washed three times with PBST (CMF-PBS, 0.05% Tween 20). Antibody 179H was biotinylated at 1.5 mg/ml using NHS-LC-Biotin (Pierce) following suggested manufacturer's protocol, diluted 1:2000, and added to the wells (100 µl/well). The resulting mixture was incubated for 30 minutes at 37°C and the plates washed three times with PBST. Streptavidin-HRP (Pierce) was added (100 µl, 0.25 µg/ml) to each well and this mixture incubated at 37°C for 30 minutes. The plates were washed four times with PBST before addition of 100 µl of Tetramethylbenzidine (Sigma) (10 mg/ml stock in DMSO) diluted 1:100 in buffered substrate (13.6 g/L sodium acetate trihydrate, pH to 5.5 with 1 M citric acid, with 150 µl/L 30% hydrogen peroxide added just prior to developing). The reaction was allowed to develop for 30 minutes at room temperature in the dark, after which the reaction was stopped with addition of 50 µl/well 15% H₂SO₄. The absorbance was read at 450 nm.

Results indicated that the assay was capable of detecting soluble HuICAM-4 D1-3 recombinant protein at a concentration as low as 5-10 ng/ml with the linear portion of the curve being in the 10 - 100 ng/ml range. No cross-reactivity to HuICAM4 D4-8 was observed when this protein region was tested at 1 and 10 µg/ml.

### EXAMPLE 16

### Assessment of Soluble ICAM-4 in Serum from Stroke Patients

In order to assess the role of ICAM-4 in neurologic diseases and conditions, serum from twenty-eight patients suffering from acute stroke and twenty young healthy volunteers (not age matched) was assayed as described above for differences in serum concentration of soluble ICAM-4.

Results indicated that serum from the healthy volunteers had no detectable level of ICAM-4. Twenty out of twenty-eight acute stroke patients, however, had detectable levels of soluble ICAM-4. The signal from the positive stroke patients corresponded to a range of 5-38 ng/ml of the standard (soluble ICAM-4 D1-3 recombinant protein).

### EXAMPLE 17

### ICAM-4 mRNA Levels in Hippocampus in a Rat Model of Epilepsy

- Levels of rat ICAM-4 mRNA expressed were assessed in hippocampus of rats treated in a manner to create a kindling epileptogenesis 'animal model [Lothman, *et al., Brain Res*. 360:83-91 (1985)]. In the model, the rat hippocampus is stimulated with a series of subconvulsive electric shocks through an electrode implanted in the region of the brain which gradually elicits severe behavioral seizures. The kindling process involves twelve stimulations per day administered every other day for eight days. Once fully kindled, a single stimulus can elicit behavioral seizures and histologic changes that are similar to human epilepsy. Fully kindled rats received two stimulations per day over a two week period and animals were sacrificed 24 hours after the last stimulation. The hippocampus was removed and dissected for RNA preparation.

Total RNA was prepared from each sample using the guanid-inium/phenol/chloroform extraction procedure [Chomezynski and Sacchi, *Anal. Biochem.* 162:156-159 (1987)]. RNA was separated on denaturing formaldehyde agarose gels, transferred to nylon membranes, and hybridized with radiolabelled rat ICAM-4 and glyceraldehyde-3-phosphate dehydrogenase (GAPDH) specific DNA probes. GAPDH is a basally expressed gene that is commonly used as a control to detect lane to lane variation in the amount of RNA loaded on a gel. Fluctuations in the ratio of the ICAM-4/GAPDH are interpreted as changes in the level of ICAM-4 expression. Hybridizing bands for ICAM-4 and GAPDH were quantitated with a phosphorimager and a ratio of ICAM-4/GAPDH determined.

The ratio of ICAM-4/GAPDH was significantly higher in the control animals that were not kindled (n=5) compared to the kindled test group (n=5), suggesting that ICAM-4 was down regulated as a consequence of the kindling process. It should be noted, however, that the control group did not undergo any sham treatment so the possibility exists that ICAM-4 mRNA levels were modulated in response to the surgical treatment associated with kindling.

### EXAMPLE 18

### Serum ICAM-4 Concentration as a Marker for Neurodegenerative Disorders

Circulating serum concentrations of ICAM-4 were assessed as a possible indicator for various neurodegenerative disorders. Serum and/or plasma samples from anonymous donors were assayed as described in Example 16 above and compared to samples drawn from control donors with no previous history of neurological disorders.

### Control Donors

In order to establish a baseline average for circulating ICAM-4 in normal healthy individuals, serum samples from 100 donors were examined. The results showed that twelve individuals (12%) had circulating levels of ICAM-4 greater than 10 ng/ml. Of these twelve, the ICAM-4 concentration in five samples averaged 10-20 ng/ml, three samples showed an average ICAM-4 concentration of 20-100 ng/ml, two samples showed ICAM-4 levels between 100-500 ng/ml, and two samples contained ICAM-4 at a concentration in excess of 500 ng/ml.

Samples were taken at the same time from both donors with very high levels at varying timepoints over an eight month period to assess the stability of the observations over time. It was observed that over a period of months, the readings did fluctuate. No medical information was available on these donors, making correlations with the ICAM-4 levels and the physical well-being of the donors not possible. When both serum and plasma samples were prepared from the same individual, no difference was observed in the level of ICAM-4 present.

This observation indicated that an assay for soluble ICAM-4 would be versatile in its use of either serum or plasma. In addition, the results indicated that ICAM-4 is very stable in blood, suggesting that an elevated level of ICAM-4 as a result of some pathological state probably would not be transient. Finally, because of the apparent stability of ICAM-4 in a blood environment, assays for soluble ICAM-4 can utilize blood bank samples thus reducing the need for fresh blood with each assay.

In order to determine if the methods of collection and/or storage affected the observations, the stability of ICAM-4 serum was assessed by treating samples from the one individual with the highest level of circulating ICAM-4 in a variety of ways followed by a measurement of the levels of ICAM-4. Neither incubation at 37°C for 24 hours nor from one to three freeze/thaw cycles altered the level of detectable ICAM-4 in the serum.

### Donors with Epilepsy

The serum concentration of ICAM-4 in samples from twenty patients with Temporal Lobe Epilepsy (TLE) was measured and compared to serum samples from control group patients that had experienced Grand Mal Seizures (38 different patients), Syncope (8 patients) or were normal healthy donors (20 individuals). The assay method described in Example 15 was again employed and the results expressed as ng/ml relative to the internal standard used for the assay, soluble HuICAM-4 D1-3 recombinant protein (described in Example 13).

Serum from all 20 patients with TLE had measurable levels of ICAM-4 with an average of approximately 140 ng/ml. In serum samples from all 3 control groups, including the Grand Mal Seizure group, ICAM-4 concentration averaged below 10 ng/ml. These observations suggest that an individual's ICAM-4 serum level may represent a biochemical marker which can distinguish between focused seizures, like those experienced in TLE, and more generalized Grand Mal Seizures.

### Donors with AIDS

Serum concentration of ICAM-4 in the sera from a limited number of AIDS patients was also examined. The patients were grouped according to CD4 counts and the presence of any signs of dementia. A first group comprised sixteen early stage, asymptomatic patients with CD4 counts greater than 500 were tested. A second group comprised seven later stage patients with CD4 counts less than 300; signs of dementia were not determined for this group. The last group comprised nine late stage AIDS patients, each showing signs of dementia.

The results showed that serum samples from four of the sixteen (25%) early stage, asymptomatic patients had detectable levels of soluble ICAM-4; three of the four samples had an ICAM-4 concentration in excess of 500 ng/ml. Four of the seven (57%) serum samples from later stage patients were also positive for ICAM-4, with two of the four having ICAM-4 concentrations in excess of 500 ng/ml. Samples from the late stage patients showing signs of dementia had no detectable levels of ICAM-4. The results of this preliminary study suggest that ICAM-4 may be an early marker of the neurodegeneration associated with AIDS dementia.

### Donors with Other Neurodegenerative Diseases .

The results from the study of serum from epilepsy and AIDS donors suggest that ICAM-4 levels in the blood may reflect damage to the neurons that normally express it. There are a number of other neurologic diseases that might also show, as part of their etiology, damage to specific ICAM-4 expressing neurons that could result in changes in the serum concentration of ICAM-4 in the periphery.

For example, Alzheimer's disease is associated with extensive neuronal damage in the regions of the telencephalon where ICAM-4 is expressed. Assessment of ICAM-4 levels in serum from patients with the Early-onset Familial forms of the disease, as well as patients with the sporadic form of the disease, may provide a marker for the various stages of the disease thereby permitting assessment of possible therapeutic interventions.

As another example, because other cortical dementias, such as Pick's disease, diffuse cortical Lewy body disease, and frontal lobe degeneracy, are sometimes mistaken for Alzheimer's, but may be distinguishable from each other and from Alzheimer's disease through serum ICAM-4 analysis. As another example, serum ICAM-4 concentration in . patients suffering from a subcortical dementia, including Parkinson's disease, Huntington's disease, and progressive supranuclear, may be elevated as a result of common pathological indications of this class of disorders.

As another example, a number of the primary psychiatric disorders, such as depression, schizophrenia and psychosis, are characterized in part by degrees of neurodegeneration that might be associated with detectable levels of ICAM-4 in the blood.

As another example, elevated levels of ICAM-4 may be associated with a number of nongenetic dementias arising from infections, vasculitis, metabolic and nutritional disorders (e.g., thyroid, vitamin B12 deficiency), vascular disorders (multiple infarct, lacunar state, Binswanger's disease), toxic encephalopathies (e.g., exposure to carbon monoxide, heavy metals or other industrial pollutants) and tumors.

### EXAMPLE 19

### Cloning and Analysis of Human ICAM-4 Upstream Regulatory DNA

ICAM-4 gene expression is spatially and temporally regulated, with expression limited to the most anterior or ventral region of the brain, the telencephalon. In an attempt to identify gene sequences responsible for the restricted transcriptional regulation of ICAM-4, the nucleotide region 5' to human ICAM-4 coding sequences was examined.

A 2607 base pair *Bam*HI/PstI fragment derived from a 7.0 kb genomic *Bam*HI fragment (described in Example 10) was sequenced and found to contain 1684 nucleotides upstream of the ATG start codon. The complete sequence for this upstream region is set out in SEQ ID NO: 33. With respect to the position of the ICAM-4 coding region, the "A" in ATG start codon (numbered in SEQ ID NO: 33 as nucleotides 1685-1687) is designated the + 1 nucleotide and the nucleotide immediately 5' to the A⁺¹ nucleotide is designated -1. Thus the entire sequence is shown as extending from nucleotide -1684 to nucleotide +3, corresponding to numbering in the Sequence Listing nucleotide 1 to nucleotide 1687.

Based on the genomic HuICAM-4 sequence, oligonucleotides were synthesized and used in PCR to generate DNA molecules of various lengths within the upstream regulatory region. Each oligonucleotide set out in Table 3 contained a spacer region (shown in italics) approximately 6-10 bp to allow enzymatic digestion of the PCR product, an *Nhe*I or *Hind*III restriction site (shown in bold), and a specific hybridization primer sequence (underlined). The oligonucleotide names contain numbers that designate its 'location within the upstream regulatory region. In the PCR amplifications, oligonucleotides were paired as shown in Table 4 to generate DNA fragments containing specific regions of the upstream regulatory region.

The restriction sites and spacer region generated within each oligonucleotide allowed for enzymatic digestion and subsequent directional cloning of individual PCR products into the pGL3 Basic Vector (Promega, Madison, WI) which contains a luciferase reporter gene immediately downstream of a multiple cloning site (MCS). Promoter activity cloned into the MCS region of the vector drives expression of the luciferase reporter gene in transfected cell lines, and light production from expressed luciferase can be measured as an indicator of promoter activity. The pGL3 Basic Vector has

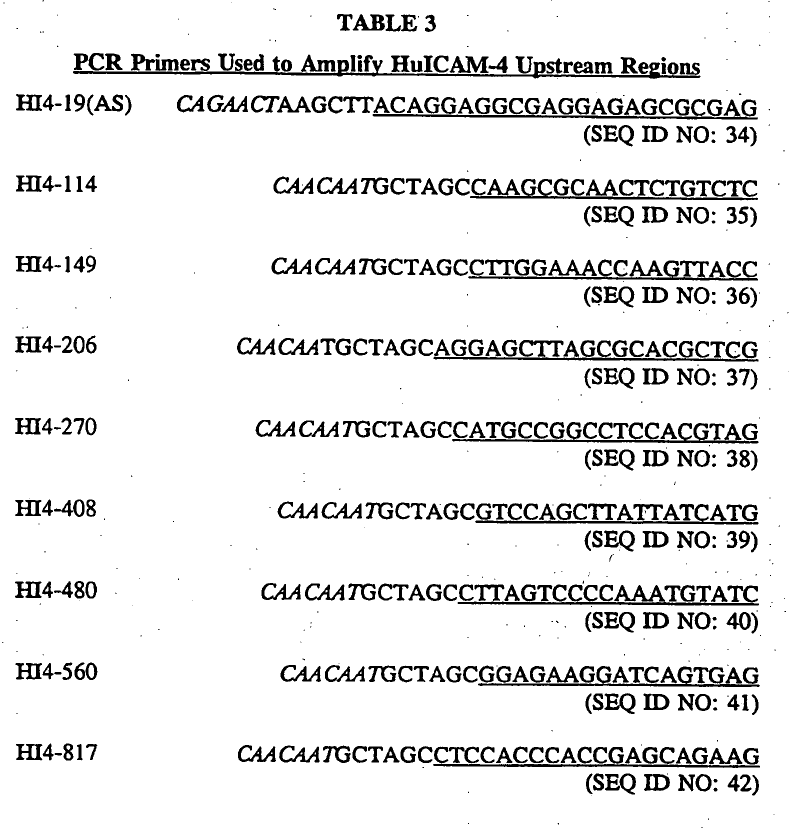

no promoter and therefore served as the negative control, while a pGL3 vector containing an SV40 promoter served as a positive control. The sequence of each expression construct was verified by restriction analysis and DNA sequencing.

Plasmids containing each of the amplified sequences described in Table 4 were transfected into mammalian cells using a Transfection MBS Mammalian Transfection Kit (Stratagene, La Jolla, CA) according to manufacturer's suggested protocol. Each plasmid was introduced into two different cell lines, COS 7 and NT2 Precursor Cells (Ntera2/D1 from Stratagene). COS 7 cells are a commonly used simian fibroblast-like cell line transformed with SV40 making them well suited for driving expression of a gene under control of the SV40 promoter in cells transfected with the positive control pGL3 Promoter Vector. NT2 precursor cells are a committed neuronal precursor cell line, and while they do not express ICAM-4, they may be more representative of a cell type that does express ICAM-4.

**TABLE 4**

| **Primers Paired and Regions Amplified** | |
|---|---|
| Oligonucleotide Pairs | Corresponding Upstream Regulatory Region |
| HI4-19 (AS) with HI4-114 | -19 → -114 |
| HI4-19 (AS) with HI4-149 | -19 → -149 |
| HI4-19 (AS) with HI4-206 | -19 → -206 |
| HI4-19 (AS) with HI4-270 | -1,9 → -270 |
| HI4-19 (AS) with HI4-408 | -19 → -408 |
| HI4-19 (AS) with HI4-480 | -19 → -480 |
| HI4-19 (AS) with HI4-560 | -19 → -560 |
| HI4-19 (AS) with HI4-817 | -19 → -817 |

Each well of a 6 well flat bottom tissue culture plate (intron) was seeded with 2.5x10⁵ cells. Transfections of COS 7 and NT2 cells were done side by side in duplicate using 5 µg of plasmid DNA for each well. The cells were cultured at 37°C for 48 hours, lysed and assayed for luciferase activity with a Luciferase Assay System (Promega).

Results of the experiment, summarized in Table 5, indicate a high level of promoter activity contained within the -408 through -19 and -480 through -19 regions of the upstream regulatory region of ICAM-4 in NT2 cells. Because NT2 cells are of neuronal origin, they may express certain transcription factors recognizing the ICAM-4 promoter that are not found in other cell types. The highest level of promoter activity in COS cell transfectants was obtained with the plasmid containing nucleotides -560 through -19. While the positive control pGL3 Promoter Vector worked well in COS cells, it showed very low promoter activity in NT2 cells, thus illustrating a cell type specific preference for certain promoter sequences.

**TABLE 5**

| **Promoter Activity of 5' ICAM-4 Regions** | | |
|---|---|---|
| Upstream Region | Luminescence | |
| | COS | NT2 |
| - 114 through -19 | 0.003 | 0.376 |
| -149 through -19 | 0.008 | 0.628 |
| -206 through -19 | 0.443 | 0.622 |
| -270 through -19 | 0.056 | 1.140 |
| -408 through -19 | 0.401 | 7.970 |
| -480 through -19 | 0.274 | 4.630 |
| -560 through -19 | 3.227 | 1.232 |
| -817 through -19 | 0.035 | 4.453 |
| pGL3 Promoter Vector | 29.070 | 0.063 |
| pGL3 Basic Vector | 0.008 | 0.014 |

Since neither COS 7 or NT2 cells normally express ICAM-4, the same experiment will be repeated using primary cultured rat hippocampal neurons which do express ICAM-4 and necessarily express transcriptional machinery required for ICAM-4 promoter activity. By transfecting the individual promoter constructs described herein; as well as others, into the more natural environment, it may be possible to identify more precisely which nucleotides in the upstream regulatory region are responsible for tight regulation of the ICAM-4 gene in the brain.

The foregoing illustrative examples relate to presently preferred embodiments of the invention and numerous modifications and variations thereof will be expected to occur to those skilled in the art. Thus only such limitations as appear in the appended claims should be placed upon the scope of the present invention.

Preferred features of the invention are included, as follows:
1. A method of screening for neuropathology in an individual comprising the steps of:
   a) obtaining a fluid sample from the individual;
   b) contacting the sample with an antibody specifically immunoreactive with ICAM-4;
   c) quantitating the level of ICAM-4/antibody binding in the sample; and
   d) comparing the level of ICAM-4/antibody binding in the sample to the level of ICAM-4/antibody binding in individuals known to be free of neuropathology,
2. The method of feature 1, wherein the fluid sample is serum.
3. The method of feature 1, wherein the fluid sample is plasma.
4. The method of feature 1, wherein the fluid sample is cerebrospinal fluid.
5. The method of feature 1, wherein the ICAM-4/antibody binding is quantitated by radioimmunoassay (RIA).
6. The method of feature 1, wherein the ICAM-4/antibody binding is quantitated by enzyme-linked immunosorbent assay (ELISA).
7. The method according to any one of features 1 through 6, wherein the neuropathology is epilepsy.
8. The method according to any one of features 1 through 6, wherein the neuropathology is dementia associated with AIDS progression.
9. The method according to any one of features 1 through 6, wherein the neuropathology is Alzheimer's disease.
10. The method according to any one of features 1 through 6, wherein the neuropathology is a cortical dementia.
11. The method according to feature 10, wherein the cortical dementia is selected from the group consisting of Pick's disease, diffuse cortical Lewy body disease, and frontal lobe degeneracy.
12. The method according to any one of features 1 through 6, wherein the neuropathology is a subcortical dementia.
13. The method according to feature 12, wherein the subcortical dementia is selected from the group consisting of Parkinson's disease, Huntington's disease, and progressive supranuclear.
14. The method according to any one of features 1 through 6, wherein the neuropathology is a primary psychiatric disorder.
15. The method according to feature 14, wherein the primary psychiatric disorder is selected from the group consisting of depression, schizophrenia and psychosis.
16. The method according to any one of features 1 through 6, wherein the neuropathology is a nongenetic dementia.
17. The method according to feature 16, wherein the nongenetic dementia arises from a condition selected from the group consisting of infection, vasculitis, a metabolic disorder, a nutritional disorder, a vascular disorder, toxic encephalopathies, and tumors.

## Claims

1. A method of screening for neuropathology in an individual comprising the steps of:
a) contacting a fluid sample obtained from the individual with an antibody specifically immunoreactive with ICAM-4;
b) quantitating the level of ICAM-4/antibody binding in the sample; and
c) comparing the level of ICAM-4/antibody binding in the sample to the level of ICAM-4/antibody binding in individuals known to be free of neuropathology,
wherein the neuropathology is selected from dementia associated with AIDS progression, Alzheimer's disease, cortical dementia, subcortical dementia, a primary psychiatric disorder or a non-genetic dementia.

2. The method of claim 1, wherein the fluid sample is serum.

3. The method of claim 1, wherein the fluid sample is plasma.

4. The method of claim 1, wherein the fluid sample is cerebrospinal fluid.

5. The method of claim 1, wherein the ICAM-4/antibody binding is quantitated by radioimmunoassay (RIA).

6. The method of claim 1, wherein the ICAM-4/antibody binding is quantitated by enzyme-linked immunosorbent assay (ELISA).

7. The method according to any one of claims 1 through 6, wherein the neuropathology is dementia associated with AIDS progression.

8. The method according to any one of claims 1 through 6, wherein the neuropathology is Alzheimer's disease.

9. The method according to any one of claims 1 through 6, wherein the neuropathology is a cortical dementia.

10. The method according to claim 9, wherein the cortical dementia is selected from the group consisting of Pick's disease, diffuse cortical Lewy body disease, and frontal lobe degeneracy.

11. The method according to any one of claims 1 through 6, wherein the neuropathology is a subcortical dementia.

12. The method according to claim 11, wherein the subcortical dementia is selected from the group consisting of Parkinson's disease, Huntington's disease, and progressive supranuclear palsy.

13. The method according to any one of claims 1 through 6, wherein the neuropathology is a primary psychiatric disorder.

14. The method according to claim 13, wherein the primary psychiatric disorder is selected from the group consisting of depression, schizophrenia and psychosis.

15. The method according to any one of claims 1 through 6, wherein the neuropathology is a nongenetic dementia.

16. The method according to claim 15, wherein the nongenetic dementia arises from a condition selected from the group consisting of infection, vasculitis, a metabolic disorder, a nutritional disorder, a vascular disorder, toxic encephalopathies, and tumors.


SEQUENCE LISTING

(1) GENERAL INFORMATION:

(i) APPLICANT: Gallatin, W. Michael
Kilgannon, Patrick D.

(ii) TITLE OF INVENTION: ICAM-4 Materials and Methods

(iii) NUMBER OF SEQUENCES: 42

(iv) CORRESPONDENCE ADDRESS:
(A) ADDRESSEE: Marshall, O'Toole, Gerstein, Murray & Borun
(B) STREET: 233 South Wacker Drive, 6300 Sears Tower
(C) CITY: Chicago
(D) STATE: Illinois
(E) COUNTRY: United States of America
(F) ZIP: 60606-6402

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: US 07/827,689
(B) FILING DATE: 27-JAN-1992

(vii) PRIOR APPLICATION DATA:
(A) APPLICATION NUMBER: US 07/889,724
(B) FILING DATE: 26-MAY-1992

(vii) PRIOR APPLICATION DATA:
(A) APPLICATION NUMBER: US 07/894,061
(B) FILING DATE: 05-JUN-1992

(vii) PRIOR APPLICATION DATA:
(A) APPLICATION NUMBER: US 08/009,266
(B) FILING DATE: 22-JAN-1993

(vii) PRIOR APPLICATION DATA:
(A) APPLICATION NUMBER: US 08/102,852
(B) FILING DATE: 05-AUG-1993

(vii) PRIOR APPLICATION DATA:
(A) APPLICATION NUMBER: US 08/245,295
(B) FILING DATE: 18-MAY-1994

(vii) PRIOR APPLICATION DATA:
(A) APPLICATION NUMBER: US 08/485,604
(B) FILING DATE: 07-JUN-1995

(viii) ATTORNEY/AGENT INFORMATION:
(A) NAME: WILLIAMS, JR. JOSEPH A.
(B) REGISTRATION NUMBER: 38,659
(C) REFERENCE/DOCKET NUMBER: 27866/33321

(ix) TELECOMMUNICATION INFORMATION:
(A) TELEPHONE: 312-474-6300
(B) TELEFAX: 312-474-0448
(C) TELEX: 25-3856

(2) INFORMATION FOR SEQ ID NO:1:

(i) SEQUENCE CHARACTERISTICS:
(A) LENGTH: 2988 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 61..2814

(xi) SEQUENCE DESCRIPTION: SEQ ID NO:1:

AATTCGATCA CTCGCGCTCC CCTCGCCTTC TGCGCTCTCC CCTCCCTGGC AGCGGCGGCA 60

ATG CCG GGG CCT TCA CCA GGG CTG CGC CGA ACG CTC CTC GGC CTC TGG 108
Met Pro Gly Pro Ser Pro Gly Leu Arg Arg Thr Leu Leu Gly Leu Trp
1               5                   10                  15

GCT GCC CTG GGC CTG GGG ATC CTA GGC ATC TCA GCG GTC GCG CTA GAA 156
Ala Ala Leu Gly Leu Gly Ile Leu Gly Ile Ser Ala Val Ala Leu Glu
            20                  25                  30

CCT TTC TGG GCG GAC CTT CAG CCC CGC GTG GCG CTC GTG GAG CGC GGG 204
Pro Phe Trp Ala Asp Leu Gln Pro Arg Val Ala Leu Val Glu Arg Gly
        35                  40                  45

GGC TCG CTG TGG CTC AAC TGC AGC ACT AAC TGT CCG AGG CCG GAG CGC 252
Gly Ser Leu Trp Leu Asn Cys Ser Thr Asn Cys Pro Arg Pro Glu Arg
    50                  55                  60

GGT GGC CTG GAG ACC TCG CTA CGC CGA AAC GGG ACC CAG AGG GGT CTG 300
Gly Gly Leu Glu Thr Ser Leu Arg Arg Asn Gly Thr Gln Arg Gly Leu
65                  70                  75                  80

CGC TGG CTG GCT CGA CAG CTG GTG GAC ATC CGA GAG CCT GAA ACC CAG 348
Arg Trp Leu Ala Arg Gln Leu Val Asp Ile Arg Glu Pro Glu Thr Gln
                85                  90                  95

CCG GTC TGC TTC TTC CGC TGC GCG CGC CGC ACA CTC CAA GCG CGT GGG 396
Pro Val Cys Phe Phe Arg Cys Ala Arg Arg Thr Leu Gln Ala Arg Gly
            100                 105                 110

CTC ATC CGA ACT TTC CAG CGA CCG GAT CGG GTA GAG CTA GTG CCT CTG 444
Leu Ile Arg Thr Phe Gln Arg Pro Asp Arg Val Glu Leu Val Pro Leu
        115                 120                 125

CCT CCT TGG CAG CCT GTA GGT GAG AAC TTC ACC TTG AGC TGC AGG GTC 492
Pro Pro Trp Gln Pro Val Gly Glu Asn Phe Thr Leu Ser Cys Arg Val
    130                 135                 140

CCG GGG GCA GGA CCC CGA GCG AGC CTC ACA TTG ACC TTG CTG CGA GGC 540
Pro Gly Ala Gly Pro Arg Ala Ser Leu Thr Leu Thr Leu Leu Arg Gly
145                 150                 155                 160

GGC CAG GAG CTG ATT CGC CGA AGT TTC GTA GGC GAG CCA CCC CGA GCT 588
Gly Gln Glu Leu Ile Arg Arg Ser Phe Val Gly Glu Pro Pro Arg Ala
                165                 170                 175

CGG GGT GCG ATG CTC ACC GCC ACG GTC CTG GCG CGC AGA GAG GAT CAC 636
Arg Gly Ala Met Leu Thr Ala Thr Val Leu Ala Arg Arg Glu Asp His
            180                 185                 190

AGG GCC AAT TTC TCA TGC CTC GCG GAG CTT GAC CTG CGG CCA CAC GGC 684
Arg Ala Asn Phe Ser Cys Leu Ala Glu Leu Asp Leu Arg Pro His Gly
        195                 200                 205

TTG GGA CTG TTT GCA AAC AGC TCA GCC CCC AGA CAG CTC CGC ACG TTT 732
Leu Gly Leu Phe Ala Asn Ser Ser Ala Pro Arg Gln Leu Arg Thr Phe
    210                 215                 220

GCC ATG CCT CCA CTT TCC CCG AGC CTT ATT GCC CCA CGA TTC TTA GAA 780
Ala Met Pro Pro Leu Ser Pro Ser Leu Ile Ala Pro Arg Phe Leu Glu
225                 230                 235                 240

GTG GGC TCA GAA AGG CCG GTG ACT TGC ACT TTG GAT GGA CTG TTT CCT 828
Val Gly Ser Glu Arg Pro Val Thr Cys Thr Leu Asp Gly Leu Phe Pro
                245                 250                 255

GCC CCA GAA GCC GGG GTT TAC CTC TCT CTG GGA GAT CAG AGG CTT CAT 876
Ala Pro Glu Ala Gly Val Tyr Leu Ser Leu Gly Asp Gln Arg Leu His
            260                 265                 270

CCT AAT GTG ACC CTC GAC GGG GAG AGC CTT GTG GCC ACT GCC ACA GCT 924
Pro Asn Val Thr Leu Asp Gly Glu Ser Leu Val Ala Thr Ala Thr Ala
        275                 280                 285

ACA GCA AGT GAA GAA CAG GAA GGC ACC AAA CAG CTG ATG TGC ATC GTG 972
Thr Ala Ser Glu Glu Gln Glu Gly Thr Lys Gln Leu Met Cys Ile Val
    290                 295                 300

ACC CTC GGG GGC GAA AGC AGG GAG ACC CAG GAA AAC CTG ACT GTC TAC 1020
Thr Leu Gly Gly Glu Ser Arg Glu Thr Gln Glu Asn Leu Thr Val Tyr
305                 310                 315                 320

AGC TTC CCG GCT CCT CTT CTG ACT TTA AGT GAG CCA GAA GCC CCC GAG 1068
Ser Phe Pro Ala Pro Leu Leu Thr Leu Ser Glu Pro Glu Ala Pro Glu
                325                 330                 335

GGA AAG ATG GTG ACC GTA AGC TGC TGG GCA GGG GCC CGA GCC CTT GTC 1116
Gly Lys Met Val Thr Val Ser Cys Trp Ala Gly Ala Arg Ala Leu Val
            340                 345                 350

ACC TTG GAG GGA ATT CCA GCT GCG GTC CCT GGG CAG CCC GCT GAG CTC 1164
Thr Leu Glu Gly Ile Pro Ala Ala Val Pro Gly Gln Pro Ala Glu Leu
        355                 360                 365

CAG TTA AAT GTC ACA AAG AAT GAC GAC AAG CGG GGC TTC TTC TGC GAC 1212
Gln Leu Asn Val Thr Lys Asn Asp Asp Lys Arg Gly Phe Phe Cys Asp
    370                 375                 380

GCT GCC CTC GAT GTG GAC GGG GAA ACT CTG AGA AAG AAC CAG AGC TCT 1260
Ala Ala Leu Asp Val Asp Gly Glu Thr Leu Arg Lys Asn Gln Ser Ser
385                 390                 395                 400

GAG CTT CGT GTT CTG TAC GCA CCT CGG CTG GAT GAC TTG GAC TGT CCC 1308
Glu Leu Arg Val Leu Tyr Ala Pro Arg Leu Asp Asp Leu Asp Cys Pro
                405                 410                 415

AGG AGC TGG ACG TGG CCA GAG GGT CCA GAG CAG ACC CTC CAC TGC GAG 1356
Arg Ser Trp Thr Trp Pro Glu Gly Pro Glu Gln Thr Leu His Cys Glu
            420                 425                 430

GCC CGT GGA AAC CCT GAG CCC TCC GTG CAC TGT GCA AGG CCT GAC GGT 1404
Ala Arg Gly Asn Pro Glu Pro Ser Val His Cys Ala Arg Pro Asp Gly
        435                 440                 445

GGG GCG GTG CTA GCG CTG GGC CTG TTG GGT CCA GTG ACC CGT GCC CTC 1452
Gly Ala Val Leu Ala Leu Gly Leu Leu Gly Pro Val Thr Arg Ala Leu
    450                 455                 460

GCG GGC ACT TAC CGA TGT ACA GCA ATC AAT GGG CAA GGC CAG GCG GTC 1500
Ala Gly Thr Tyr Arg Cys Thr Ala Ile Asn Gly Gln Gly Gln Ala Val
465                 470                 475                 480

AAG GAT GTG ACC CTG ACT GTG GAA TAT GCC CCA GCG CTG GAC AGT GTA 1548
Lys Asp Val Thr Leu Thr Val Glu Tyr Ala Pro Ala Leu Asp Ser Val
                485                 490                 495

GGC TGC CCA GAA CGT ATT ACT TGG CTG GAG GGG ACA GAG GCA TCG CTT 1596
Gly Cys Pro Glu Arg Ile Thr Trp Leu Glu Gly Thr Glu Ala Ser Leu
            500                 505                 510

AGC TGT GTG GCA CAC GGG GTC CCA CCA CCT AGC GTG AGC TGT GTG CGC 1644
Ser Cys Val Ala His Gly Val Pro Pro Pro Ser Val Ser Cys Val Arg
        515                 520                 525

TCT GGA AAG GAG GAA GTC ATG GAA GGG CCC CTG CGT GTG GCC CGG GAG 1692
Ser Gly Lys Glu Glu Val Met Glu Gly Pro Leu Arg Val Ala Arg Glu
    530                 535                 540

CAC GCT GGC ACT TAC CGA TGC GAA GCC ATC AAC GCC AGG GGA TCA GCG 1740
His Ala Gly Thr Tyr Arg Cys Glu Ala Ile Asn Ala Arg Gly Ser Ala
545                 550                 555                 560

GCC AAA AAT GTG GCT GTC ACG GTG GAA TAT GGT CCC AGT TTT GAG GAG 1788
Ala Lys Asn Val Ala Val Thr Val Glu Tyr Gly Pro Ser Phe Glu Glu
                565                 570                 575

TTG GGC TGC CCC AGC AAC TGG ACT TGG GTA GAA GGA TCT GGA AAA CTG 1836
Leu Gly Cys Pro Ser Asn Trp Thr Trp Val Glu Gly Ser Gly Lys Leu
            580                 585                 590

TTT TCC TGT GAA GTT GAT GGG AAG CCG GAA CCA CGC GTG GAG TGC GTG 1884
Phe Ser Cys Glu Val Asp Gly Lys Pro Glu Pro Arg Val Glu Cys Val
        595                 600                 605

GGC TCG GAG GGT GCA AGC GAA GGG GTA GTG TTG CCC CTG GTG TCC TCG 1932
Gly Ser Glu Gly Ala Ser Glu Gly Val Val Leu Pro Leu Val Ser Ser
    610                 615                 620

AAC TCT GGT TCC AGA AAC TCT ATG ACT CCT GGT AAC CTG TCA CCG GGT 1980
Asn Ser Gly Ser Arg Asn Ser Met Thr Pro Gly Asn Leu Ser Pro Gly
625                 630                 635                 640

ATT TAC CTC TGC AAC GCC ACC AAC CGG CAT GGC TCC ACA GTC AAA ACA 2028
Ile Tyr Leu Cys Asn Ala Thr Asn Arg His Gly Ser Thr Val Lys Thr
                645                 650                 655

GTC GTC GTG AGC GCG GAA TCA CCG CCA CAG ATG GAT GAA TCC AGT TGC 2076
Val Val Val Ser Ala Glu Ser Pro Pro Gln Met Asp Glu Ser Ser Cys
            660                 665                 670

CCG AGT CAC CAG ACA TGG CTG GAA GGA GCC GAG GCT ACT GCG CTG GCC 2124
Pro Ser His Gln Thr Trp Leu Glu Gly Ala Glu Ala Thr Ala Leu Ala
        675                 680                 685

TGC AGT GCC AGA GGC CGC CCC TCT CCA CGC GTG CGC TGT TCC AGG GAA 2172
Cys Ser Ala Arg Gly Arg Pro Ser Pro Arg Val Arg Cys Ser Arg Glu
    690                 695                 700

GGT GCA GCC AGG CTG GAG AGG CTA CAG GTG TCC CGA GAG GAT GCG GGG 2220
Gly Ala Ala Arg Leu Glu Arg Leu Gln Val Ser Arg Glu Asp Ala Gly
705                 710                 715                 720

ACC TAC CTG TGT GTG GCT ACC AAC GCG CAT GGC ACG GAT TCA CGG ACC 2268
Thr Tyr Leu Cys Val Ala Thr Asn Ala His Gly Thr Asp Ser Arg Thr
                725                 730                 735

GTC ACT GTG GGT GTG GAA TAC CGG CCT GTG GTG GCT GAG CTG GCA GCC 2316
Val Thr Val Gly Val Glu Tyr Arg Pro Val Val Ala Glu Leu Ala Ala
            740                 745                 750

TCG CCC CCA AGC GTG CGG CCT GGC GGA AAC TTC ACT CTG ACC TGC CGT 2364
Ser Pro Pro Ser Val Arg Pro Gly Gly Asn Phe Thr Leu Thr Cys Arg
        755                 760                 765

GCA GAG GCC TGG CCT CCA GCC CAG ATC AGC TGG CGC GCG CCC CCG GGA 2412
Ala Glu Ala Trp Pro Pro Ala Gln Ile Ser Trp Arg Ala Pro Pro Gly
    770                 775                 780

GCT CTC AAC CTC GGT CTC TCC AGC AAC AAC AGC ACG CTG AGC GTG GCG 2460
Ala Leu Asn Leu Gly Leu Ser Ser Asn Asn Ser Thr Leu Ser Val Ala
785                 790                 795                 800

GGT GCC ATG GGC AGC CAT GGT GGC GAG TAT GAG TGC GCA GCC ACC AAT 2508
Gly Ala Met Gly Ser His Gly Gly Glu Tyr Glu Cys Ala Ala Thr Asn
                805                 810                 815

GCG CAT GGG CGC CAC GCA CGG CGC ATC ACG GTG CGC GTG GCC GGT CCA 2556
Ala His Gly Arg His Ala Arg Arg Ile Thr Val Arg Val Ala Gly Pro
            820                 825                 830

TGG CTG TGG GTC GCT GTG GGC GGT GCG GCA GGG GGC GCG GCG CTG CTG 2604
Trp Leu Trp Val Ala Val Gly Gly Ala Ala Gly Gly Ala Ala Leu Leu
        835                 840                 845

GCC GCA GGG GCC GGC CTG GCC TTC TAC GTG CAG TCC ACC GCT TGC AAG 2652
Ala Ala Gly Ala Gly Leu Ala Phe Tyr Val Gln Ser Thr Ala Cys Lys
    850                 855                 860

AAG GGA GAG TAC AAC GTC CAG GAG GCT GAG AGC TCA GGC GAG GCG GTG 2700
Lys Gly Glu Tyr Asn Val Gln Glu Ala Glu Ser Ser Gly Glu Ala Val
865                 870                 875                 880

TGT CTC AAT GGC GCG GGC GGG ACA CCG GGT GCA GAA GGC GGA GCA GAG 2748
Cys Leu Asn Gly Ala Gly Gly Thr Pro Gly Ala Glu Gly Gly Ala Glu
                885                 890                 895

ACC CCC GGC ACT GCC GAG TCA CCT GCA GAT GGC GAG GTT TTC GCC ATC 2796
Thr Pro Gly Thr Ala Glu Ser Pro Ala Asp Gly Glu Val Phe Ala Ile
            900                 905                 910

CAG CTG ACA TCT TCC TGAGCCTGTA TCCAGCTCCC CCAGGGGCCT CGAAAGCACA 2851
Gln Leu Thr Ser Ser
        915

GGGGTGGACG TATGTATTGT TCACTCTCTA TTTATTCAAC TCCAGGGGCG TCGTCCCCGT 2911

TTTCTACCCA TTCCCTTAAT AAAGTTTTTA TAGGAGAAAA AAAAAAAAAA AAAAAAAAAA 2971

AAAAAAAAAA AAAAAAA 2988

(2) INFORMATION FOR SEQ ID NO:2:

(i) SEQUENCE CHARACTERISTICS:
(A) LENGTH: 917 amino acids
(B) TYPE: amino acid
(D) TOPOLOGY: linear

(ii) MOLECULE TYPE: protein

(xi) SEQUENCE DESCRIPTION: SEQ ID NO:2:

Met Pro Gly Pro Ser Pro Gly Leu Arg Arg Thr Leu Leu Gly Leu Trp
1               5                   10                  15

Ala Ala Leu Gly Leu Gly Ile Leu Gly Ile Ser Ala Val Ala Leu Glu
            20                  25                  30

Pro Phe Trp Ala Asp Leu Gln Pro Arg Val Ala Leu Val Glu Arg Gly
        35                  40                  45

Gly Ser Leu Trp Leu Asn Cys Ser Thr Asn Cys Pro Arg Pro Glu Arg
    50                  55                  60

Gly Gly Leu Glu Thr Ser Leu Arg Arg Asn Gly Thr Gln Arg Gly Leu
65                  70                  75                  80

Arg Trp Leu Ala Arg Gln Leu Val Asp Ile Arg Glu Pro Glu Thr Gln
                85                  90                  95

Pro Val Cys Phe Phe Arg Cys Ala Arg Arg Thr Leu Gln Ala Arg Gly
            100                 105                 110

Leu Ile Arg Thr Phe Gln Arg Pro Asp Arg Val Glu Leu Val Pro Leu
        115                 120                 125

Pro Pro Trp Gln Pro Val Gly Glu Asn Phe Thr Leu Ser Cys Arg Val
    130                 135                 140

Pro Gly Ala Gly Pro Arg Ala Ser Leu Thr Leu Thr Leu Leu Arg Gly
145                 150                 155                 160

Gly Gln Glu Leu Ile Arg Arg Ser Phe Val Gly Glu Pro Pro Arg Ala
                165                 170                 175

Arg Gly Ala Met Leu Thr Ala Thr Val Leu Ala Arg Arg Glu Asp His
            180                 185                 190

Arg Ala Asn Phe Ser Cys Leu Ala Glu Leu Asp Leu Arg Pro His Gly
        195                 200                 205

Leu Gly Leu Phe Ala Asn Ser Ser Ala Pro Arg Gln Leu Arg Thr Phe
    210                 215                 220

Ala Met Pro Pro Leu Ser Pro Ser Leu Ile Ala Pro Arg Phe Leu Glu
225                 230                 235                 240

Val Gly Ser Glu Arg Pro Val Thr Cys Thr Leu Asp Gly Leu Phe Pro
                245                 250                 255

Ala Pro Glu Ala Gly Val Tyr Leu Ser Leu Gly Asp Gln Arg Leu His
            260                 265                 270

Pro Asn Val Thr Leu Asp Gly Glu Ser Leu Val Ala Thr Ala Thr Ala
        275                 280                 285

Thr Ala Ser Glu Glu Gln Glu Gly Thr Lys Gln Leu Met Cys Ile Val
    290                 295                 300

Thr Leu Gly Gly Glu Ser Arg Glu Thr Gln Glu Asn Leu Thr Val Tyr
305                 310                 315                 320

Ser Phe Pro Ala Pro Leu Leu Thr Leu Ser Glu Pro Glu Ala Pro Glu
                325                 330                 335

Gly Lys Met Val Thr Val Ser Cys Trp Ala Gly Ala Arg Ala Leu Val
            340                 345                 350

Thr Leu Glu Gly Ile Pro Ala Ala Val Pro Gly Gln Pro Ala Glu Leu
        355                 360                 365

Gln Leu Asn Val Thr Lys Asn Asp Asp Lys Arg Gly Phe Phe Cys Asp
    370                 375                 380

Ala Ala Leu Asp Val Asp Gly Glu Thr Leu Arg Lys Asn Gln Ser Ser
385                 390                 395                 400

Glu Leu Arg Val Leu Tyr Ala Pro Arg Leu Asp Asp Leu Asp Cys Pro
                405                 410                 415

Arg Ser Trp Thr Trp Pro Glu Gly Pro Glu Gln Thr Leu His Cys Glu
            420                 425                 430

Ala Arg Gly Asn Pro Glu Pro Ser Val His Cys Ala Arg Pro Asp Gly
        435                 440                 445

Gly Ala Val Leu Ala Leu Gly Leu Leu Gly Pro Val Thr Arg Ala Leu
    450                 455                 460

Ala Gly Thr Tyr Arg Cys Thr Ala Ile Asn Gly Gln Gly Gln Ala Val
465                 470                 475                 480

Lys Asp Val Thr Leu Thr Val Glu Tyr Ala Pro Ala Leu Asp Ser Val
                485                 490                 495

Gly Cys Pro Glu Arg Ile Thr Trp Leu Glu Gly Thr Glu Ala Ser Leu
            500                 505                 510

Ser Cys Val Ala His Gly Val Pro Pro Pro Ser Val Ser Cys Val Arg
        515                 520                 525

Ser Gly Lys Glu Glu Val Met Glu Gly Pro Leu Arg Val Ala Arg Glu
    530                 535                 540

His Ala Gly Thr Tyr Arg Cys Glu Ala Ile Asn Ala Arg Gly Ser Ala
545                 550                 555                 560

Ala Lys Asn Val Ala Val Thr Val Glu Tyr Gly Pro Ser Phe Glu Glu
                565                 570                 575

Leu Gly Cys Pro Ser Asn Trp Thr Trp Val Glu Gly Ser Gly Lys Leu
            580                 585                 590

Phe Ser Cys Glu Val Asp Gly Lys Pro Glu Pro Arg Val Glu Cys Val
        595                 600                 605

Gly Ser Glu Gly Ala Ser Glu Gly Val Val Leu Pro Leu Val Ser Ser
    610                 615                 620

Asn Ser Gly Ser Arg Asn Ser Met Thr Pro Gly Asn Leu Ser Pro Gly
625                 630                 635                 640

Ile Tyr Leu Cys Asn Ala Thr Asn Arg His Gly Ser Thr Val Lys Thr
                645                 650                 655

Val Val Val Ser Ala Glu Ser Pro Pro Gln Met Asp Glu Ser Ser Cys
            660                 665                 670

Pro Ser His Gln Thr Trp Leu Glu Gly Ala Glu Ala Thr Ala Leu Ala
        675                 680                 685

Cys Ser Ala Arg Gly Arg Pro Ser Pro Arg Val Arg Cys Ser Arg Glu
    690                 695                 700

Gly Ala Ala Arg Leu Glu Arg Leu Gln Val Ser Arg Glu Asp Ala Gly
705                 710                 715                 720

Thr Tyr Leu Cys Val Ala Thr Asn Ala His Gly Thr Asp Ser Arg Thr
                725                 730                 735

Val Thr Val Gly Val Glu Tyr Arg Pro Val Val Ala Glu Leu Ala Ala
            740                 745                 750

Ser Pro Pro Ser Val Arg Pro Gly Gly Asn Phe Thr Leu Thr Cys Arg
        755                 760                 765

Ala Glu Ala Trp Pro Pro Ala Gln Ile Ser Trp Arg Ala Pro Pro Gly
    770                 775                 780

Ala Leu Asn Leu Gly Leu Ser Ser Asn Asn Ser Thr Leu Ser Val Ala
785                 790                 795                 800

Gly Ala Met Gly Ser His Gly Gly Glu Tyr Glu Cys Ala Ala Thr Asn
                805                 810                 815

Ala His Gly Arg His Ala Arg Arg Ile Thr Val Arg Val Ala Gly Pro
            820                 825                 830

Trp Leu Trp Val Ala Val Gly Gly Ala Ala Gly Gly Ala Ala Leu Leu
        835                 840                 845

Ala Ala Gly Ala Gly Leu Ala Phe Tyr Val Gln Ser Thr Ala Cys Lys
    850                 855                 860

Lys Gly Glu Tyr Asn Val Gln Glu Ala Glu Ser Ser Gly Glu Ala Val
865                 870                 875                 880

Cys Leu Asn Gly Ala Gly Gly Thr Pro Gly Ala Glu Gly Gly Ala Glu
                885                 890                 895

Thr Pro Gly Thr Ala Glu Ser Pro Ala Asp Gly Glu Val Phe Ala Ile
            900                 905                 910

Gln Leu Thr Ser Ser
        915

(2) INFORMATION FOR SEQ ID NO:3:

     (i) SEQUENCE CHARACTERISTICS:
          (A) LENGTH: 315 base pairs
          (B) TYPE: nucleic acid
          (C) STRANDEDNESS: single
          (D) TOPOLOGY: linear

    (ii) MOLECULE TYPE: DNA (genomic)

    (ix) FEATURE:
          (A) NAME/KEY: CDS
          (B) LOCATION: 1..315

    (xi) SEQUENCE DESCRIPTION: SEQ ID NO:3:

CCG GAT CGG GTA GAG CTA GTG CCT CTG CCT CCT TGG CAG CCT GTA GGT      48
Pro Asp Arg Val Glu Leu Val Pro Leu Pro Pro Trp Gln Pro Val Gly
  1               5                  10                  15

GAG AAC TTC ACC TTG AGC TGC AGG GTC CCG GGG GCA GGA CCC CGA GCG      96
Glu Asn Phe Thr Leu Ser Cys Arg Val Pro Gly Ala Gly Pro Arg Ala
             20                  25                  30

AGC CTC ACA TTG ACC TTG CTG CGA GGC GGA CAG GAG CTG ATT CGC CGA     144
Ser Leu Thr Leu Thr Leu Leu Arg Gly Gly Gln Glu Leu Ile Arg Arg
         35                  40                  45

AGT TTC GTA GGC GAG CCA CCC CGA GCT CGG TGT GCG ATG CTC ACC GCC     192
Ser Phe Val Gly Glu Pro Pro Arg Ala Arg Cys Ala Met Leu Thr Ala
     50                  55                  60

ACG GTC CTG GCG CGC AGA GAG GAT CAC AGG GAC AAT TTC TCA TGC CTC     240
Thr Val Leu Ala Arg Arg Glu Asp His Arg Asp Asn Phe Ser Cys Leu
 65                  70                  75                  80

GCG GAG CTT GAC CTG CGG ACA CAC GGC TTG GGA CTG TTT GCA AAC AGC     288
Ala Glu Leu Asp Leu Arg Thr His Gly Leu Gly Leu Phe Ala Asn Ser
                 85                  90                  95

TCA GCC CCC AGA CAG CTC CGC ACG TTT                                 315
Ser Ala Pro Arg Gln Leu Arg Thr Phe
            100                 105

(2) INFORMATION FOR SEQ ID NO:4:

     (i) SEQUENCE CHARACTERISTICS:
          (A) LENGTH: 1781 base pairs
          (B) TYPE: nucleic acid
          (C) STRANDEDNESS: single
          (D) TOPOLOGY: linear

    (ii) MOLECULE TYPE: cDNA

    (ix) FEATURE:
          (A) NAME/KEY: CDS
          (B) LOCATION: 16..1659

    (xi) SEQUENCE DESCRIPTION: SEQ ID NO:4:

CAGCTCTCTG TCAGA ATG GCC ACC ATG GTA CCA TCC GTG TTG TGG CCC AGG 51
Met Ala Thr Met Val Pro Ser Val Leu Trp Pro Arg
1               5                   10

GCC TGC TGG ACT CTG CTG GTC TGC TGT CTG CTG ACC CCA GGT GTC CAG 99
Ala Cys Trp Thr Leu Leu Val Cys Cys Leu Leu Thr Pro Gly Val Gln
        15                  20                  25

GGG CAG GAG TTC CTT TTG CGG GTG GAG CCC CAG AAC CCT GTG CTC TCT 147
Gly Gln Glu Phe Leu Leu Arg Val Glu Pro Gln Asn Pro Val Leu Ser
    30                  35                  40

GCT GGA GGG TCC CTG TTT GTG AAC TGC AGT ACT GAT TGT CCC AGC TCT 195
Ala Gly Gly Ser Leu Phe Val Asn Cys Ser Thr Asp Cys Pro Ser Ser
45                  50                  55                  60

GAG AAA ATC GCC TTG GAG ACG TCC CTA TCA AAG GAG CTG GTG GCC AGT 243
Glu Lys Ile Ala Leu Glu Thr Ser Leu Ser Lys Glu Leu Val Ala Ser
                65                  70                  75

GGC ATG GGC TGG GCA GCC TTC AAT CTC AGC AAC GTG ACT GGC AAC AGT 291
Gly Met Gly Trp Ala Ala Phe Asn Leu Ser Asn Val Thr Gly Asn Ser
            80                  85                  90

CGG ATC CTC TGC TCA GTG TAC TGC AAT GGC TCC CAG ATA ACA GGC TCC 339
Arg Ile Leu Cys Ser Val Tyr Cys Asn Gly Ser Gln Ile Thr Gly Ser
        95                  100                 105

TCT AAC ATC ACC GTG TAC GGG CTC CCG GAG CGT GTG GAG CTG GCA CCC 387
Ser Asn Ile Thr Val Tyr Gly Leu Pro Glu Arg Val Glu Leu Ala Pro
    110                 115                 120

CTG CCT CCT TGG CAG CCG GTG GGC CAG AAC TTC ACC CTG CGC TGC CAA 435
Leu Pro Pro Trp Gln Pro Val Gly Gln Asn Phe Thr Leu Arg Cys Gln
125                 130                 135                 140

GTG GAG GGT GGG TCG CCC CGG ACC AGC CTC ACG GTG GTG CTG CTT CGC 483
Val Glu Gly Gly Ser Pro Arg Thr Ser Leu Thr Val Val Leu Leu Arg
                145                 150                 155

TGG GAG GAG GAG CTG AGC CGG CAG CCC GCA GTG GAG GAG CCA GCG GAG 531
Trp Glu Glu Glu Leu Ser Arg Gln Pro Ala Val Glu Glu Pro Ala Glu
            160                 165                 170

GTC ACT GCC ACT GTG CTG GCC AGC AGA GAC GAC CAC GGA GCC CCT TTC 579
Val Thr Ala Thr Val Leu Ala Ser Arg Asp Asp His Gly Ala Pro Phe
        175                 180                 185

TCA TGC CGC ACA GAA CTG GAC ATG CAG CCC CAG GGG CTG GGA CTG TTC 627
Ser Cys Arg Thr Glu Leu Asp Met Gln Pro Gln Gly Leu Gly Leu Phe
    190                 195                 200

GTG AAC ACC TCA GCC CCC CGC CAG CTC CGA ACC TTT GTC CTG CCC GTG 675
Val Asn Thr Ser Ala Pro Arg Gln Leu Arg Thr Phe Val Leu Pro Val
205                 210                 215                 220

ACC CCC CCG CGC CTC GTG GCC CCC CGG TTC TTG GAG GTG GAA ACG TCG 723
Thr Pro Pro Arg Leu Val Ala Pro Arg Phe Leu Glu Val Glu Thr Ser
                225                 230                 235

TGG CCG GTG GAC TGC ACC CTA GAC GGG CTT TTT CCA GCC TCA GAG GCC 771
Trp Pro Val Asp Cys Thr Leu Asp Gly Leu Phe Pro Ala Ser Glu Ala
            240                 245                 250

CAG GTC TAC CTG GCG CTG GGG GAC CAG ATG CTG AAT GCG ACA GTC ATG 819
Gln Val Tyr Leu Ala Leu Gly Asp Gln Met Leu Asn Ala Thr Val Met
        255                 260                 265

AAC CAC GGG GAC ACG CTA ACG GCC ACA GCC ACA GCC ACG GCG CGC GCG 867
Asn His Gly Asp Thr Leu Thr Ala Thr Ala Thr Ala Thr Ala Arg Ala
    270                 275                 280

GAT CAG GAG GGT GCC CGG GAG ATC GTC TGC AAC GTG ACC CTA GGG GGC 915
Asp Gln Glu Gly Ala Arg Glu Ile Val Cys Asn Val Thr Leu Gly Gly
285                 290                 295                 300

GAG AGA CGG GAG GCC CGG GAG AAC TTG ACG GTC TTT AGC TTC CTA GGA 963
Glu Arg Arg Glu Ala Arg Glu Asn Leu Thr Val Phe Ser Phe Leu Gly
                305                 310                 315

CCC ATT GTG AAC CTC AGC GAG CCC ACC GCC CAT GAG GGG TCC ACA GTG 1011
Pro Ile Val Asn Leu Ser Glu Pro Thr Ala His Glu Gly Ser Thr Val
            320                 325                 330

ACC GTG AGT TGC ATG GCT GGG GCT CGA GTC CAG GTC ACG CTG GAC GGA 1059
Thr Val Ser Cys Met Ala Gly Ala Arg Val Gln Val Thr Leu Asp Gly
        335                 340                 345

GTT CCG GCC GCG GCC CCG GGG CAG ACA GCT CAA CTT CAG CTA AAT GCT 1107
Val Pro Ala Ala Ala Pro Gly Gln Thr Ala Gln Leu Gln Leu Asn Ala
    350                 355                 360

ACC GAG AGT GAC GAC GGA CGC AGC TTC TTC TGC AGT GCC ACT CTC GAG 1155
Thr Glu Ser Asp Asp Gly Arg Ser Phe Phe Cys Ser Ala Thr Leu Glu
365                 370                 375                 380

GTG GAC GGC GAG TTC TTG CAC AGG AAC AGT AGC GTC CAG CTG CGA GTC 1203
Val Asp Gly Glu Phe Leu His Arg Asn Ser Ser Val Gln Leu Arg Val
                385                 390                 395

CTG TAT GGT CCC AAA ATT GAC CGA GCC ACA TGC CCC CAG CAC TTG AAA 1251
Leu Tyr Gly Pro Lys Ile Asp Arg Ala Thr Cys Pro Gln His Leu Lys
            400                 405                 410

TGG AAA GAT AAA ACG AGA CAC GTC CTG CAG TGC CAA GCC AGG GGC AAC 1299
Trp Lys Asp Lys Thr Arg His Val Leu Gln Cys Gln Ala Arg Gly Asn
        415                 420                 425

CCG TAC CCC GAG CTG CGG TGT TTG AAG GAA GGC TCC AGC CGG GAG GTG 1347
Pro Tyr Pro Glu Leu Arg Cys Leu Lys Glu Gly Ser Ser Arg Glu Val
    430                 435                 440

CCG GTG GGG ATC CCG TTC TTC GTC AAC GTA ACA CAT AAT GGT ACT TAT 1395
Pro Val Gly Ile Pro Phe Phe Val Asn Val Thr His Asn Gly Thr Tyr
445                 450                 455                 460

CAG TGC CAA GCG TCC AGC TCA CGA GGC AAA TAC ACC CTG GTC GTG GTG 1443
Gln Cys Gln Ala Ser Ser Ser Arg Gly Lys Tyr Thr Leu Val Val Val
                465                 470                 475

ATG GAC ATT GAG GCT GGG AGC TCC CAC TTT GTC CCC GTC TTC GTG GCG 1491
Met Asp Ile Glu Ala Gly Ser Ser His Phe Val Pro Val Phe Val Ala
            480                 485                 490

GTG TTA CTG ACC CTG GGC GTG GTG ACT ATC GTA CTG GCC TTA ATG TAC 1539
Val Leu Leu Thr Leu Gly Val Val Thr Ile Val Leu Ala Leu Met Tyr
        495                 500                 505

GTC TTC AGG GAG CAC CAA CGG AGC GGC AGT TAC CAT GTT AGG GAG GAG 1587
Val Phe Arg Glu His Gln Arg Ser Gly Ser Tyr His Val Arg Glu Glu
    510                 515                 520

AGC ACC TAT CTG CCC CTC ACG TCT ATG CAG CCG ACA GAA GCA ATG GGG 1635
Ser Thr Tyr Leu Pro Leu Thr Ser Met Gln Pro Thr Glu Ala Met Gly
525                 530                 535                 540

GAA GAA CCG TCC AGA GCT GAG TGACGCTGGG ATCCGGGATC AAAGTTGGCG 1686
Glu Glu Pro Ser Arg Ala Glu
                545

GGGGCTTGGC TGTGCCCTCA GATTCCGCAC CAATAAAGCC TTCAAACTCC CAAAAAAAAA 1746

AAAAAAAAAA AAAAAAAAAA AAAAAAAAAA AAAAA 1781

(2) INFORMATION FOR SEQ ID NO:5:

(i) SEQUENCE CHARACTERISTICS:
(A) LENGTH: 4900 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:5:

CCGAACGCTC CTCGGCCTCT GGTCTNCTCT GGNCCTGGGG ATCCTAGGCA TCTCAGGTAA 60

GAAGAGCCCG CCCGTGGAGC NAGGTGGATA AGGCGGGGGC GGAATTGAAG GACCAGAGAG 120

GGCGGCCCGG GTGTCCCCCT CCAGGCTCCG CCCTCTTCTA GCTTCCCACG CTTCTGTCAC 180

CACCTGGAGN TCGGGGCTTC TCCCCGTCCT TCCTCCACCC CAACACACCT CAATCTTTCA 240

GANCTGAACC CAGCACCTTT TCTGGANTNG GGGNNTTGCA CCTAACCTGT CTCAGGAGAN 300

ACTGTGGCTC TCCTGTCCTC TCCTGCTCTG TNATGCCCTA TGGTTCACAG ACTGGCATCA 360

TCCCTATTCA TGATCCTCAA AGACNCCATC TCCTCAACTG TCATAACTCA GAGCTCTATT 420

CCCCCTCCAC CTGGAGCCCT GGAAACCGGC TTTCTAGGGC TTTTCTCCGC GGTTCTTTCC 480

CGGAGTTCAG CGTTGTGGCT TTTTGTCCAA GTTACTCAAG TTTGGGGACA ATCTCCTTTA 540

AGCCTTTGAC TCAGTCTCAT TTCCACTTTG CTTTTGCCCC AAGCCTCTGT GTCTCTCCCC 600

CATTTCCTGA CGATCTGTCA GAGTCTTAAG AGTGATTTGG TTCCCCATCC CCCCTCCAAC 660

TGGAGTCTCC TCCTCACTAT TGATGTGTGC ATCTGAGACC CCCATCCCCG CACCGAGTTT 720

CCCCATCTCT GTCAGTAAAG AGCAAGGCTT CCAGAGACAA CCCTCTAATA GCGCGTCAGT 780

CCCGAATCTT GAGTGGGATG CGGGACTCCC GTGCTATTTC TTGGCGGAGG TCTTTCCTGG 840

TCCTTATGGA CACCCCTGGT TTGGGATATG GGGGCCGCTA AGATTTCAGA GATGGGGTCC 900

CTAGGCTGAG NCCGCGTTTT CCCGGGCAGC GGTCGCGCTA GAACCTTTCT GGGCGGACCT 960

TCAGCCCCGC GTGGCGCTCG TGGAGCGCGG GGGCTCGCTG TGGCTCAACT GCAGCACTAA 1020

CTGTCCGAGG CCGGAGCGCG GTGGCCTGGA GACCTCGCTA CGCCGAAACG GGACCCAGAG 1080

GGGTCTGNAC TGNCTGGCTC GACAGCTGGT GGACATCCGA GANCCTGAAA CCCAGCCGGT 1140
CTGCTTCTTC CNCTGCGCGC GCCGCACACT CCAAGCGCGT GGGCTCATCC GAACTTTCCG 1200
TGAGTTCAGG GTGGGCACNC CCCTTGGGTC TCTGGACCTC CCCCTCAAGC TCCTCCCACC 1260
CGCCCTCTGA TCCTCCTGCT TGTTCTGAAA GTACTACAGC TGGCTAGAGC GGAGTTTTTG 1320
GTCCCTTGCA GAGCGACCGG ATCGGGTAGA GCTAGTGCCT CTGCCTCCTT GGCAGCCTGT 1380
AGGTGAGAAC TTCACCTTGA GCTGCAGGGT CCCGGGGGCA GGACCCCGAG CGAGCCTCAC 1440
ATTGACCTTG CTGCGAGGCG GCCAGGAGCT GATTCGCCGA AGTTTCGTAG GCGAGCCACC 1500
CCGAGCTCGG GGTGCGATGC TCACCGCCAC GGTCCTGGCG CGCAGAGAGG ATCACAGGGC 1560
CAATTTCTCA TGCCTCGCGG AGCTTGACCT GCGNCCACAC GGCTTGGGAC TGTTTGCANA 1620
CAGCTCAGCC CCCAGACAGC TCCGCACGTT TGGTGAGTGT GGACCCTAAC TGACAGATTT 1680
TAAGAAGTTT AGGGCAGCCA GGCGTGGTGG CATGGTGTCG TAGGCCCTAA GTCCCAGCCC 1740
AAGCAGANCT AAGNCGGATC TCTTGTGAAT TAAAAGTCTA GCTCGTCTAC ATAACGAGGN 1800
CTGCATAGTT AAATCCCCCA AAAGTCTAAG CAGCTAGCCC TTACTTCCAA CACAAGTACT 1860
AGCTTAAGTA CTTTCTCCTG TGAGCTTTTT CCTTTATGTA TTTACTCGTT GAGAGAAAAA 1920
GAGAGTGTGT GTACGTGCCT TTATGCACAT GCCGCAGTGC TTGTATGGAA GTTAAAGAAT 1980
AAGGAGGCGT TCTGCCCTTC CATCCTGTGG GTCCTAGGGG TGGTATTAGC TCCTCAGGCT 2040
TTGTTAGTNA CAAGCGCCTA GGCTTGGGGA GCCATCTCGC CCGCTCCTCT GTATCTTTAG 2100
GGTGAAACCA GACAATGCAT GCAAATTGGT TGATCAACAC TGAATGTTTA GTTCGTAAAT 2160
TCAAGCTCTG TTCTTTGTCT TCCTCAGCCA TGCCTCCACT TTCCCCCGAG CCTTATTGCC 2220
CCACGATTCT TAGAAGTGGG CTCAGAAAGG CCGGTGACKT GCACTTTGGA TGGACTGTTT 2280
CCTGCCCCAG AAGCCGGGGT TTACTTCTCT CTGGGAGATC AGAGGCTTCA TCCTAATGTG 2340
ACCCTCGACG GGGAGAGCCT TGTGGCCACT GCCACAGCTA CAGCAAGTGA AGAACAGGAA 2400
GGCACCAAAC AGCTGATGTG CATCGTGACC CTCGGGGGCG AAAGCAGGGA GACCCAGGAA 2460
AACCTGACTG TCTACAGTAA GGGGAATCCA ACAAGACCTT CAATAGCTCA GACTGGGGCT 2520
GGGGCTGGGT CTGGGTCTGG GGCCAGAGTC TCACAAAGGC GGAGCCTATA AAGTGGGCGG 2580
GACCTCCACA CCAGAACAAG CCGGGCGGGA GAGTTCCAGG GCAGGAGCAG ATAGAAGTTG 2640
GAAATTAATA GATTGGGTTG AGTTCCCTGA GTGGGGAGTG AACCCCACCC AATTCTCTGT 2700
CCCCAGGCTT CCCGGCTCCT CTTCTGACTT TAAGTGAGCC AGAAGCCCCC GAGGGAAAGA 2760
TGGTGACCGT AAGCTGCTGG GCAGGGGCCC GAGCCCTTGT CACCTTGGAG GGAATTCCAA 2820
GGACCCTCTT ACCGGCCCCA TCTTTAACCT TATCGTATCC CCTCTGCCTC ATGCCCGCAG 2880
ACGCACCTCG GCTGGATGAC TTGGACTGTC CCAGGAGCTG GACGTGGCCA GAGGGTCCAG 2940
AGCAGACCCT CCACTGCGAG GCCCGTGGAA ACCCTGAGCC CTCCGTGCAC TGTGCAAGGC 3000

CTGACGGTGG GGCGGTGCTA GCGCTGGGCC TGTTGGGTCC AGTGACCCGT GCCCTCGCGG 3060
GCACTTACCG ATGTACAGCA ATCAATGGGC AAGGCCAGGC GGTCAAGGAT GTGACCCTGA 3120
CTGTGGAATG TGAGTAGGGG GAGGTGGGCA TGCTTATCCC TTTAAGGTCA CGGAGTGTAC 3180
TGGGAGACTG GCTATACGGA AAGGAAAGAA GCCTAGGTTC AGCAGGGATT GGGAAAACAC 3240
TGAAGGAAAG TGGTGTGGTG TTTACAAACT TAACGGTGGT AACTGGGCAC GGTCTGGCAA 3300
AAACAGACAG CCAAGAGAGT GTGCCTGGGA AGCTGCAATG GGGGCTTTGT GGGAATTGGT 3360
CAACAGCACC CTGAGATCTC AGGAAAGGGG CCTGAAGTTA TCTCCAGAAC CCATGTGAAG 3420
GCAGGAAGAG AGAACGCCCA CCTTTTCCTG CTCCCCCCAA CCCCCCCCCA CATATCACAC 3480
GGAGTATATA AATAAATAAA ATGGCTCCTG CCGGAGGGAG TGAGAAGCTG TCTCCTGCAG 3540
GCTCAGAGCA GTGGTAGTGC ATGCCTTTAA TCCCAGCACT CGGTAGGCAA AGGCAGGCAG 3600
ATCTCTGTGA ATGTGGGGCC AGCCTGGTCT GTACAGAGAA ATCCTGTCTC AAAACAAACC 3660
AGCAAAGAAA CAAAACCAAA ATCAATTCCA GATGCCCCAG CGCTGGACAG TGTAGGCTGC 3720
CCANGACGTA TTACTTGNCT GGAGGGGACA GAGGCATCGC TTAGCTGTGT GGCACACGGG 3780
GTCCCACCAC CTAGCGTGAG CTGTGTGCGC TCTGGAAAGG AGGAAGTCAT GGAAGGGCCC 3840
CTGCGTGTGG CCCGGGAGCA CGCTGGCACT TACCGATGCG AAGCCATCAA CGCCAGGGGA 3900
TCAGCGGNCA AAAATGTGGC TGTCACGGTG GAATGTGAGT AGGGGTGGCT ACGGAAATGT 3960
CCACACCTGC GTCCTCTGTC CTCAGTGTGA ACTCCTATTT CCCTGCTTCC TAGATGGTCC 4020
CAGTTNTGAG GAGTTGGGCT GCCCCAGCAA CTGGACTTGG GTAGAAGGAT CTGGAAAACT 4080
GTTTTCCTGT GAAGTTGATG GGAAGCCGGA ACCACGCGTG GAGTGCGTGG GCTCGGAGGG 4140
TGCAAGCGAA GGGGTAGTGT TGCCCCTGGT GTCCTCGAAC TCTGGTTCCA GAAACTCTAT 4200
GACTCCTGGT AACCTGTCAC CGGGTATTTA CCTCTGCAAC GCCACCAACC GGCATGGCTC 4260
CACAGTCAAA ACAGTCGTCG TGAGCGCGGA ATGTGAGCAG GGGCCCAGGT GGGCGGAGAG 4320
TACCGGGTGT CCCAGGATCT TTTCTTTCCC TGATGCCCCT CCTTATGGTG GCTGATCTGC 4380
AGCACCGCCA CAGATGGATG AATCCAGTTG CCCGAGTCAC CAGACATGGC TGGAAGGAGC 4440
CGAGGCTACT GCGCTGGCCT GCAGTGACAG GGGNCGCCCC TCTCCACGCG TGCGCTGTTC 4500
CAGGGAAGGT GCAGCCAGGC TGGAGAGGCT ACAGGTGTCC CGAGAGGATG CGGGGACCTA 4560
CCTGTGTGTG GCTACCAACG CGCATGGCAC GGATTCACGG ACCGTCACTG TGGGTGTGGA 4620
ATGTGAGTGA GGACAGCGCT GAATGAAGAC GACTCAGACC GCCAGAAAAG TGCCTTGAGG 4680
CCTGGGATGT ATGATCCAGT GGGTAGAGTG CTCAATTAGC ACTCACTAAA ATGTATATTC 4740
TATTCCTAAT ACTCTTTAAT TTTANCCTTT GGGAGGCAGA GACAGGCAGA TCTCTGTTCC 4800
GGGATAACCT GCTCTCTGTC TAGGACAGCT TGGTCTACAG AGGGGNTACA GGCCCCCCCT 4860
CCCAAGATTG NATAGCAACC CTCTGGCTCC CTGTCTCTCT 4900

(2) INFORMATION FOR SEQ ID NO:6:

(i) SEQUENCE CHARACTERISTICS:
(A) LENGTH: 1295 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:6:

NGAATTCCGG CGGATCGGGT AGAGCTAGTG CCTCTGCCTC CTTGGCAGCC TGTAGGTGAG 60

AACTTCACCT TGAGCTGCAG GGTCCCGGGG GCAGGACCCC GAGCGAGCCT CACATTGACC 120

TTGCTGCGAG GCGGCCAGGA GCTGATTCGC CGAAGTTTCG TAGGCGAGCC ACCCCGAGCT 180

CGGGGTGCGA TGCTCACCGC CACGGTCCTG GCGCGCAGAG AGGATCACAG GGCCAATTTC 240

TCATGCCTCG CGGAGCTTGA CCTGCGGCCA CACGGCTTGG GACTGTTTGC AAACAGCTCA 300

GCCCCCAGAC AGCTCCGCAC GTTTGCCATG CCTCCACTTT CCCCGAGCCT TATTGCCCCA 360

CGATTCTTAG AAGTGGGCTC AGAAAGGCCG GTGACTTGCA CTTTGGATGG ACTGTTTCCT 420

GCCCCAGAAG CCGGGGTTTA CCTCTCTCTG GGAGATCAGA GGCTTCATCC TAATGTGACC 480

CTCGACGGGG AGAGCCTTGT GGCCACTGCC ACAGCTACAG CAAGTGAAGA ACAGGAAGGC 540

ACCAAACAGC TGATGTGCAT CGTGACCCTC GGGGGCGAAA GCAGGGAGAC CCAGGAAAAC 600

CTGACTGTCT ACAGCTTCCC GGCTCCTCTT CTGACTTTAA GTGAGCCAGA AGCCCCCGAG 660

GGAAAGATGG TGACCGTAAG CTGCTGGGCA GGGGCCCGAG CCCTTGTCAC CTTGGAGGGA 720

ATTCCAAGGA CCCTCTTACC GGCCCCATCT TTAACCTTAT CGTATCCCCT CTGCCTCATG 780

CCCGCAGACG CACCTCGGCT GGATGACTTG GACTGTCCCA GGAGCTGGAC GTGGCCAGAG 840

GGTCCAGAGC AGACCCTCCA CTGCGAGGCC CGTGGAAACC CTGAGCCCTC CGTGCACTGT 900

GCAAGGCCTG ACGGTGGGGC GGTGCTAGCG CTGGGCCTGT TGGGTCCAGT GACCCGTGCC 960

CTCGCGGGCA CTTACCGATG TACAGCAATC AATGGGCAAG GCCAGGCGGT CAAGGATGTG 1020

ACCCTGACTG TGGAATATGC CCCAGCGCTG GACAGTGTAG GCTGCCCAGA ACGTATTACT 1080

TGGCTGGAGG GGACAGAGGC ATCGCTTAGC TGTGTGGCAC ACGGGGTCCC ACCACCTAGC 1140

GTGAGCTGTG TGCGCTCTGG AAAGGAGGAA GTCATGGAAG GGCCCCTGCG TTTTGGCCGG 1200

GAGCACGCTG GCACTTACCG ATGCGAAGCC ATCAACGCCA GGGGATCAGC GGCCAAAAAT 1260

GTGGCTGTCA CGGTGGAATA TGGTCCCCGG AATTC 1295

(2) INFORMATION FOR SEQ ID NO:7:

(i) SEQUENCE CHARACTERISTICS:
(A) LENGTH: 2214 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:7:

CGAATCTTGA GTGGGATGCG GGACTCCCGT GCTATTTCTT GGCGGAGGTC TTTCCTGGTC 60

CTTATGGACA CCCCTGGTTT GGGATATGGG GGCCGCTAAG ATTTCAGAGA TGGGGTCCCT 120

AGGCTGAGCC CGCGTTTTCC CGGGCAGCGG TCGCGCTAGA ACCTTTCTGG GCGGACCTTC 180

AGCCCCGCGT GGCGCTCGTG GAGCGCGGGG GCTCGCTGTG GCTCAACTGC AGCACTAACT 240

GTCCGAGGCC GGAGCGCGGT GGYCTGGAGA CCTCGCTACG CCGAAACGGG ACCCAGAGGG 300

GTCTGCGCTG GCTGGCTCGA CAGMTGGTGG ACATCCGAGA GCCTGAAACC CAGTCGGTCT 360

GCTTCTTCCG CTGGGCGCGC CGCACACTCC AAGNGAGTGG GCTCATCCGA ACTTTCCAGC 420

GACCGGATCG GGTAGAGCTA GTGCCTCTGN CTCCTTGGCA GCCTGTAGGT GAGAACTTCA 480

CCTTGAGCTG CAGGGTCCCG GGGGCAGGAC CCCGAGCGAG CCTCACATTG ACCTTGCTGC 540

GAGGCGGCCA GGAGCTGATT CGCCGAAGTT TCGTAGGCGA GCCACCCCGA GCTCGGGGTG 600

CGATGCTCAC CGCCACGGTC CTGGCGCGCA GAGAGGATCA CAGGGCCAAT TTCTCATGCC 660

TCGCGGAGCT TGACCTGCGG ACACACGGCT TGGGACTGTT TGCAAACAGC TCAGCCCCCA 720

GACAGCTCCG CACGTTTGGC ATGCCTCCAC TTTCCCCGAG CCTTATTGNC CCACGATTCT 780

TAGAAGTGGG CTCAGAAAGG CCGGTGACTT GCACTTTGGA TGGACTGTTT CCTGCCCCAG 840

AAGCCGGGGT TTACCTCTCT CTGGGAGATC AGAGGCTTCA TCCTAATGTG ACCCTCGACG 900

GGGAGAGCCT TGTGGCCACT GNCACAGMTA CAGCAAGTGA AGAACAGGAA GGCACCAAAC 960

AGCTGATGTG CATCGTGACC CTCGGGGGCG AAAGCAGGGA GACCCAGGAA AACCTGACTG 1020

TCTACAGCTT CCCGGCTCCT CTTCTGACTT TAAGTGAGCC AGAAGCCCCC GAGGGAAAGA 1080

TGGTGACCGT AAGCTGCTGG GCAGGGGCCC GAGCCCTTGT CACCTTGGAG GGAATTCCAG 1140

CTGCGGTCCC TGGGCAGCCC GCTGAGCTCC AGTTAAATGT CACAAAGAAT GACGACAAGC 1200

GGGGCTTCTT CTGCGACGCT GCCCTCGATG TGGACGGGGA AACTCTGAGA AAGAACCAGA 1260

GCTCTGAGCT TCGTGTTCTG TACGCACCTC GGCTGGATGA CTTGGACTGT CCCAGGAGCT 1320

GGACGTGGCC AGAGGGTCCA GAGCAGACCC TCCACTGCGA GGCCCGTGGA AACCCTGAGC 1380

CCTCCGTGCA CTGTGCAAGG CCTGACGGTG GGGCGGTGCT AGCGCTGGGC CTGTTGGGTC 1440

CAGTGACCCG TGCCCTCGCG GGAACTTACC GATGTACAGC AATCAATGGG CAAGGCCAGG 1500

CGGTCAAGGA TGTGACCCTG ACTGTGGAAT ATGCCCCAGC GCTGGACAGT GTAGGCTGCC 1560

CAGAACGTAT TACTTGGCTG GAGGGGACAG AGGCATCGCT TAGCTGTGTG GCACACGGGG 1620

TCCCACCACC TAGCGTGAGC TGTGTGCGCT CTGGAAAGGA GGAAGTCATG GAAGGGCCCC 1680

TGCGTGTGGC CCGGGAGCAC GCTGGCACTT ACCGATGCGA AGCCATCAAC GNCAGGGGAT 1740

CAGCGGWCAA AAATGTGGCT GTCACGGTGG AATATGGTCC CAGTTTGGAG GAGTTGGGCT 1800

GCCCCAGYAA CTGGACTTGG GTAGAAGGAT CTGGAAAACT GTTTTCCTGT GAAGTTGATG 1860

GGAAGCCGGA ACCACGCGTG GAGTGCGTGG GCTCGGAGGG TGCAAGCGAA GGGGTAGTGT 1920

TGCCCCTGGT GTCCTCGAAC TCTGGTTCCA GAAACTCTAT GACTCCTGGT AACCTGTCAC 1980

CGGGTATTTA CCTCTGCAAC GCCACCAACC GGMATGGNTC CACAGTCAAA ACAGTCGTCG 2040

TGAGCGCGGA ATCACCGCCA CAGATGGATG AATCCAGTTG CCCGAGTCAC CAGACATGGN 2100

TGGAAGGAGC CGAGGNTACT GCGCTGGCCT GCAGTGCCAG AGGNCGCCCC TCTCCACGCG 2160

TGCGCTGTTC CAGGGAAGGT GCAGMCAGGC TGGAGAGGNT ACAGGTGTCC CGAG 2214

(2) INFORMATION FOR SEQ ID NO:8:

(i) SEQUENCE CHARACTERISTICS:
(A) LENGTH: 5077 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:8:

CCGAACGCTC CTCGGCCTCT GGTCTNCTCT GGNCCTGGGG ATCCTAGGCA TCTCAGGTAA 60

GAAGAGCCCG CCCGTGGAGC NAGGTGGATA AGGCGGGGGC GGAATTGAAG GACCAGAGAG 120

GGCGGCCCGG GTGTCCCCCT CCAGGCTCCG CCCTCTTCTA GCTTCCCACG CTTCTGTCAC 180

CACCTGGAGN TCGGGGCTTC TCCCCGTCCT TCCTCCACCC CAACACACCT CAATCTTTCA 240

GANCTGAACC CAGCACCTTT TCTGGANTNG GGGNNTTGCA CCTAACCTGT CTCAGGAGAN 300

ACTGTGGCTC TCCTGTCCTC TCCTGCTCTG TNATGCCCTA TGGTTCACAG ACTGGCATCA 360

TCCCTATTCA TGATCCTCAA AGACNCCATC TCCTCAACTG TCATAACTCA GAGCTCTATT 420

CCCCCTCCAC CTGGAGCCCT GGAAACCGGC TTTCTAGGGC TTTTCTCCGC GGTTCTTTCC 480

CGGAGTTCAG CGTTGTGGCT TTTTGTCCAA GTTACTCAAG TTTGGGGACA ATCTCCTTTA 540

AGCCTTTGAC TCAGTCTCAT TTCCACTTTG CTTTTGCCCC AAGCCTCTGT GTCTCTCCCC 600

CATTTCCTGA CGATCTGTCA GAGTCTTAAG AGTGATTTGG TTCCCCATCC CCCCTCCAAC 660

TGGAGTCTCC TCCTCACTAT TGATGTGTGC ATCTGAGACC CCCATCCCCG CACCGAGTTT 720

CCCCATCTCT GTCAGTAAAG AGCAAGGCTT CCAGAGACAA CCCTCTAATA GCGCGTCAGT 780

CCCGAATCTT GAGTGGGATG CGGGACTCCC GTGCTATTTC TTGGCGGAGG TCTTTCCTGG 840

TCCTTATGGA CACCCCTGGT TTGGGATATG GGGGCCGCTA AGATTTCAGA GATGGGGTCC 900

CTAGGCTGAG NCCGCGTTTT CCCGGGCAGC GGTCGCGCTA GAACCTTTCT GGGCGGACCT 960

TCAGCCCCGC GTGGCGCTCG TGGAGCGCGG GGGCTCGCTG TGGCTCAACT GCAGCACTAA 1020

CTGTCCGAGG CCGGAGCGCG GTGGCCTGGA GACCTCGCTA CGCCGAAACG GGACCCAGAG 1080

GGGTCTGNAC TGNCTGGCTC GACAGCTGGT GGACATCCGA GANCCTGAAA CCCAGCCGGT 1140

CTGCTTCTTC CNCTGCGCGC GCCGCACACT CCAAGCGCGT GGGCTCATCC GAACTTTCCG 1200
TGAGTTCAGG GTGGGCACNC CCCTTGGGTC TCTGGACCTC CCCCTCAAGC TCCTCCCACC 1260
CGCCCTCTGA TCCTCCTGCT TGTTCTGAAA GTACTACAGC TGGCTAGAGC GGAGTTTTTG 1320
GTCCCTTGCA GAGCGACCGG ATCGGGTAGA GCTAGTGCCT CTGCCTCCTT GGCAGCCTGT 1380
AGGTGAGAAC TTCACCTTGA GCTGCAGGGT CCCGGGGGCA GGACCCCGAG CGAGCCTCAC 1440
ATTGACCTTG CTGCGAGGCG GCCAGGAGCT GATTCGCCGA AGTTTCGTAG GCGAGCCACC 1500
CCGAGCTCGG GGTGCGATGC TCACCGCCAC GGTCCTGGCG CGCAGAGAGG ATCACAGGGC 1560
CAATTTCTCA TGCCTCGCGG AGCTTGACCT GCGNCCACAC GGCTTGGGAC TGTTTGCANA 1620
CAGCTCAGCC CCCAGACAGC TCCGCACGTT TGGTGAGTGT GGACCCTAAC TGACAGATTT 1680
TAAGAAGTTT AGGGCAGCCA GGCGTGGTGG CATGGTGTCG TAGGCCCTAA GTCCCAGCCC 1740
AAGCAGANCT AAGNCGGATC TCTTGTGAAT TAAAAGTCTA GCTCGTCTAC ATAACGAGGN 1800
CTGCATAGTT AAATCCCCCA AAAGTCTAAG CAGCTAGCCC TTACTTCCAA CACAAGTACT 1860
AGCTTAAGTA CTTTCTCCTG TGAGCTTTTT CCTTTATGTA TTTACTCGTT GAGAGAAAAA 1920
GAGAGTGTGT GTACGTGCCT TTATGCACAT GCCGCAGTGC TTGTATGGAA GTTAAAGAAT 1980
AAGGAGGCGT TCTGCCCTTC CATCCTGTGG GTCCTAGGGG TGGTATTAGC TCCTCAGGCT 2040
TTGTTAGTNA CAAGCGCCTA GGCTTGGGGA GCCATCTCGC CCGCTCCTCT GTATCTTTAG 2100
GGTGAAACCA GACAATGCAT GCAAATTGGT TGATCAACAC TGAATGTTTA GTTCGTAAAT 2160
TCAAGCTCTG TTCTTTGTCT TCCTCAGCCA TGCCTCCACT TTCCCCCGAG CCTTATTGCC 2220
CCACGATTCT TAGAAGTGGG CTCAGAAAGG CCGGTGACKT GCACTTTGGA TGGACTGTTT 2280
CCTGCCCCAG AAGCCGGGGT TTACTTCTCT CTGGGAGATC AGAGGCTTCA TCCTAATGTG 2340
ACCCTCGACG GGGAGAGCCT TGTGGCCACT GCCACAGCTA CAGCAAGTGA AGAACAGGAA 2400
GGCACCAAAC AGCTGATGTG CATCGTGACC CTCGGGGGCG AAAGCAGGGA GACCCAGGAA 2460
AACCTGACTG T[?]TACAGTAA GGGGAATCCA ACAAGACCTT CAATAGCTCA GACTGGGGCT 2520
GGGGCTGGGT CTGGGTCTGG GGCCAGAGTC TCACAAAGGC GGAGCCTATA AAGTGGGCGG 2580
GACCTCCACA CCAGAACAAG CCGGGCGGGA GAGTTCCAGG GCAGGAGCAG ATAGAAGTTG 2640
GAAATTAATA GATTGGGTTG AGTTCCCTGA GTGGGGAGTG AACCCCACCC AATTCTCTGT 2700
CCCCAGGCTT CCCGGCTCCT CTTCTGACTT TAAGTGAGCC AGAAGCCCCC GAGGGAAAGA 2760
TGGTGACCGT AAGCTGCTGG GCAGGGGCCC GAGCCCTTGT CACCTTGGAG GGAATTCCAG 2820
CTGCGGTCCC TGGGCAGCCC GCTGAGCTCC AGTTAAATGT CACAAAGAAT GACGACAAGC 2880
GGGGCTTCTT CTGCGACGCT GCCCTCGATG TGGACGGGGA AACTCTGAGA AAGAACCAGA 2940
GCTCTGAGCT TCGTGTTCTG TGTGAGTGGA TGTTCACTTT ATCTCTGTGA ATTCCAAGGA 3000
CCCTCTTACC GGCCCCATCT TTAACCTTAT CGTATCCCCT CTGCCTCATG CCCGCAGACG 3060

CACCTCGGCT GGATGACTTG GACTGTCCCA GGAGCTGGAC GTGGCCAGAG GGTCCAGAGC 3120
AGACCCTCCA CTGCGAGGCC CGTGGAAACC CTGAGCCCTC CGTGCACTGT GCAAGGCCTG 3180
ACGGTGGGGC GGTGCTAGCG CTGGGCCTGT TGGGTCCAGT GACCCGTGCC CTCGCGGGCA 3240
CTTACCGATG TACAGCAATC AATGGGCAAG GCCAGGCGGT CAAGGATGTG ACCCTGACTG 3300
TGGAATGTGA GTAGGGGGAG GTGGGCATGC TTATCCCTTT AAGGTCACGG AGTGTACTGG 3360
GAGACTGGCT ATACGGAAAG GAAAGAAGCC TAGGTTCAGC AGGGATTGGG AAAACACTGA 3420
AGGAAAGTGG TGTGGTGTTT ACAAACTTAA CGGTGGTAAC TGGGCACGGT CTGGCAAAAA 3480
CAGACAGCCA AGAGAGTGTG CCTGGGAAGC TGCAATGGGG GCTTTGTGGG AATTGGTCAA 3540
CAGCACCCTG AGATCTCAGG AAAGGGGCCT GAAGTTATCT CCAGAACCCA TGTGAAGGCA 3600
GGAAGAGAGA ACGCCCACCT TTTCCTGCTC CCCCCAACCC CCCCCCACAT ATCACACGGA 3660
GTATATAAAT AAATAAAATG GCTCCTGCCG GAGGGAGTGA GAAGCTGTCT CCTGCAGGCT 3720
CAGAGCAGTG GTAGTGCATG CCTTTAATCC CAGCACTCGG TAGGCAAAGG CAGGCAGATC 3780
TCTGTGAATG TGGGGCCAGC CTGGTCTGTA CAGAGAAATC CTGTCTCAAA ACAAACCAGC 3840
AAAGAAACAA AACCAAAATC AATTCCAGAT GCCCCAGCGC TGGACAGTGT AGGCTGCCCA 3900
NGACGTATTA CTTGNCTGGA GGGGACAGAG GCATCGCTTA GCTGTGTGGC ACACGGGGTC 3960
CCACCACCTA GCGTGAGCTG TGTGCGCTCT GGAAAGGAGG AAGTCATGGA AGGGCCCCTG 4020
CGTGTGGCCC GGGAGCACGC TGGCACTTAC CGATGCGAAG CCATCAACGC CAGGGGATCA 4080
GCGGNCAAAA ATGTGGCTGT CACGGTGGAA TGTGAGTAGG GGTGGCTACG GAAATGTCCA 4140
CACCTGCGTC CTCTGTCCTC AGTGTGAACT CCTATTTCCC TGCTTCCTAG ATGGTCCCAG 4200
TTNTGAGGAG TTGGGCTGCC CCAGCAACTG GACTTGGGTA GAAGGATCTG GAAAACTGTT 4260
TTCCTGTGAA GTTGATGGGA AGCCGGAACC ACGCGTGGAG TGCGTGGGCT CGGAGGGTGC 4320
AAGCGAAGGG GTAGTGTTGC CCCTGGTGTC CTCGAACTCT GGTTCCAGAA ACTCTATGAC 4380
TCCTGGTAAC CTGTCACCGG GTATTTACCT CTGCAACGCC ACCAACCGGC ATGGCTCCAC 4440
AGTCAAAACA GTCGTCGTGA GCGCGGAATG TGAGCAGGGG CCCAGGTGGG CGGAGAGTAC 4500
CGGGTGTCCC AGGATCTTTT CTTTCCCTGA TGCCCCTCCT TATGGTGGCT GATCTGCAGC 4560
ACCGCCACAG ATGGATGAAT CCAGTTGCCC GAGTCACCAG ACATGGCTGG AAGGAGCCGA 4620
GGCTACTGCG CTGGCCTGCA GTGACAGGGG NCGCCCCTCT CCACGCGTGC GCTGTTCCAG 4680
GGAAGGTGCA GCCAGGCTGG AGAGGCTACA GGTGTCCCGA GAGGATGCGG GGACCTACCT 4740
GTGTGTGGCT ACCAACGCGC ATGGCACGGA TTCACGGACC GTCACTGTGG GTGTGGAATG 4800
TGAGTGAGGA CAGCGCTGAA TGAAGACGAC TCAGACCGCC AGAAAAGTGC CTTGAGGCCT 4860
GGGATGTATG ATCCAGTGGG TAGAGTGCTC AATTAGCACT CACTAAAATG TATATTCTAT 4920
TCCTAATACT CTTTAATTTT ANCCTTTGGG AGGCAGAGAC AGGCAGATCT CTGTTCCGGG 4980

ATAACCTGCT CTCTGTCTAG GACAGCTTGG TCTACAGAGG GGNTACAGGC CCCCCCTCCC 5040
AAGATTGNAT AGCAACCCTC TGGCTCCCTG TCTCTCT 5077
(2) INFORMATION FOR SEQ ID NO:9:
(i) SEQUENCE CHARACTERISTICS:
(A) LENGTH: 1472 base pairs
(B) TYPE: nucleic acid
(C) STRANDEDNESS: single
(D) TOPOLOGY: linear
(ii) MOLECULE TYPE: cDNA
(xi) SEQUENCE DESCRIPTION: SEQ ID NO:9:
NGAATTCCGG CGGATCGGGT AGAGCTAGTG CCTCTGCCTC CTTGGCAGCC TGTAGGTGAG 60
AACTTCACCT TGAGCTGCAG GGTCCCGGGG GCAGGACCCC GAGCGAGCCT CACATTGACC 120
TTGCTGCGAG GCGGCCAGGA GCTGATTCGC CGAAGTTTCG TAGGCGAGCC ACCCCGAGCT 180
CGGGGTGCGA TGCTCACCGC CACGGTCCTG GCGCGCAGAG AGGATCACAG GGCCAATTTC 240
TCATGCCTCG CGGAGCTTGA CCTGCGGCCA CACGGCTTGG GACTGTTTGC AAACAGCTCA 300
GCCCCCAGAC AGCTCCGCAC GTTTGCCATG CCTCCACTTT CCCCGAGCCT TATTGCCCCA 360
CGATTCTTAG AAGTGGGCTC AGAAAGGCCG GTGACTTGCA CTTTGGATGG ACTGTTTCCT 420
GCCCCAGAAG CCGGGGTTTA CCTCTCTCTG GGAGATCAGA GGCTTCATCC TAATGTGACC 480
CTCGACGGGG AGAGCCTTGT GGCCACTGCC ACAGCTACAG CAAGTGAAGA ACAGGAAGGC 540
ACCAAACAGC TGATGTGCAT CGTGACCCTC GGGGGCGAAA GCAGGGAGAC CCAGGAAAAC 600
CTGACTGTCT ACAGCTTCCC GGCTCCTCTT CTGACTTTAA GTGAGCCAGA AGCCCCCGAG 660
GGAAAGATGG TGACCGTAAG CTGCTGGGCA GGGGCCCGAG CCCTTGTCAC CTTGGAGGGA 720
ATTCCAGCTG CGGTCCCTGG GCAGCCCGCT GAGCTCCAGT TAAATGTCAC AAAGAATGAC 780
GACAAGCGGG GCTTCTTCTG CGACGCTGCC CTCGATGTGG ACGGGGAAAC TCTGAGAAAG 840
AACCAGAGCT CTGAGCTTCG TGTTCTGTGT GAGTGGATGT TCACTTTATC TCTGTGAATT 900
CCAAGGACCC TCTTACCGGC CCCATCTTTA ACCTTATCGT ATCCCCTCTG CCTCATGCCC 960
GCAGACGCAC CTCGGCTGGA TGACTTGGAC TGTCCCAGGA GCTGGACGTG GCCAGAGGGT 1020
CCAGAGCAGA CCCTCCACTG CGAGGCCCGT GGAAACCCTG AGCCCTCCGT GCACTGTGCA 1080
AGGCCTGACG GTGGGGCGGT GCTAGCGCTG GGCCTGTTGG GTCCAGTGAC CCGTGCCCTC 1140
GCGGGCACTT ACCGATGTAC AGCAATCAAT GGGCAAGGCC AGGCGGTCAA GGATGTGACC 1200
CTGACTGTGG AATATGCCCC AGCGCTGGAC AGTGTAGGCT GCCCAGAACG TATTACTTGG 1260
CTGGAGGGGA CAGAGGCATC GCTTAGCTGT GTGGCACACG GGGTCCCACC ACCTAGCGTG 1320
AGCTGTGTGC GCTCTGGAAA GGAGGAAGTC ATGGAAGGGC CCCTGCGTTT TGGCCGGGAG 1380
CACGCTGGCA CTTACCGATG CGAAGCCATC AACGCCAGGG GATCAGCGGC CAAAAATGTG 1440

GCTGTCACGG TGGAATATGG TCCCCGGAAT TC 1472

(2) INFORMATION FOR SEQ ID NO:10:

(i) SEQUENCE CHARACTERISTICS:
(A) LENGTH: 2550 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:10:

CCTCTGCCTC CTTGGCAGCC TGTAGGTGAG AACTTCACCT TGAGCTGCAG GGTCCCGGGG 60

GCAGGACCCC GAGCGAGCCT CACATTGACC TTGCTGCGAG GCGGCCAGGA GCTGATTCGC 120

CGAAGTTTCG TAGGCGAGCC ACCCCGAGCT CGGGGTGCGA TGCTCACCGC CACGGTCCTG 180

GCGCGCAGAG AGGATCACAG GGCCAATTTC TCATGCCTCG CGGAGCTTGA CCTGCGGCCA 240

CACGGCTTGG GACTGTTTGC AAACAGCTCA GCCCCCAGAC AGCTCCGCAC GTTTGCCATG 300

CCTCCACTTT CCCCGAGCCT TATTGCCCCA CGATTCTTAG AAGTGGGCTC AGAAAGGCCG 360

GTGACTTGCA CTTTGGATGG ACTGTTTCCT GCCCCAGAAG CCGGGGTTTA CCTCTCTCTG 420

GGAGATCAGA GGCTTCATCC TAATGTGACC CTCGACGGGG AGAGCCTTGT GGCCACTGCC 480

ACAGCTACAG CAAGTGAAGA ACAGGAAGGC ACCAAACAGC TGATGTGCAT CGTGACCCTC 540

GGGGGCGAAA GCAGGGAGAC CCAGGAAAAC CTGACTGTCT ACAGCTTCCC GGCTCCTCTT 600

CTGACTTTAA GTGAGCCAGA AGCCCCCGAG GGAAAGATGG TGACCGTAAG CTGCTGGGCA 660

GGGGCCCGAG CCCTTGTCAC CTTGGAGGGA ATTCCAGCTG CGGTCCCTGG GCAGCCCGCT 720

GAGCTCCAGT TAAATGTCAC AAAGAATGAC GACAAGCGGG GCTTCTTCTG CGACGCTGCC 780

CTCGATGTGG ACGGGGAAAC TCTGAGAAAG AACCAGAGCT CTGAGCTTCG TGTTCTGTAC 840

GCACCTCGGC TGGATGACTT GGACTGTCCC AGGAGCTGGA CGTGGCCAGA GGGTCCAGAG 900

CAGACCCTCC ACTGCGAGGC CCGTGGAAAC CCTGAGCCCT CCGTGCACTG TGCAAGGCCT 960

GACGGTGGGG CGGTGCTAGC GCTGGGCCTG TTGGGTCCAG TGACCCGTGC CCTCGCGGGC 1020

ACTTACCGAT GTACAGCAAT CAATGGGCAA GGCCAGGCGG TCAAGGATGT GACCCTGACT 1080

GTGGAATATG CCCCAGCGCT GGACAGTGTA GGCTGCCCAG AACGTATTAC TTGGCTGGAG 1140

GGGACAGAGG CATCGCTTAG CTGTGTGGCA CACGGGGTCC CACCACCTAG CGTGAGCTGT 1200

GTGCGCTCTG GAAAGGAGGA AGTCATGGAA GGGCCCCTGC GTGTGGCCCG GGAGCACGCT 1260

GGCACTTACC GATGCGAAGC CATCAACGCC AGGGGATCAG CGGCCAAAAA TGTGGCTGTC 1320

ACGGTGGAAT ATGGTCCCAG TTTTGAGGAG TTGGGCTGCC CCAGCAACTG GACTTGGGTA 1380

GAAGGATCTG GAAAACTGTT TTCCTGTGAA GTTGATGGGA AGCCGGAACC ACGCGTGGAG 1440

TGCGTGGGCT CGGAGGGTGC AAGCGAAGGG GTAGTGTTGC CCCTGGTGTC CTCGAACTCT 1500

GGTTCCAGAA ACTCTATGAC TCCTGGTAAC CTGTCACCGG GTATTTACCT CTGCAACGCC 1560
ACCAACCGGC ATGGCTCCAC AGTCAAAACA GTCGTCGTGA GCGCGGAATC ACCGCCACAG 1620
ATGGATGAAT CCAGTTGCCC GAGTCACCAG ACATGGCTGG AAGGAGCCGA GGCTACTGCG 1680
CTGGCCTGCA GTGCCAGAGG CCGCCCCTCT CCACGCGTGC GCTGTTCCAG GGAAGGTGCA 1740
GCCAGGCTGG AGAGGCTACA GGTGTCCCGA GAGGATGCGG GGACCTACCT GTGTGTGGCT 1800
ACCAACGCGC ATGGCACGGA TTCACGGACC GTCACTGTGG GTGTGGAATA CCGGCCTGTG 1860
GTGGCTGAGC TGGCAGCCTC GCCCCCAAGC GTGCGGCCTG GCGGAAACTT CACTCTGACC 1920
TGCCGTGCAG AGGCCTGGCC TCCAGCCCAG ATCAGCTGGC GCGCGCCCCC GGGAGCTCTC 1980
AACCTCGGTC TCTCCAGCAA CAACAGCACG CTGAGCGTGG CGGGTGCCAT GGGCAGCCAT 2040
GGTGGCGAGT ATGAGTGCGC AGCCACCAAT GCGCATGGGC GCCACGCACG GCGCATCACG 2100
GTGCGCGTGG CCGGTCCATG GCTGTGGGTC GCTGTGGGCG GTGCGGCAGG GGGCGCGGCG 2160
CTGCTGGCCG CAGGGGCCGG CCTGGCCTTC TACGTGCAGT CCACCGCTTG CAAGAAGGGA 2220
GAGTACAACG TCCAGGAGGC TGAGAGCTCA GGCGAGGCGG TGTGTCTCAA TGGCGCGGGC 2280
GGGACACCGG GTGCAGAAGG CGGAGCAGAG ACCCCCGGCA CTGCCGAGTC ACCTGCAGAT 2340
GGCGAGGTTT TCGCCATCCA GCTGACATCT TCCTGAGCCT GTATCCAGCT CCCCCAGGGG 2400
CCTCGAAAGC ACAGGGGTGG ACGTATGTAT TGTTCACTCT CTATTTATTC AACTCCAGGG 2460
GCGTCGTCCC CGTTTTCTAC CCATTCCCTT AATAAAGTTT TTATAGGAGA AAAAAAAAAA 2520
AAAAAAAAAA AAAAAAAAAA AAAAAAAAAA 2550

(2) INFORMATION FOR SEQ ID NO:11:

(i) SEQUENCE CHARACTERISTICS:
(A) LENGTH: 222 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:11:

AATTCGATCA CTCGCGCTCC CCTCGCCTTC TGCGCTCTCC CCTCCCTGGC AGCGGCGGCA 60
ATGCCGGGGC CTTCACCAGG GCTGCGCCGA ACGCTCCTCG GCCTCTGGGC TGCCCTGGGC 120
CTGGGGATCC TAGGCATCTC AGCGGTCGCG CTAGAACCTT TCTGGGCGGA CCTTCAGCCC 180
CGCGTGGCGC TCGTGGAGCG CGGGGGCTCG CTGTGGCTCA AC 222

(2) INFORMATION FOR SEQ ID NO:12:

(i) SEQUENCE CHARACTERISTICS:
(A) LENGTH: 292 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:12:

TGTGGAGCTG GCACCCCTGC CTCCTTGGCA GCCGGTGGGC CAGAACTTCA CCCTGCGCTG 60

CCAAGTGGAG GGTGGGTCGC CCCGGACCAG CCTCACGGTG GTGCTGCTTC GCTGGGAGGA 120

GGAGCTGAGC CGGCAGCCCG CAGTGGAGGA GCCAGCGGAG GTCACTGCCA CTGTGCTGGC 180

CAGCAGAGAC GACCACGGAG CCCCTTTCTC ATGCCGCACA GAACTGGACA TGCAGCCCCA 240

GGGGCTGGGA CTGTTCGTGA ACACCTCAGC CCCCCGCCAG CTCCGAACCT TT 292

(2) INFORMATION FOR SEQ ID NO:13:

(i) SEQUENCE CHARACTERISTICS:
(A) LENGTH: 105 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:13:

Pro Asp Arg Val Glu Leu Val Pro Leu Pro Pro Trp Gln Pro Val Gly
1               5                   10                  15

Glu Asn Phe Thr Leu Ser Cys Arg Val Pro Gly Ala Gly Pro Arg Ala
            20                  25                  30

Ser Leu Thr Leu Thr Leu Leu Arg Gly Gly Gln Glu Leu Ile Arg Arg
        35                  40                  45

Ser Phe Val Gly Glu Pro Pro Arg Ala Arg Cys Ala Met Leu Thr Ala
    50                  55                  60

Thr Val Leu Ala Arg Arg Glu Asp His Arg Asp Asn Phe Ser Cys Leu
65                  70                  75                  80

Ala Glu Leu Asp Leu Arg Thr His Gly Leu Gly Leu Phe Ala Asn Ser
                85                  90                  95

Ser Ala Pro Arg Gln Leu Arg Thr Phe
            100                 105

(2) INFORMATION FOR SEQ ID NO:14:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:14:

GAACTCGAGG CCATGCCTCC ACTTTCC 27

(2) INFORMATION FOR SEQ ID NO:15:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:15:

CCATAAGCTT TATTCCACCG TGACAGCCAC 30

(2) INFORMATION FOR SEQ ID NO:16:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:16:

AACGTGCGGA GCTGTCTG 18

(2) INFORMATION FOR SEQ ID NO:17:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:17:

ACGGAATTCG AAGCCATCAA CGCCAGG 27

(2) INFORMATION FOR SEQ ID NO:18:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:18:

CATGAATTCC GAATCTTGAG TGGGATG 27

(2) INFORMATION FOR SEQ ID NO:19:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:19:

ATAGAATTCC TCGGGACACC TGTAGCC 27

(2) INFORMATION FOR SEQ ID NO:20:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:20:

CARGGTGACA AGGGCTCG 18

(2) INFORMATION FOR SEQ ID NO:21:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:21:

TATGAATTCA GTTGAGCCAC AGCGAGC 27

(2) INFORMATION FOR SEQ ID NO:22:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:22:

CCGGGTCCTA GAGGTGGACA CGCA 24

(2) INFORMATION FOR SEQ ID NO:23:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:23:

TGCAGTGTCT CCTGGCTCTG GTTC 24

(2) INFORMATION FOR SEQ ID NO:24:

(i) SEQUENCE CHARACTERISTICS:

(A) LENGTH: 992 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:24:

GCGAAAACCG GGAGACCCGG GAGAACGTGA CCATCTACAG CTTCCCGGCA CCACTCCTGA 60

CCCTGAGCGA ACCCAGCGTC TCCGAGGGGC AGATGGTGAC AGTAACCTGC GCAGCTGGGG 120

CCCAAGCTCT GGTCACACTG GAGGGAGTTC CAGCCGCGGT CCCGGGGCAG CCCGCCCAGC 180

TTCAGCTAAA TGCCACCGAG AACGACGACA GACGCAGCTT CTTCTGCGAC GCCACCCTCG 240

ATGTGGACGG GGAGACCCTG ATCAAGAACA GGAGCGCAGA GCTTCGTGTC CTATACGCTC 300

CCCGGCTAGA CGATTCGGAC TGCCCCAGGA GTTGGACGTG GCCCGAGGGC CCAGAGCAGA 360

CGCTGCGCTG CGAGGCCCGC GGGAACCCAG AACCCTCAGT GCACTGTGCG CGCTCCGACG 420

GCGGGGCCGT GCTGGCTCTG GGCCTGCTGG GTCCAGTCAC TCGGGCGCTC TCAGGCACTT 480

ACCGCTGCAA GGCGGCCAAT GATCAAGGCG AGGCGGTCAA GGACGTAACG CTAACGGTGG 540

AGTACGCACC AGCGCTGGAC AGCGTGGGCT GCCCAGAACG CATTACTTGG CTGGAGGGAA 600

CAGAAGCCTC GCTGAGCTGT GTGGCGCACG GGGTACCGCC GCCTGATGTG ATCTGCGTGC 660

GCTCTGGAGA ACTCGGGGCC GTCATCGAGG GGCTGTTGCG TGTGGCCCGG GAGCATGCGG 720

GCACTTACCG CTGCGAAGCC ACCAACCCTC GGGGCTCTGC GGCCAAAAAT GTGGCCGTCA 780

CGGTGGAATA TGGCCCCAGG TTTGAGGAGC CGAGCTGCCC CAGCAATTGG ACATGGGTGG 840

AAGGATCTGG GCGCCTGTTT TCCTGTGAGG TCGATGGGAA GCCACAGCCA AGCGTGAAGT 900

GCGTGGGCTC CGGGGGCACC ACTGAGGGGG TGCTGCTGCC GCTGGCACCC CCAGACCCTA 960

GTCCCAGAGC TCCCAGAATC CCTAGAGTCC TG 992

(2) INFORMATION FOR SEQ ID NO:25:

(i) SEQUENCE CHARACTERISTICS:
(A) LENGTH: 2775 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:25:

GCAGCCTCGC GTGGCGTTCG TGGAGCGCGG GGGCTCGCTG TGGCTGAATT GCAGCACCAA 60

CTGCCCTCGG CCGGAGCGCG GTGGCCTGGA GACCTCGCTG CGCCGAAACG GGACCCAGAG 120

GGGTTTGCGT TGGTTGGCGC GGCAGCTGGT GGACATTCGC GAGCCGGAGA CTCAGCCCGT 180

CTGCTTCTTC CGCTGCGCGC GGCGCACACT ACAGGCGCGT GGGCTCATTC GCACTTTCCA 240

GCGACCAGAT CGCGTAGAGC TGATGCCGCT GCCTCCCTGG CAGCCGGTGG GCGAGAACTT 300
CACCCTGAGC TGTAGGGTCC CCGGCGCCGG GCCCCGTGCG AGCCTCACGC TGACCCTGCT 360
GCGGGGCGCC CAGGAGCTGA TCCGCCGCAG CTTCGCCGGT GAACCACCCC GAGCGCGGGG 420
CGCGGTGCTC ACAGCCACGG TACTGGCTCG GAGGGAGGAC CATGGAGCCA ATTTCTCGTG 480
TCGCGCCGAG CTGGACCTGC GGCCGCACGG ACTGGGACTG TTTGAAAACA GCTCGGCCCC 540
CAGAGAGCTC CGAACCTTCT CCCTGTCTCC GGATGCCCCG CGCCTCGCTG CTCCCCGGCT 600
CTTGGAAGTT GGCTCGGAAA GGCCCGTGAG CTGCACTCTG GACGGACTGT TTCCAGCCTC 660
AGAGGCCAGG GTCTACCTCG CACTGGGGGA CCAGAATCTG AGTCCTGATG TCACCCTCGA 720
AGGGGACGCA TTCGTGGCCA CTGCCACAGC CACAGCTAGC GCAGAGCAGG AGGGTGCCAG 780
GCAGCTGGTC TGCAACGTCA CCCTGGGGGG CGAAAACCGG GAGACCCGGG AGAACGTGAC 840
CATCTACAGC TTCCCGGCAC CACTCCTGAC CCTGAGCGAA CCCAGCGTCT CCGAGGGGCA 900
GATGGTGACA GTAACCTGCG CAGCTGGGGC CCAAGCTCTG GTCACACTGG AGGGAGTTCC 960
AGCCGCGGTC CCGGGGCAGC CCGCCCAGCT TCAGCTAAAT GCCACCGAGA ACGACGACAG 1020
ACGCAGCTTC TTCTGCGACG CCACCCTCGA TGTGGACGGG GAGACCCTGA TCAAGAACAG 1080
GAGCGCAGAG CTTCGTGTCC TATACGCTCC CCGGCTAGAC GATTCGGACT GCCCCAGGAG 1140
TTGGACGTGG CCCGAGGGCC CAGAGCAGAC GCTGCGCTGC GAGGCCCGCG GGAACCCAGA 1200
ACCCTCAGTG CACTGTGCGC GCTCCGACGG CGGGGCCGTG CTGGCTCTGG GCCTGCTGGG 1260
TCCAGTCACT CGGGCGCTCT CAGGCACTTA CCGCTGCAAG GCGGCCAATG ATCAAGGCGA 1320
GGCGGTCAAG GACGTAACGC TAACGGTGGA GTACGCACCA GCGCTGGACA GCGTGGGCTG 1380
CCCAGAACGC ATTACTTGGC TGGAGGGAAC AGAAGCCTCG CTGAGCTGTG TGGCGCACGG 1440
GGTACCGCCG CCTGATGTGA TCTGCGTGCG CTCTGGAGAA CTCGGGGCCG TCATCGAGGG 1500
GCTGTTGCGT GTGGCCCGGG AGCATGCGGG CACTTACCGC TGCGAAGCCA CCAACCCTCG 1560
GGGCTCTGCG GCCAAAAATG TGGCCGTCAC GGTGGAATAT GGCCCCAGGT TTGAGGAGCC 1620
GAGCTGCCCC AGCAATTGGA CATGGGTGGA AGGATCTGGG CGCCTGTTTT CCTGTGAGGT 1680
CGATGGGAAG CCACAGCCAA GCGTGAAGTG CGTGGGCTCC GGGGGCACCA CTGAGGGGGT 1740
GCTGCTGCCG CTGGCACCCC CAGACCCTAG TCCCAGAGCT CCCAGAATCC CTAGAGTCCT 1800
GGCACCCGGT ATCTACGTCT GCAACGCCAC CAACCGCCAC GGCTCCGTGG CCAAAACAGT 1860
CGTCGTGAGC GCGGAGTCGC CACCGGAGAT GGATGAATCT ACCTGCCCAA GTCACCAGAC 1920
GTGGCTGGAA GGGGCTGAGG CTTCCGCGCT GGCCTGCGCC GCCCGGGGTC GCCCTTCCCC 1980
AGGAGTGCGC TGCTCTCGGG AAGGCATCCC ATGGCCTGAG CAGCAGCGCG TGTCCCGAGA 2040
GGACGCGGGC ACTTACCACT GTGTGGCCAC CAATGCGCAT GGCACGGACT CCCGGACCGT 2100
CACTGTGGGC GTGGAATACC GGCCAGTGGT GGCCGAACTT GCTGCCTCGC CCCCTGGAGG 2160

CGTGCGCCCA GGAGGAAACT TCACGTTGAC CTGCCGCGCG GAGGCCTGGC CTCCAGCCCA 2220
GATCAGCTGG CGCGCGCCCC CGAGGGCCCT CAACATCGGC CTGTCGAGCA ACAACAGCAC 2280
ACTGAGCGTG GCAGGCGCCA TGGGAAGCCA CGGCGGCGAG TACGAGTGCG CACGCACCAA 2340
CGCGCACGGG CGCCACGCGC GGCGCATCAC GGTGCGCGTG GCCGGTCCGT GGCTATGGGT 2400
CGCCGTGGGC GGCGCGGCGG GGGGCGCGGC GCTGCTGGCC GCGGGGGCCG GCCTGGCCTT 2460
CTACGTGCAG TCCACCGCCT GCAAGAAGGG CGAGTACAAC GTGCAGGAGG CCGAGAGCTC 2520
AGGCGAGGCC GTGTGTCTGA ACGGAGCGGG CGGCGGCGCT GGCGGGGCGG CAGGCGCGGA 2580
GGGCGGACCC GAGGCGGCGG GGGGCGCGGC CGAGTCGCCG GCGGAGGGCG AGGTCTTCGC 2640
CATACAGCTG ACATCGGCGT GAGCCCGCTC CCCTCTCCGC GGGCCGGGAC GCCCCCCAGA 2700
CTCACACGGG GGCTTATTTA TTGCTTTATT TATTTACTTA TTCATTTATT TATGTATTCA 2760
ACTCCAAGGG AATTC 2775

(2) INFORMATION FOR SEQ ID NO:26:

(i) SEQUENCE CHARACTERISTICS:
(A) LENGTH: 1557 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:26:

CGCGCTCTCC TCGCCTCCTG TGCTTTCCCC GCCGCGGCGA TGCCAGGGCC TTCGCCAGGG 60
CTGCGCCGGG CGCTACTCGG CCTCTGGGCT GCTCTGGGCC TGGGGCTCTT CGGCCTCTCA 120
GCGGTCTCGC AGGAGCCCTT CTGGGCGGAC CTGCAGCCTC GCGTGGCGTT CGTGGAGCGC 180
GGGGGCTCGC TGTGGCTGAA TTGCAGCACC AACTGCCCTC GGCCGGAGCG CGGTGGCCTG 240
GAGACCTCGC TGCGCCGAAA CGGGACCCAG AGGGGTTTGC GTTGGTTGGC GCGGCAGCTG 300
GTGGACATTC GCGAGCCGGA GACTCAGCCC GTCTGCTTCT TCCGCTGCGC GCGGCGCACA 360
CTACAGGCGC GTGGGCTCAT TCGCACTTTC CAGCGACCAG ATCGCGTAGA GCTGATGCCG 420
CTGCCTCCCT GGCAGCCGGT GGGCGAGAAC TTCACCCTGA GCTGTAGGGT CCCCGGCGCC 480
GGGCCCCGTG CGAGCCTCAC GCTGACCCTG CTGCGGGGCG CCCAGGAGCT GATCCGCCGC 540
AGCTTCGCCG GTGAACCACC CCGAGCGCGG GGCGCGGTGC TCACAGCCAC GGTACTGGCT 600
CGGAGGGAGG ACCATGGAGC CAATTTCTCG TGTCGCGCCG AGCTGGACCT GCGGCCGCAC 660
GGACTGGGAC TGTTTGAAAA CAGCTCGGCC CCCAGAGAGC TCCGAACCTT CTCCCTGTCT 720
CCGGATGCCC CGCGCCTCGC TGCTCCCCGG CTCTTGGAAG TTGGCTCGGA AAGGCCCGTG 780
AGCTGCACTC TGGACGGACT GTTTCCAGCC TCAGAGGCCA GGGTCTACCT CGCACTGGGG 840
GACCAGAATC TGAGTCCTGA TGTCACCCTC GAAGGGGACG CATTCGTGGC CACTGCCACA 900

GCCACAGCTA GCGCAGAGCA GGAGGGTGCC AGGCAGCTGG TCTGCAACGT CACCCTGGGG 960

GGCGAAAACC GGGAGACCCG GGAGAACGTG ACCATCTACA GCTTCCCGGC ACCACTCCTG 1020

ACCCTGAGCG AACCCAGCGT CTCCGAGGGG CAGATGGTGA CAGTAACCTG CGCAGCTGGG 1080

GCCCAAGCTC TGGTCACACT GGAGGGAGTT CCAGCCGCGG TCCCGGGGCA GCCCGCCCAG 1140

CTTCAGCTAA ATGCCACCGA GAACGACGAC AGACGCAGCT TCTTCTGCGA CGCCACCCTC 1200

GATGTGGACG GGGAGACCCT GATCAAGAAC AGGAGCGCAG AGCTTCGTGT CCTATACGCT 1260

CCCCGGCTAG ACGATTCGGA CTGCCCCAGG AGTTGGACGT GGCCCGAGGG CCCAGAGCAG 1320

ACGCTGCGCT GCGAGGCCCG CGGGAACCCA GAACCCTCAG TGCACTGTGC GCGCTCCGAC 1380

GGCGGGGCCG TGCTGGCTCT GGGCCTGCTG GGTCCAGTCA CTCGGGCGCT CTCAGGCACT 1440

TACCGCTGCA AGGCGGCCAA TGATCAAGGC GAGGCGGTCA AGGACGTAAC GCTAACGGTG 1500

GAGTACGCAC CAGCGCTGGA CAGCGTGGGC TGCCCAGAAC GCATTACTTG GCTGGAG 1557

(2) INFORMATION FOR SEQ ID NO:27:

(i) SEQUENCE CHARACTERISTICS:
(A) LENGTH: 2927 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 40..2814

(xi) SEQUENCE DESCRIPTION: SEQ ID NO:27:

CGCGCTCTCC TCGCCTCCTG TGCTTTCCCC GCCGCGGCG ATG CCA GGG CCT TCG 54
Met Pro Gly Pro Ser
1               5

CCA GGG CTG CGC CGG GCG CTA CTC GGC CTC TGG GCT GCT CTG GGC CTG 102
Pro Gly Leu Arg Arg Ala Leu Leu Gly Leu Trp Ala Ala Leu Gly Leu
                10                  15                  20

GGG CTC TTC GGC CTC TCA GCG GTC TCG CAG GAG CCC TTC TGG GCG GAC 150
Gly Leu Phe Gly Leu Ser Ala Val Ser Gln Glu Pro Phe Trp Ala Asp
            25                  30                  35

CTG CAG CCT CGC GTG GCG TTC GTG GAG CGC GGG GGC TCG CTG TGG CTG 198
Leu Gln Pro Arg Val Ala Phe Val Glu Arg Gly Gly Ser Leu Trp Leu
        40                  45                  50

AAT TGC AGC ACC AAC TGC CCT CGG CCG GAG CGC GGT GGC CTG GAG ACC 246
Asn Cys Ser Thr Asn Cys Pro Arg Pro Glu Arg Gly Gly Leu Glu Thr
    55                  60                  65

TCG CTG CGC CGA AAC GGG ACC CAG AGG GGT TTG CGT TGG TTG GCG CGG 294
Ser Leu Arg Arg Asn Gly Thr Gln Arg Gly Leu Arg Trp Leu Ala Arg
70                  75                  80                  85

CAG CTG GTG GAC ATT CGC GAG CCG GAG ACT CAG CCC GTC TGC TTC TTC 342
Gln Leu Val Asp Ile Arg Glu Pro Glu Thr Gln Pro Val Cys Phe Phe
                90                  95                  100

CGC TGC GCG CGG CGC ACA CTA CAG GCG CGT GGG CTC ATT CGC ACT TTC 390
Arg Cys Ala Arg Arg Thr Leu Gln Ala Arg Gly Leu Ile Arg Thr Phe
            105                 110                 115

CAG CGA CCA GAT CGC GTA GAG CTG ATG CCG CTG CCT CCC TGG CAG CCG 438
Gln Arg Pro Asp Arg Val Glu Leu Met Pro Leu Pro Pro Trp Gln Pro
        120                 125                 130

GTG GGC GAG AAC TTC ACC CTG AGC TGT AGG GTC CCC GGC GCC GGG CCC 486
Val Gly Glu Asn Phe Thr Leu Ser Cys Arg Val Pro Gly Ala Gly Pro
    135                 140                 145

CGT GCG AGC CTC ACG CTG ACC CTG CTG CGG GGC GCC CAG GAG CTG ATC 534
Arg Ala Ser Leu Thr Leu Thr Leu Leu Arg Gly Ala Gln Glu Leu Ile
150                 155                 160                 165

CGC CGC AGC TTC GCC GGT GAA CCA CCC CGA GCG CGG GGC GCG GTG CTC 582
Arg Arg Ser Phe Ala Gly Glu Pro Pro Arg Ala Arg Gly Ala Val Leu
                170                 175                 180

ACA GCC ACG GTA CTG GCT CGG AGG GAG GAC CAT GGA GCC AAT TTC TCG 630
Thr Ala Thr Val Leu Ala Arg Arg Glu Asp His Gly Ala Asn Phe Ser
            185                 190                 195

TGT CGC GCC GAG CTG GAC CTG CGG CCG CAC GGA CTG GGA CTG TTT GAA 678
Cys Arg Ala Glu Leu Asp Leu Arg Pro His Gly Leu Gly Leu Phe Glu
        200                 205                 210

AAC AGC TCG GCC CCC AGA GAG CTC CGA ACC TTC TCC CTG TCT CCG GAT 726
Asn Ser Ser Ala Pro Arg Glu Leu Arg Thr Phe Ser Leu Ser Pro Asp
    215                 220                 225

GCC CCG CGC CTC GCT GCT CCC CGG CTC TTG GAA GTT GGC TCG GAA AGG 774
Ala Pro Arg Leu Ala Ala Pro Arg Leu Leu Glu Val Gly Ser Glu Arg
230                 235                 240                 245

CCC GTG AGC TGC ACT CTG GAC GGA CTG TTT CCA GCC TCA GAG GCC AGG 822
Pro Val Ser Cys Thr Leu Asp Gly Leu Phe Pro Ala Ser Glu Ala Arg
                250                 255                 260

GTC TAC CTC GCA CTG GGG GAC CAG AAT CTG AGT CCT GAT GTC ACC CTC 870
Val Tyr Leu Ala Leu Gly Asp Gln Asn Leu Ser Pro Asp Val Thr Leu
            265                 270                 275

GAA GGG GAC GCA TTC GTG GCC ACT GCC ACA GCC ACA GCT AGC GCA GAG 918
Glu Gly Asp Ala Phe Val Ala Thr Ala Thr Ala Thr Ala Ser Ala Glu
        280                 285                 290

CAG GAG GGT GCC AGG CAG CTG GTC TGC AAC GTC ACC CTG GGG GGC GAA 966
Gln Glu Gly Ala Arg Gln Leu Val Cys Asn Val Thr Leu Gly Gly Glu
    295                 300                 305

AAC CGG GAG ACC CGG GAG AAC GTG ACC ATC TAC AGC TTC CCG GCA CCA 1014
Asn Arg Glu Thr Arg Glu Asn Val Thr Ile Tyr Ser Phe Pro Ala Pro
310                 315                 320                 325

CTC CTG ACC CTG AGC GAA CCC AGC GTC TCC GAG GGG CAG ATG GTG ACA 1062
Leu Leu Thr Leu Ser Glu Pro Ser Val Ser Glu Gly Gln Met Val Thr
                330                 335                 340

GTA ACC TGC GCA GCT GGG GCC CAA GCT CTG GTC ACA CTG GAG GGA GTT 1110
Val Thr Cys Ala Ala Gly Ala Gln Ala Leu Val Thr Leu Glu Gly Val
            345                 350                 355

CCA GCC GCG GTC CCG GGG CAG CCC GCC CAG CTT CAG CTA AAT GCC ACC 1158
Pro Ala Ala Val Pro Gly Gln Pro Ala Gln Leu Gln Leu Asn Ala Thr
        360                 365                 370

GAG AAC GAC GAC AGA CGC AGC TTC TTC TGC GAC GCC ACC CTC GAT GTG 1206
Glu Asn Asp Asp Arg Arg Ser Phe Phe Cys Asp Ala Thr Leu Asp Val
    375                 380                 385

GAC GGG GAG ACC CTG ATC AAG AAC AGG AGC GCA GAG CTT CGT GTC CTA 1254
Asp Gly Glu Thr Leu Ile Lys Asn Arg Ser Ala Glu Leu Arg Val Leu
390                 395                 400                 405

TAC GCT CCC CGG CTA GAC GAT TCG GAC TGC CCC AGG AGT TGG ACG TGG 1302
Tyr Ala Pro Arg Leu Asp Asp Ser Asp Cys Pro Arg Ser Trp Thr Trp
                410                 415                 420

CCC GAG GGC CCA GAG CAG ACG CTG CGC TGC GAG GCC CGC GGG AAC CCA 1350
Pro Glu Gly Pro Glu Gln Thr Leu Arg Cys Glu Ala Arg Gly Asn Pro
            425                 430                 435

GAA CCC TCA GTG CAC TGT GCG CGC TCC GAC GGC GGG GCC GTG CTG GCT 1398
Glu Pro Ser Val His Cys Ala Arg Ser Asp Gly Gly Ala Val Leu Ala
        440                 445                 450

CTG GGC CTG CTG GGT CCA GTC ACT CGG GCG CTC TCA GGC ACT TAC CGC 1446
Leu Gly Leu Leu Gly Pro Val Thr Arg Ala Leu Ser Gly Thr Tyr Arg
    455                 460                 465

TGC AAG GCG GCC AAT GAT CAA GGC GAG GCG GTC AAG GAC GTA ACG CTA 1494
Cys Lys Ala Ala Asn Asp Gln Gly Glu Ala Val Lys Asp Val Thr Leu
470                 475                 480                 485

ACG GTG GAG TAC GCA CCA GCG CTG GAC AGC GTG GGC TGC CCA GAA CGC 1542
Thr Val Glu Tyr Ala Pro Ala Leu Asp Ser Val Gly Cys Pro Glu Arg
                490                 495                 500

ATT ACT TGG CTG GAG GGA ACA GAA GCC TCG CTG AGC TGT GTG GCG CAC 1590
Ile Thr Trp Leu Glu Gly Thr Glu Ala Ser Leu Ser Cys Val Ala His
            505                 510                 515

GGG GTA CCG CCG CCT GAT GTG ATC TGC GTG CGC TCT GGA GAA CTC GGG 1638
Gly Val Pro Pro Pro Asp Val Ile Cys Val Arg Ser Gly Glu Leu Gly
        520                 525                 530

GCC GTC ATC GAG GGG CTG TTG CGT GTG GCC CGG GAG CAT GCG GGC ACT 1686
Ala Val Ile Glu Gly Leu Leu Arg Val Ala Arg Glu His Ala Gly Thr
    535                 540                 545

TAC CGC TGC GAA GCC ACC AAC CCT CGG GGC TCT GCG GCC AAA AAT GTG 1734
Tyr Arg Cys Glu Ala Thr Asn Pro Arg Gly Ser Ala Ala Lys Asn Val
550                 555                 560                 565

GCC GTC ACG GTG GAA TAT GGC CCC AGG TTT GAG GAG CCG AGC TGC CCC 1782
Ala Val Thr Val Glu Tyr Gly Pro Arg Phe Glu Glu Pro Ser Cys Pro
                570                 575                 580

AGC AAT TGG ACA TGG GTG GAA GGA TCT GGG CGC CTG TTT TCC TGT GAG 1830
Ser Asn Trp Thr Trp Val Glu Gly Ser Gly Arg Leu Phe Ser Cys Glu
            585                 590                 595

GTC GAT GGG AAG CCA CAG CCA AGC GTG AAG TGC GTG GGC TCC GGG GGC 1878
Val Asp Gly Lys Pro Gln Pro Ser Val Lys Cys Val Gly Ser Gly Gly
        600                 605                 610

ACC ACT GAG GGG GTG CTG CTG CCG CTG GCA CCC CCA GAC CCT AGT CCC 1926
Thr Thr Glu Gly Val Leu Leu Pro Leu Ala Pro Pro Asp Pro Ser Pro
    615                 620                 625

AGA GCT CCC AGA ATC CCT AGA GTC CTG GCA CCC GGT ATC TAC GTC TGC 1974
Arg Ala Pro Arg Ile Pro Arg Val Leu Ala Pro Gly Ile Tyr Val Cys
630                 635                 640                 645

AAC GCC ACC AAC CGC CAC GGC TCC GTG GCC AAA ACA GTC GTC GTG AGC 2022
Asn Ala Thr Asn Arg His Gly Ser Val Ala Lys Thr Val Val Val Ser
                650                 655                 660

GCG GAG TCG CCA CCG GAG ATG GAT GAA TCT ACC TGC CCA AGT CAC CAG 2070
Ala Glu Ser Pro Pro Glu Met Asp Glu Ser Thr Cys Pro Ser His Gln
            665                 670                 675

ACG TGG CTG GAA GGG GCT GAG GCT TCC GCG CTG GCC TGC GCC GCC CGG 2118
Thr Trp Leu Glu Gly Ala Glu Ala Ser Ala Leu Ala Cys Ala Ala Arg
        680                 685                 690

GGT CGC CCT TCC CCA GGA GTG CGC TGC TCT CGG GAA GGC ATC CCA TGG 2166
Gly Arg Pro Ser Pro Gly Val Arg Cys Ser Arg Glu Gly Ile Pro Trp
    695                 700                 705

CCT GAG CAG CAG CGC GTG TCC CGA GAG GAC GCG GGC ACT TAC CAC TGT 2214
Pro Glu Gln Gln Arg Val Ser Arg Glu Asp Ala Gly Thr Tyr His Cys
710                 715                 720                 725

GTG GCC ACC AAT GCG CAT GGC ACG GAC TCC CGG ACC GTC ACT GTG GGC 2262
Val Ala Thr Asn Ala His Gly Thr Asp Ser Arg Thr Val Thr Val Gly
                730                 735                 740

GTG GAA TAC CGG CCA GTG GTG GCC GAA CTT GCT GCC TCG CCC CCT GGA 2310
Val Glu Tyr Arg Pro Val Val Ala Glu Leu Ala Ala Ser Pro Pro Gly
            745                 750                 755

GGC GTG CGC CCA GGA GGA AAC TTC ACG TTG ACC TGC CGC GCG GAG GCC 2358
Gly Val Arg Pro Gly Gly Asn Phe Thr Leu Thr Cys Arg Ala Glu Ala
        760                 765                 770

TGG CCT CCA GCC CAG ATC AGC TGG CGC GCG CCC CCG AGG GCC CTC AAC 2406
Trp Pro Pro Ala Gln Ile Ser Trp Arg Ala Pro Pro Arg Ala Leu Asn
    775                 780                 785

ATC GGC CTG TCG AGC AAC AAC AGC ACA CTG AGC GTG GCA GGC GCC ATG 2454
Ile Gly Leu Ser Ser Asn Asn Ser Thr Leu Ser Val Ala Gly Ala Met
790                 795                 800                 805

GGA AGC CAC GGC GGC GAG TAC GAG TGC GCA CGC ACC AAC GCG CAC GGG 2502
Gly Ser His Gly Gly Glu Tyr Glu Cys Ala Arg Thr Asn Ala His Gly
                810                 815                 820

CGC CAC GCG CGG CGC ATC ACG GTG CGC GTG GCC GGT CCG TGG CTA TGG 2550
Arg His Ala Arg Arg Ile Thr Val Arg Val Ala Gly Pro Trp Leu Trp
            825                 830                 835

GTC GCC GTG GGC GGC GCG GCG GGG GGC GCG GCG CTG CTG GCC GCG GGG 2598
Val Ala Val Gly Gly Ala Ala Gly Gly Ala Ala Leu Leu Ala Ala Gly
        840                 845                 850

GCC GGC CTG GCC TTC TAC GTG CAG TCC ACC GCC TGC AAG AAG GGC GAG 2646
Ala Gly Leu Ala Phe Tyr Val Gln Ser Thr Ala Cys Lys Lys Gly Glu
    855                 860                 865

TAC AAC GTG CAG GAG GCC GAG AGC TCA GGC GAG GCC GTG TGT CTG AAC 2694
Tyr Asn Val Gln Glu Ala Glu Ser Ser Gly Glu Ala Val Cys Leu Asn
870                 875                 880                 885

GGA GCG GGC GGC GGC GCT GGC GGG GCG GCA GGC GCG GAG GGC GGA CCC 2742
Gly Ala Gly Gly Gly Ala Gly Gly Ala Ala Gly Ala Glu Gly Gly Pro
                890                 895                 900

GAG GCG GCG GGG GGC GCG GCC GAG TCG CCG GCG GAG GGC GAG GTC TTC 2790
Glu Ala Ala Gly Gly Ala Ala Glu Ser Pro Ala Glu Gly Glu Val Phe
            905                 910                 915

GCC ATA CAG CTG ACA TCG GCG TGAGCCCGCT CCCCTCTCCG CGGGCCGGGA 2841
Ala Ile Gln Leu Thr Ser Ala
920 925

CGCCCCCCAG ACTCACACGG GGGCTTATTT ATTGCTTTAT TTATTTACTT ATTCATTTAT 2901

TTATGTATTC AACTCCAAGG GAATTC 2927

(2) INFORMATION FOR SEQ ID NO:28:

(i) SEQUENCE CHARACTERISTICS:
(A) LENGTH: 924 amino acids
(B) TYPE: amino acid
(D) TOPOLOGY: linear

(ii) MOLECULE TYPE: protein

(xi) SEQUENCE DESCRIPTION: SEQ ID NO:28:

Met Pro Gly Pro Ser Pro Gly Leu Arg Arg Ala Leu Leu Gly Leu Trp
1               5                   10                  15

Ala Ala Leu Gly Leu Gly Leu Phe Gly Leu Ser Ala Val Ser Gln Glu
            20                  25                  30

Pro Phe Trp Ala Asp Leu Gln Pro Arg Val Ala Phe Val Glu Arg Gly
        35                  40                  45

Gly Ser Leu Trp Leu Asn Cys Ser Thr Asn Cys Pro Arg Pro Glu Arg
    50                  55                  60

Gly Gly Leu Glu Thr Ser Leu Arg Arg Asn Gly Thr Gln Arg Gly Leu
65                  70                  75                  80

Arg Trp Leu Ala Arg Gln Leu Val Asp Ile Arg Glu Pro Glu Thr Gln
                85                  90                  95

Pro Val Cys Phe Phe Arg Cys Ala Arg Arg Thr Leu Gln Ala Arg Gly
            100                 105                 110

Leu Ile Arg Thr Phe Gln Arg Pro Asp Arg Val Glu Leu Met Pro Leu
        115                 120                 125

Pro Pro Trp Gln Pro Val Gly Glu Asn Phe Thr Leu Ser Cys Arg Val
    130                 135                 140

Pro Gly Ala Gly Pro Arg Ala Ser Leu Thr Leu Thr Leu Leu Arg Gly
145                 150                 155                 160

Ala Gln Glu Leu Ile Arg Arg Ser Phe Ala Gly Glu Pro Pro Arg Ala
                165                 170                 175

Arg Gly Ala Val Leu Thr Ala Thr Val Leu Ala Arg Arg Glu Asp His
            180                 185                 190

Gly Ala Asn Phe Ser Cys Arg Ala Glu Leu Asp Leu Arg Pro His Gly
        195                 200                 205

Leu Gly Leu Phe Glu Asn Ser Ser Ala Pro Arg Glu Leu Arg Thr Phe
    210                 215                 220

Ser Leu Ser Pro Asp Ala Pro Arg Leu Ala Ala Pro Arg Leu Leu Glu
225                 230                 235                 240

Val Gly Ser Glu Arg Pro Val Ser Cys Thr Leu Asp Gly Leu Phe Pro
                245                 250                 255

Ala Ser Glu Ala Arg Val Tyr Leu Ala Leu Gly Asp Gln Asn Leu Ser
            260                 265                 270

Pro Asp Val Thr Leu Glu Gly Asp Ala Phe Val Ala Thr Ala Thr Ala
        275                 280                 285

Thr Ala Ser Ala Glu Gln Glu Gly Ala Arg Gln Leu Val Cys Asn Val
    290                 295                 300

Thr Leu Gly Gly Glu Asn Arg Glu Thr Arg Glu Asn Val Thr Ile Tyr
305                 310                 315                 320

Ser Phe Pro Ala Pro Leu Leu Thr Leu Ser Glu Pro Ser Val Ser Glu
                325                 330                 335

Gly Gln Met Val Thr Val Thr Cys Ala Ala Gly Ala Gln Ala Leu Val
            340                 345                 350

Thr Leu Glu Gly Val Pro Ala Ala Val Pro Gly Gln Pro Ala Gln Leu
        355                 360                 365

Gln Leu Asn Ala Thr Glu Asn Asp Asp Arg Arg Ser Phe Phe Cys Asp
    370                 375                 380

Ala Thr Leu Asp Val Asp Gly Glu Thr Leu Ile Lys Asn Arg Ser Ala
385                 390                 395                 400

Glu Leu Arg Val Leu Tyr Ala Pro Arg Leu Asp Asp Ser Asp Cys Pro
                405                 410                 415

Arg Ser Trp Thr Trp Pro Glu Gly Pro Glu Gln Thr Leu Arg Cys Glu
            420                 425                 430

Ala Arg Gly Asn Pro Glu Pro Ser Val His Cys Ala Arg Ser Asp Gly
        435                 440                 445

Gly Ala Val Leu Ala Leu Gly Leu Leu Gly Pro Val Thr Arg Ala Leu
    450                 455                 460

Ser Gly Thr Tyr Arg Cys Lys Ala Ala Asn Asp Gln Gly Glu Ala Val
465                 470                 475                 480

Lys Asp Val Thr Leu Thr Val Glu Tyr Ala Pro Ala Leu Asp Ser Val
                485                 490                 495

Gly Cys Pro Glu Arg Ile Thr Trp Leu Glu Gly Thr Glu Ala Ser Leu
            500                 505                 510

Ser Cys Val Ala His Gly Val Pro Pro Pro Asp Val Ile Cys Val Arg
        515                 520                 525

Ser Gly Glu Leu Gly Ala Val Ile Glu Gly Leu Leu Arg Val Ala Arg
    530                 535                 540

Glu His Ala Gly Thr Tyr Arg Cys Glu Ala Thr Asn Pro Arg Gly Ser
545                 550                 555                 560

Ala Ala Lys Asn Val Ala Val Thr Val Glu Tyr Gly Pro Arg Phe Glu
                565                 570                 575

Glu Pro Ser Cys Pro Ser Asn Trp Thr Trp Val Glu Gly Ser Gly Arg
            580                 585                 590

Leu Phe Ser Cys Glu Val Asp Gly Lys Pro Gln Pro Ser Val Lys Cys
        595                 600                 605

Val Gly Ser Gly Gly Thr Thr Glu Gly Val Leu Leu Pro Leu Ala Pro
    610                 615                 620

Pro Asp Pro Ser Pro Arg Ala Pro Arg Ile Pro Arg Val Leu Ala Pro
625                 630                 635                 640

Gly Ile Tyr Val Cys Asn Ala Thr Asn Arg His Gly Ser Val Ala Lys
                645                 650                 655

Thr Val Val Val Ser Ala Glu Ser Pro Pro Glu Met Asp Glu Ser Thr
            660                 665                 670

Cys Pro Ser His Gln Thr Trp Leu Glu Gly Ala Glu Ala Ser Ala Leu
        675                 680                 685

Ala Cys Ala Ala Arg Gly Arg Pro Ser Pro Gly Val Arg Cys Ser Arg
    690                 695                 700

Glu Gly Ile Pro Trp Pro Glu Gln Gln Arg Val Ser Arg Glu Asp Ala
705                 710                 715                 720

Gly Thr Tyr His Cys Val Ala Thr Asn Ala His Gly Thr Asp Ser Arg
                725                 730                 735

Thr Val Thr Val Gly Val Glu Tyr Arg Pro Val Val Ala Glu Leu Ala
            740                 745                 750

Ala Ser Pro Pro Gly Gly Val Arg Pro Gly Gly Asn Phe Thr Leu Thr
        755                 760                 765

Cys Arg Ala Glu Ala Trp Pro Pro Ala Gln Ile Ser Trp Arg Ala Pro
    770                 775                 780

Pro Arg Ala Leu Asn Ile Gly Leu Ser Ser Asn Asn Ser Thr Leu Ser
785                 790                 795                 800

Val Ala Gly Ala Met Gly Ser His Gly Gly Glu Tyr Glu Cys Ala Arg
                805                 810                 815

Thr Asn Ala His Gly Arg His Ala Arg Arg Ile Thr Val Arg Val Ala
            820                 825                 830

Gly Pro Trp Leu Trp Val Ala Val Gly Gly Ala Ala Gly Gly Ala Ala
        835                 840                 845

Leu Leu Ala Ala Gly Ala Gly Leu Ala Phe Tyr Val Gln Ser Thr Ala
    850                 855                 860

Cys Lys Lys Gly Glu Tyr Asn Val Gln Glu Ala Glu Ser Ser Gly Glu
865                 870                 875                 880

Ala Val Cys Leu Asn Gly Ala Gly Gly Gly Ala Gly Gly Ala Ala Gly
                885                 890                 895

Ala Glu Gly Gly Pro Glu Ala Ala Gly Gly Ala Ala Glu Ser Pro Ala
            900                 905                 910

Glu Gly Glu Val Phe Ala Ile Gln Leu Thr Ser Ala
        915                 920

(2) INFORMATION FOR SEQ ID NO:29:

(i) SEQUENCE CHARACTERISTICS:
(A) LENGTH: 65 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:29:

GTACTTACAG GATCCGCGGT CTCGCAGGAG CCCTTCTGGG CGGACCTACA GCCTGCGTGG 60

CGTTC 65

(2) INFORMATION FOR SEQ ID NO:30:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:30:

ATTTCTCTCG AGGATGGTCA CGTTCTCCCG G 31

(2) INFORMATION FOR SEQ ID NO:31:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:31:

ATTTCTGGAT CCTACAGCTT CCCGGCACCA CTC 33

(2) INFORMATION FOR SEQ ID NO:32:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:32:

ATTTCTCTCG AGTTCCACGC CCACAGTGAC GG 32

(2) INFORMATION FOR SEQ ID NO:33:

(i) SEQUENCE CHARACTERISTICS:
(A) LENGTH: 1687 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:33:

GGATCCTTTG AGCCCTGAAA GTCGAGGTTG CAGTGAGCCT TGATCGTGCC ACTGCACTCC 60
AGCCTGGGGG ACAGAGCACG ACCCTGTCTC CAAAAATAAA ATAAAAATAA AAATAAATAT 120
TGGCGGGGGA ACCCTCTGGA ATCAATAAAG GCTTCCTTAA CCAGCCTCTG TCCTGTGACC 180
TAAGGGTCCG CATTACTGCC CTTCTTCGGA GGAACTGGTT TGTTTTTGTT GTTGTTGTTG 240
TTTTTGCGAT CACTTTCTCC AAGTTCCTTG TCTCCCTGAG GGCACCTGAG GTTTCCTCAC 300
TCAGGGCCCA CCTGGGGTCC CGAAGCCCCA GACTCTGTGT ATCCCCAGCG GGTGTCACAG 360
AAACCTCTCC TTCTGCTGGC CTTATCGAGT GGGATCAGCG CGGCCGGGGA GAGCCACGGG 420
CAGGGGCGGG GTGGGGTTCA TGGTATGGCT TTCCTGATTG GCGCCGCCGC CACCACGCGG 480
CAGCTCTGAT TGGATGTTAA GTTTCCTATC CCAGCCCCAC CTTCAGACCC TGTGCTTTCC 540
TGGAGGCCAA ACAACTGTGG AGCGAGAACT CATCTCCAAA ATAACTTACC ACGCTGGAGT 600
GAGACCACGA ATGGTGGGGA GGGGAGGGTC CCACGGACAT ATTGAGGGAC GTGGATACGC 660
AGAAGAGGTA TCCATGTGGT GGCAGCCGGG AAGGGGTGAT CAGATGGTCC ACAGGGAATA 720
TCACAAACTC GAATTCTGAC GATGTTCTGG TAGTCACCCA GCCAGATGAG CGCATGGAGT 780
TGGCGGTGGG GGGTGTCAAA GCTTGGGGCC CGGAAGCGGA GTCAAAAGCA TCACCCTCGG 840
TCCCTTGTTC TCGCGTGGAT GTCAGGGCCT CCACCCACCG AGCAGAAGGC GGACTCAGGG 900
GCGCTCCAGG GTGGCTCGAG CTCACACACG CTGAGTAGAC ACGTGCCCGC TGCACCCTGG 960
GTAAATACAG ACCCGGAGCC GAGCGGATTC TAATTTAGAC GCCCGCGAAC GCTGCGCGCA 1020
CGCACACGTG TCCTCGGCTC GCTGGCACTT TCGTCCCGCC CCCTCCGTCG CGTGCCGGAG 1080

CTGACCCGGA GGGGTGCTTA GAGGTATGGC TCCGCGGGGT CAAAAGGAGA AGGATCAGTG 1140

AGAGAGGATC CCCACACCCT CCCCTAGAAC TGTCCTTTCC CCATCCAGTG CCTCCCAAAT 1200

CTCTCTTAGT CCCCAAATGT ATCCCCGCCC TAAGGGGCGC TGGTGGGAGG AGCTAAATGT 1260

GGGGGCGGAG CTCGGAGTCC AGCTTATTAT CATGGCATCT CAGCCAGGGC TGGGGTAGGG 1320

GTTTGGGAAG GGCAACCCAG CATCCCCCGA TCCCAGAGTC GCGGCCGGGG ATGACGCGAG 1380

AGAGCGTGGT CGCCCCCAGA AGGCCCTGGG CCATCATGCC GGCCTCCACG TAGACCCCAG 1440

GGGTCGCTCA CTCCTGCCAG CTCGCCTTCA CCAAGGCCAG GAGCTTAGCG CACGCTCGCC 1500

TCCCGCCCCC CCGCCGCCTC TGCCGCCGCC CCCTCCTTGG AAACCAAGTT ACCAACGTTA 1560

AACCAATCCC CAAGCGCAAC TCTGTCTCCC CCACACCCCA CCCGCCGCGC CGCGCGGAGC 1620

CGTCCTCTAG CCCAGCTCCT CGGCTCGCGC TCTCCTCGCC TCCTGTGCTT TCCCCGCCGC 1680

GGCGATG 1687

(2) INFORMATION FOR SEQ ID NO:34:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:34:

CAGAACTAAG CTTACAGGAG GCGAGGAGAG CGCGAG 36

(2) INFORMATION FOR SEQ ID NO:35:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:35:

CAACAATGCT AGCCAAGCGC AACTCTGTCT C 31

(2) INFORMATION FOR SEQ ID NO:36:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:36:

CAACAATGCT AGCCTTGGAA ACCAAGTTAC C 31

(2) INFORMATION FOR SEQ ID NO:37:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:37:

CAACAATGCT AGCAGGAGCT TAGCGCACGC TCG 33

(2) INFORMATION FOR SEQ ID NO:38:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:38:

CAACAATGCT AGCCATGCCG GCCTCCACGT AG 32

(2) INFORMATION FOR SEQ ID NO:39:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:39:

CAACAATGCT AGCGTCCAGC TTATTATCAT G 31

(2) INFORMATION FOR SEQ ID NO:40:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:40:

CAACAATGCT AGCCTTAGTC CCCAAATGTA TC 32

(2) INFORMATION FOR SEQ ID NO:41:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:41:

CAACAATGCT AGCGGAGAAG GATCAGTGAG 30

(2) INFORMATION FOR SEQ ID NO:42:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:42:

CAACAATGCT AGCCTCCACC CACCGAGCAG AAG 33